(19) Europäisches Patentamt
European Patent Office
Office européen des brevets

(11) EP 4 163 288 A1

(12) EUROPEAN PATENT APPLICATION
published in accordance with Art. 153(4) EPC

(43) Date of publication:
12.04.2023 Bulletin 2023/15

(21) Application number: 21809217.9

(22) Date of filing: 18.05.2021

(51) International Patent Classification (IPC):
*C07K 7/06* (2006.01) *A61K 47/60* (2017.01)
*A61K 47/64* (2017.01) *A61P 35/00* (2006.01)

(52) Cooperative Patent Classification (CPC):
A61K 47/60; A61K 47/64; A61P 35/00; C07K 7/06

(86) International application number:
PCT/IB2021/054235

(87) International publication number:
WO 2021/234550 (25.11.2021 Gazette 2021/47)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 19.05.2020 KR 20200059487

(71) Applicant: Anygen Co., Ltd.
Gwangju 61008 (KR)

(72) Inventors:
- KIM, Jaeil
  Gwangju 62254 (KR)
- NAM, Jeong-Seok
  Gwangju 61005 (KR)
- KIM, Jae-Hyun
  Gwangju 61514 (KR)
- RYU, Jae-Ha
  Gwangju 62278 (KR)

(74) Representative: Mewburn Ellis LLP
Aurora Building
Counterslip
Bristol BS1 6BX (GB)

Remarks:
The complete document including Reference Table(s) and the Sequence Listing(s) can be downloaded from the EPO website

# (54) NOVEL NUCLEOLIN-BINDING PEPTIDE AND USE THEREOF

(57) The present invention relates to a novel peptide that specifically binds to nucleolin and, more specifically, to a peptide represented by a specific amino acid sequence, a conjugate comprising the peptide and an anticancer drug, a fusion peptide comprising the peptide and a cell-penetrating peptide, a composition for diagnosing cancer comprising the peptide, and a composition for preventing or treating cancer comprising the peptide or the conjugate. In the present invention, it has been confirmed that a peptide ligand AGM peptide and mutants thereof, as screened using an MAP synthesis method and an OBOC combination method, specifically bind to cancer cells, and a conjugate thereof with an anticancer drug inhibits cancer growth (*in vitro* and *in vivo*). Thus, the NCL-targeting AGM peptide can be effectively used for diagnosis and targeted drug delivery in cancer therapy.

EP 4 163 288 A1

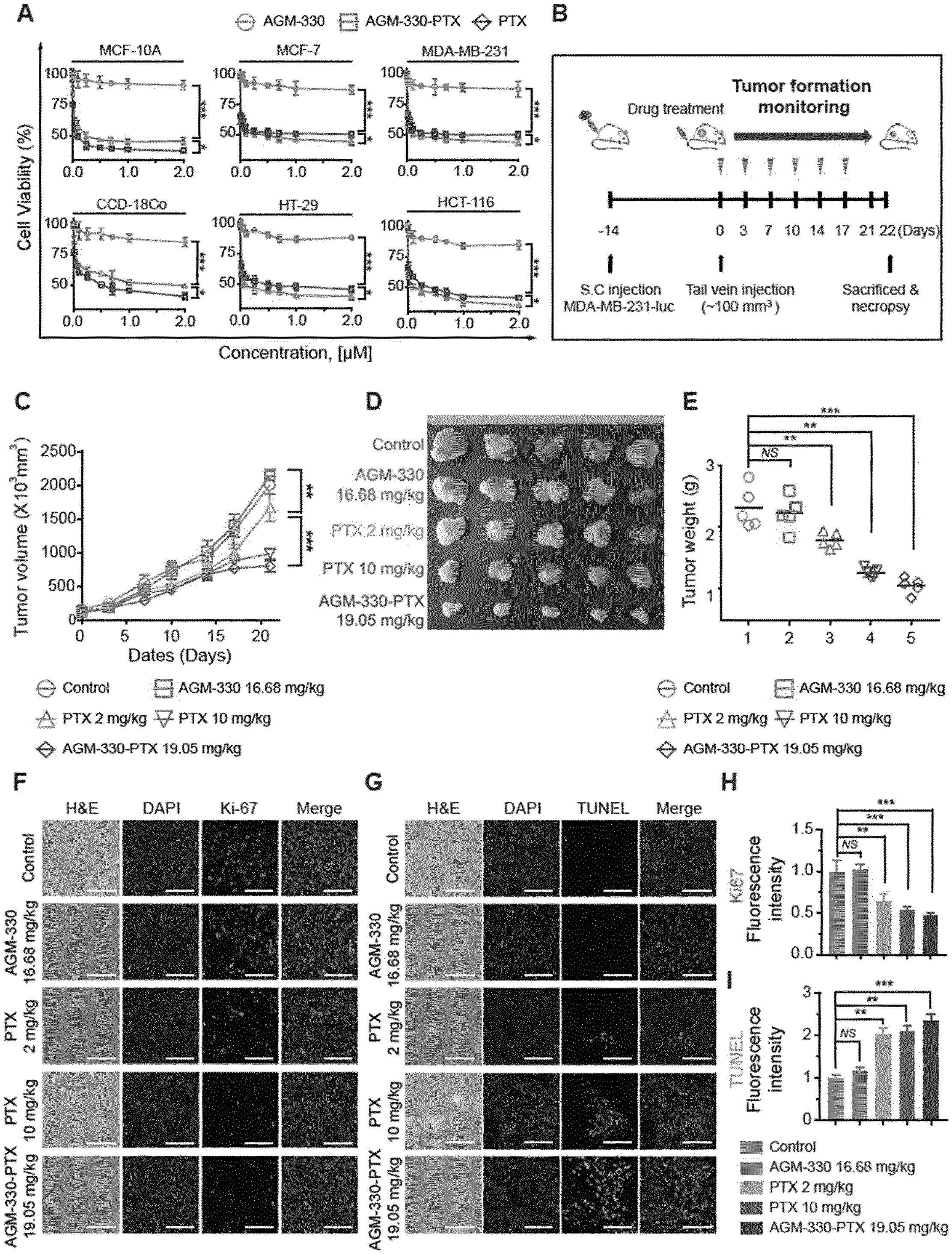
A
AGM-330
AGM-330-PTX
PTX
MCF-10A
MCF-7
MDA-MB-231
CCD-18Co
HT-29
HCT-116
Cell Viability (%)
Concentration, [μM]
B
Drug treatment
Tumor formation monitoring
-14
0 3 7 10 14 17 21 22 (Days)
S.C injection MDA-MB-231-luc
Tail vein injection (~100 mm³)
Sacrificed & necropsy
C
Tumor volume (X10³mm³)
Dates (Days)
D
Control
AGM-330 16.68 mg/kg
PTX 2 mg/kg
PTX 10 mg/kg
AGM-330-PTX 19.05 mg/kg
E
Tumor weight (g)
NS
Control
AGM-330 16.68 mg/kg
PTX 2 mg/kg
PTX 10 mg/kg
AGM-330-PTX 19.05 mg/kg
F
H&E
DAPI
Ki-67
Merge
G
H&E
DAPI
TUNEL
Merge
H
Ki67 Fluorescence intensity
I
TUNEL Fluorescence intensity
Control
AGM-330 16.68 mg/kg
PTX 2 mg/kg
PTX 10 mg/kg
AGM-330-PTX 19.05 mg/kg

Figure 14

## Description

### Technical Field

The present invention relates to a novel peptide that specifically binds to nucleolin, and more particularly, to a peptide represented by a specific amino acid sequence, a conjugate comprising the peptide and an anticancer agent, a fusion peptide comprising the peptide and a cell-penetrating peptide, a composition for diagnosing cancer comprising the peptide, and a composition for preventing or treating cancer comprising the peptide or the conjugate.

### Background Art

The use of cancer-specific ligands as pharmaceutical carriers allows relatively tissue- or cell-specific delivery of a higher payload of chemotherapeutic agents than that which is achieved by conventional drugs, which in turn decreases systemic toxicity (Allen TM. Nat Rev Cancer. 2002; 2: 750-63). Among cancer-specific ligands, nanoparticles and antibodies have been widely studied in clinical cancer diagnosis and therapy (Yao VJ, et al. J Control Release. 2016; 240: 267-86). Theragnostic nanoparticles and antibodies show great promise in the emerging field of personalized medicine because they allow the detection and monitoring of an individual patient's cancer at an early stage and the delivery of anticancer agents over an extended period for enhanced therapeutic efficacy (Palmieri D, et al. Proc Natl Acad Sci U S A. 2015; 112: 9418-23). Although nanoparticles are promising drug carrier systems, their instability in circulation, inadequate tissue distribution, and cytotoxicity present unresolved limitations to practical applications (Sukhanova A, et al. Nanoscale Res Lett. 2018; 13: 44). Additionally, the limitations of therapeutic antibodies include slow delivery and diffusion into tumor tissue due to their large size (Epenetos AA, et al. Cancer Res. 1986; 46: 3183-91).

As an alternative to classical diagnostic and therapeutic methods, cancer-specific peptides can be used to increase treatment efficiency and reduce the side effects associated with nanoparticle and antibody cancer therapy (Mori T. Curr Pharm Des. 2004; 10: 2335-43). Peptide ligands have many advantages, including easy synthesis in large quantities, low immunogenicity, the generation of nontoxic metabolites, and high *in vivo* biocompatibility (McGregor DP. Curr Opin Pharmacol. 2008; 8: 616-9). Among the many ways to discover peptides, one-bead-one-compound (OBOC) combinatorial methods are one powerful tool for screening peptide ligands (Lam KS, et al. Chem Rev. 1997; 97: 411-48). Peptide screening approaches based on OBOC combinatorial libraries have facilitated the discovery of novel peptide ligands for cellular targeting in cancer and other diseases (Mikawa M, et al. Mol Cancer Ther. 2004; 3: 1329-34). Although numerous cancer-specific peptides have been isolated using *in vitro* OBOC combinatorial screening or other methods, several challenges remain. In particular, the main drawback in the use of a peptide as a drug is its extremely short half-life due to very rapid cleavage by different peptidases (Borchardt TR, et al. Adv Drug Deliv Rev. 1997; 27: 235-56). The present inventors aimed to overcome the problems outlined above by developing a dedicated approach that synthesizes bioactive peptides in multiple-antigen peptide (MAP) dendrimeric form. The synthesis of monomeric peptides in dendrimeric forms can result in increased stability due to acquired resistance to protease and peptidase activity (McGuire MJ, et al. Sci Rep. 2014; 4: 4480) .

Accordingly, the present inventors combined OBOC combinatorial screening and MAP synthesis and discovered peptide ligand series, named AGM or AGM peptides, which specifically bind to human breast and colorectal cancer cells. *In vivo* fluorescence imaging demonstrated that AGM series peptides were specifically distributed more in tumors than in normal tissues. In addition, treatment with paclitaxel (PTX)-conjugated AGM improved PTX accumulation in cancer cells and inhibited breast and colorectal cancer cells more efficiently than treatment with PTX alone *in vitro.* Furthermore, treatment with PTX-conjugated AGM facilitated the specific localization of PTX in tumor tissues, resulting in increased cytotoxicity, enhanced drug accumulation, and improved anticancer efficacy in a xenograft model of breast cancer. Moreover, pull-down assays followed by LC-MS/MS indicated that nucleolin (NCL) is the target protein of AGM. NCL is the most abundant protein in the nucleolus. In the present invention, overexpression of NCL in the cancer cell membrane and neutralization of cell membrane NCL confirmed by using anti-NCL antibodies to inhibit cancer cell proliferation and increase apoptotic rates *in vitro.* Because of its carcinogenic roles and expression on cancer cell membranes, NCL represents an attractive target for cancer treatments. Taken together, these results indicate that AGM series peptides are novel tumor-targeting peptides for cancer diagnosis and treatment.

The information disclosed in the Background Art is only for enhancement of understanding of the background of the present invention, and it may not include information that forms conventional art that is already known in the art to which the present invention pertains.

### Summary of the Invention

An object of the present invention is to provide a peptide that specifically binds to nucleolin (NCL).

Another object of the present invention is to provide a conjugate comprising the peptide and an anticancer agent.

Still another object of the present invention is to provide a multimer comprising two or more of the peptide.

Yet another object of the present invention is to provide a conjugate comprising the multimer and an anticancer agent.

Still yet another object of the present invention is to provide a fusion peptide in which the peptide and a cell-penetrating peptide (CPP) are fused together, and a fusion peptide in which the multimer and a cell-penetrating peptide are fused together.

A further object of the present invention is to provide a composition for diagnosing cancer comprising the peptide or the multimer, a method for diagnosing cancer, the use of the peptide or the multimer for diagnosing cancer, and the use of the peptide or the multimer in the manufacture of a medicament for diagnosing cancer.

Another further object of the present invention is to provide a composition for preventing or treating cancer comprising the peptide, the conjugate or the multimer, a method for preventing or treating cancer, the use of the peptide, the conjugate or the multimer for preventing or treating cancer, and the use of the peptide, the conjugate or the multimer in the manufacture of a medicament for preventing or treating cancer.

To achieve the above objects, the present invention provides a peptide that specifically binds to nucleolin (NCL), the peptide comprising an amino acid sequence selected from the group consisting of:

(a) an amino acid sequence set forth in any one of SEQ ID NO: 1 to SEQ ID NO: 8, and
(b) an amino acid sequence comprising at least one amino acid mutation selected from the following group in the amino acid sequence set forth in SEQ ID NO: 1,

(i) a substitution for a methionine residue at position 5 from the N-terminus,
(ii) a substitution for a tyrosine residue at position 7 from the N-terminus, and
(iii) an insertion of a leucine or lysine residue at the C-terminus.

The present invention also provides a conjugate comprising the peptide and an anticancer agent.

The present invention also provides a multimer comprising the peptide.

The present invention also provides a conjugate comprising the multimer and an anticancer agent.

The present invention also provides a fusion peptide in which the peptide and a cell-penetrating peptide (CPP) are fused together.

The present invention also provides a fusion peptide in which the multimer and a cell-penetrating peptide are fused together.

The present invention also provides a composition for diagnosing cancer comprising the peptide or the multimer, a method for diagnosing cancer, the use of the peptide or the multimer for diagnosing cancer, and the use of the peptide or the multimer in the manufacture of a medicament for diagnosing cancer.

The present invention also provides a composition for preventing or treating cancer comprising the peptide, the conjugate or the multimer, a method for preventing or treating cancer, the use of the peptide, the conjugate or the multimer for preventing or treating cancer, and the use of the peptide, the conjugate or the multimer in the manufacture of a medicament for preventing or treating cancer.

## Brief Description of Drawings

FIG. 1 shows the structures of a monomer (AGM-330m), dimer (AGM-330d) and tetramer (AGM-330t) of a conjugate in which AGM-330 according to the present invention and PEG are conjugated together.
FIG. 2 shows the structure of an AGM-330-CPP fusion peptide according to the present invention in which an AGM-330-PEG conjugate and a cell-penetrating peptide (CPP) are conjugated together.
FIG. 3 is a schematic view showing peptide library synthesis and screening steps: 1. The split-mix synthesis method is performed to construct the combinatorial OBOC libraries; 2. Approximately 2,600,000 libraries are synthesized; 3. OBOC libraries are incubated with cancer cells in a $CO_2$ incubator; 4. Beads carrying ligands with an affinity for cell surface molecules become covered with cells; 5. The positive beads are picked with a pipette under an inverted microscope; 6. The peptide sequences for positive beads are determined by Edman microsequencing; 7. Cancer-specific peptide ligand candidates are identified by screening OBOC combinatorial peptide libraries.
FIG. 4 shows screening of OBOC libraries and MAP synthesis for cancer-specific ligands.
FIG. 4A shows the binding specificities of peptides against cancer cell and normal cells. FIG. 4B shows whole-cell binding assay results showing the cell binding specificity of 8 selected beads. Here, multiple breast and colorectal cancer cells and normal breast and colorectal cells were re-suspended at $10^6$ cells/ml and incubated with beads. All experiments were repeated 3 times, and the scale bar is 200 um. FIG. 4C shows the binding specificity determined

by confocal imaging of the immunofluorescence of FITC-labeled AGM-330, AGM-331, and AGM-332. Here, nuclei were stained with DAPI (blue), and scale bar is 50 μm. FIG. 4D shows the binding specificity of AGM-330. Here, a whole-cell binding assay was performed to determine the cell binding of AGM-330, and the scale bar is 200 μm. FIG. 4E shows a procedure for the synthesis of AGM-330, here, note for reagents and conditions: (i) Fmoc-Lys-(Fmoc)-OH, piperidine, DMF; (ii) Fmoc-Lys-(Fmoc)-OH, piperidine, DMF; (iii) RHGAMVYLK-OH, piperidine; (iv) piperidine, DMF. Fmoc = 9-fluorenylmethoxycarbonyl, DMF = dimethyl formamide. FIG. 4F shows FACS analysis showing the specificity of AGM-330 toward cancer cells among multiple breast and colorectal cancer cells and normal breast and colorectal cells. Here, bar graphs represent the mean ± SD, and statistical analyses were performed by one-way ANOVA with Dunnett's multiple comparison. *, **, and *** indicate $P < 0.05$, $P < 0.01$, and $P < 0.001$, respectively.

FIG. 5 shows the results of evaluating the *in vivo* stability of AGM-330 peptides (AGM-330m, AGM-330d, AGM-330t) . After injection of 2 mg/kg of AGM-330 through the tail vein of mice, blood samples were collected at 0, 0.167, 1, 2, 4, 8, 12, 24 and 48 hours from one eye using heparinized capillary tubes. After allowing for blood coagulation at 4°C, serum was collected by centrifugation at 5,000 rpm for 10 min at 4°C. The remaining peptide portion in the serum was immediately immobilized on an ELISA plate using an anti-AGM-330 antibody. Absorbance was measured at a wavelength of 450 nm using a VersaMax ELISA plate reader (Molecular Devices), and pharmacokinetics was analyzed using Phoenix WinNonlin 8.1 *(Pharsight* Corporation, Mountain View, CA, USA) program.

FIG. 6 shows the results of cell viability analysis upon treatment with AGM-330t or AGM-330d. Cancer and normal cells ($1 \times 10^4$ cells/well) were seeded in 96-well plates. After 24 h of incubation, the cells were treated with an increasing concentration of AGM-330t or AGM-330d for 48 hours with different serum concentrations. The viability of cells was assessed by a CellVia WST-1 assay (Young In Frontier) according to the manufacturer's instruction. The numbers of viable cells were measured at a wavelength of 450 nm using an VersaMax ELISA plate reader (Molecular Devices).

FIG. 7 shows characterization and identification of molecular targets of AGM-330.

FIG. 7A shows a schematic diagram of biotin pull-down assay, mass spectrometry analysis and Western blot. FIG. 7B shows Coomassie brilliant blue staining of the eluted proteins from affinity columns after separation by 10% SDS-PAGE. Lane 1: protein marker; Lane 2: total lysate before biotin pull-down assay; Lane 3: flow-through after incubation with AGM-330-biotin; Lanes 4-6: bead washing fraction; Lanes 7-8: elution of AGM-330 binding proteins. * Red star indicates that the protein band was excised for in-gel digestion followed by LC-MS/MS analysis. FIG. 7C shows protein database searches of peptides detected by MS identified NCL as an AGM-330 binding partner. FIG. 7D shows the list of genes obtained from LC-MS/MS analysis, here, the confidence score indicates the mascot score of the identified protein. FIG. 7E shows the results of immunoblotting analysis of the eluted proteins from the biotin pull-down assay using an anti-NCL antibody. Here, the arrow indicates the presence of NCL. FIG. 7F shows the results of analysis of the NCL domains binding to AGM-330. Here, different GFP-NCL constructs were transfected into HEK293T cells. AGM-330-biotin was added to NCL residues 1-710, residues 1-322, or residues 323-710 bound to streptavidin beads. Proteins on the beads were analyzed using immunoblotting with anti-GFP antibodies.

FIG. 8 shows the results of assessing AGM-330 affinity ($K_d$) for recombinant NCL by SPR. The SPR sensorgrams shown are from an experiment that is representative of a set of three separate experiments using different sensor chips with similar results. Recombinant NCL was immobilized onto a CM5 sensor chip. Various concentrations of AGM-330 (20 nM to 2.5 μM) were incubated with immobilized NCL and analyzed on a Biacore T-200 apparatus.

FIG. 9 shows the results of analyzing the direct interaction between AGM-330 and purified NCL by a biotin pull-down assay. Immunoblotting analysis of the eluted proteins forms the biotin pull-down using an anti-NCL antibody. Lane 1: protein marker; Lane 2: input of purified NCL; Lane 3: flow-through after incubated with AGM-330-biotin; Lanes 4-6: bead washing fraction; Lanes 7-8: elution of AGM-330 binding proteins. The arrow indicates the presence of NCL.

FIG. 10 shows the results of ELISA for the affinities of AGM-330 and its variants. Cancer cells ($1 \times 10^4$ cells/well) were seeded in 96-well plates and cultured for 24 hours, and then the cells were treated with increasing concentrations of 13 biotin-conjugated peptides for 2 hours. Then, avidin-HRP was attached through a high-affinity avidin-biotin interaction, and the TMB substrate was converted to blue through HRP enzyme. Then, the reaction was terminated by adding an acid, and the affinities of the 13 peptides were assessed by ELISA by reading the wells at 450 mm.

FIG. 11 shows the binding specificity of AGM-330 *in vitro* and *in vivo.*

FIGS. 11A and 11B show the results of fluorescence confocal microscopy of multiple cancer and normal cells. Here, MCF-10A, MCF-7, MDA-MB-231 (FIG. 11A) or CCD-18Co, HT-29, and HCT-116 (FIG. 11B) cells were incubated with 5 pmol/l AGM-330-FITC for 30 min at 37°C. NCL was stained by primary anti-NCL antibody, and the complex of AGM-330 and NCL was revealed using an anti-mouse secondary antibody coupled to Alexa Fluor 594. Cells were then fixed with 4% paraformaldehyde, and the nuclei were stained with DAPI. Merge represents AGM-330-FITC, NCL, and nuclear staining by DAPI. AGM-330-FITC represents FITC-conjugated AGM-330. Scale bar is 20 μm. FIG. 11C shows the results of analyzing the NCL distribution in various subcellular fractions in MCF-10A, MCF-7, and MDA-MB-231 cells. Here, plasma membrane, cytosol and nuclear NCL were immunoblotted using anti-NCL antibody. FIG. 11D shows that binding of AGM-330 to the cell membrane is inhibited by knockdown of NCL using siRNA. Here, MDA-MB-231 cells were transfected for 48 hours. Then, 5 pmol/l of AGM-330-FITC was added for 2 hours. Cells were washed twice with PBS and fixed with 4% paraformaldehyde. Fluorescence was observed using a fluorescent confocal microscope. AGM-330-FITC is FITC-conjugated AGM-330, and the scale bar is 20 $\mu$m. FIG. 11E shows the results of analyzing NCL distribution in various subcellular fractions in MCF-10A, MCF-7, and MDA-MB-231 cells after siNCL1 treatment. Here, plasma membrane, cytosol and nuclear NCL were immunoblotted using anti-NCL antibody. FIG. 11F shows a schematic representation of the experimental protocol. Here, anesthetized 6-week-old male NPG™ mice were inoculated with 1:1 mix of Matrigel and $1 \times 10^6$ MDA-MB-231-luc cells into the mammary fat pad. After tumor cell inoculation, when the tumor volume reached approximately 100 $mm^3$, free Alexa680 or AGM-330-Alexa680 was injected into the tail vein. FIGS. 11G and 11H show *in vivo* fluorescence images of mice 30 min, 1 h, 6 h, and 24 h after intravenous injections of 10 nmol free Alexa680 (FIG. 11G) or 10 nmol AGM-330-Alexa680 (FIG. 11H) in MDA-MB-231-luc tumor-bearing mice. Here, fluorescence images of the major organs including the heart, spleen, lung, brain, liver, kidney, and tumors were removed from mice with different treatments. AGM-330-FITC is FITC-conjugated AGM-330. FIGS. 11I and 11J are graphs showing the average radiant efficiency of Alexa680-associated fluorescence from live imaging of three animals in tumors (FIG. 11I) and different organs (FIG. 11J). Here, bar graphs represent the mean $\pm$ SD, and statistical analyses were performed by one-way ANOVA with Dunnett's multiple comparison. *, **, and *** indicate $P < 0.05$, $P < 0.01$, and $P < 0.001$, respectively.

FIG. 12 shows the results of analyzing the expression of NCL in membrane and cytosol extracts. MDA-MB-231 cells were transfected with non-targeting scrambled siRNA (Scr) or NCL-targeting siNCL1, siNCL2, or siNCL3. After 24 hours, the efficiencies of gene silencing were determined by real-time PCR. Based on the observed mRNA levels, siNCL1 was selected for further experiments as it showed significant efficiency on target gene knockdown. The bar graphs represent the means $\pm$ SD, and statistical analyses were performed using one-way ANOVA with Dunnett's multiple comparison. *, **, and *** indicate $P < 0.05$, $P < 0.01$, and $P < 0.001$, respectively.

FIG. 13 shows the results of expression profiling and functional study of NCL in breast and colorectal cancer.

FIG. 13A shows a significant correlation between tumor occurrence breast carcinoma or colorectal adenocarcinoma patients and the expression levels of NCL mRNA, which was observed in the Curtis and Alon datasets, which were obtained through the Oncomine dataset repository (www.oncomine.org). FIG. 13B shows the results of Kaplan-Meier survival analyses conducted in patients with breast and colorectal cancer based on NCL expression in two independent cohorts (Bertucci and Sveen). FIGS. 13C and 13D show the results of immunohistochemistry assay (IHC) for NCL in normal breast and breast cancer tissues (FIG. 13C) and normal colon and colon cancer tissues (FIG. 13D). Scale bar is 100 $\mu$m. FIGS. 13E to 13H show the viability of cells treated with the indicated amounts of anti-NCL antibody or IgG in serum-free media for 24 hours. Here, cell viability of MCF-10A (FIG. 13E), MDA-MB-231 (FIG. 13F), CCD-18Co (FIG. 13G), and HCT-116 (FIG. 13H) was measured by WST assay. Apoptotic cells were visualized by staining Annexin $V^+$ cells. Cells were neutralized with the anti-NCL antibody and incubated in serum-free media for 24 hours. Apoptotic cells were measured by FACS analysis. Bar graphs represent the mean $\pm$ SD, and statistical analyses were performed by one-way ANOVA with Dunnett's multiple comparison. *, **, and *** indicate $P < 0.05$, $P < 0.01$, and $P < 0.001$, respectively.

FIG. 14 shows the *in vivo* tumor suppression efficacy of AGM-330t-PTX.

FIG. 14A shows the results of cell proliferation inhibition by AGM-330-PTX treatment, determined for 48 hours by the WST assay in multiple breast and colorectal cancer cells and in normal cells. FIG. 14B is a schematic representation of the experimental protocol. Here, anesthetized 6-week-old male NPG mice were inoculated with a 1:1 mix of Matrigel and $1 \times 10^6$ MDA-MB-231-luc cells into the mammary fat pad. When the volume of primary tumors reached approximately 100 $mm^3$, tumor-bearing mice were treated with vehicle (PBS), AGM-330t (16.68 mg/kg), PTX (2 mg/kg or 10 mg/kg, respectively), or AGM-330-PTX (19.05 mg/kg (n = 6/group). FIGS. 14C to 14E show the therapeutic effect of AGM-330-PTX evaluated in breast cancer xenograft models. In FIG. 14C, primary tumor volume was measured twice a week until the day of sacrifice, and the primary tumor volume was calculated by the formula volume ($mm^3$) = (length (mm)) $\times$ (width (mm))$^2$ $\times$ 0.5. In FIG. 14D, on the day of sacrifice, all primary tumors were isolated. FIG. 14E shows the results of evaluating the primary tumor weights. FIGS. 14F to 14I show immunohistochemistry (IHC) sections of representative tumors with Ki-67 (FIGS. 14F and 14H) and TUNEL (FIGS. 14G and 14I) staining. Nuclei were stained with DAPI (blue), and the scale bar is 50 $\mu$m. Bar graphs represent the mean $\pm$ SD, and statistical analyses were performed by one-way ANOVA with Dunnett's multiple comparison. *, **, and *** indicate $P < 0.05$, $P < 0.01$, and $P < 0.001$, respectively.

FIG. 15 shows the results of confirming the improvement in efficacy PTX by the specificity of AGM-330t to cancer. Blood was collected from xenografted mice under isofluraneinduced deep anesthesia by cardiac puncture. After allowing for blood coagulation at 4°C, serum was collected by centrifugation at 3,000 rpm for 10 min at 4°C. Analysis of the levels of ALB (FIG. 15A), GOT (FIG. 15B), GPT (FIG. 15C), TP-PS (FIG. 15D), and UA (FIG. 15E) in the

serum was performed using a veterinary hematology analyzer (Fuji DRI-Chem 3500 s, Fujifilm, Tokyo, Japan) according to the manufacturer's protocols. ALB: albumin; GOT: glutamic oxaloacetic transaminase; GTP: glutamate pyruvate transaminase; TP-PS: total protein/protein scan; UA: uric acid. FIG. 15F shows body weight changes after 3 weeks of different treatments in xenograft mice. The bar graphs represent the means ± SD, and statistical analyses were performed using one-way ANOVA with Dunnett's multiple comparison. *, **, and *** indicate P < 0.05, P < 0.01, and P < 0.001, respectively.

FIG. 16 shows the results of evaluating the anticancer effect of AGM-330d-PTX. FIG. 16A shows the structure of AGM-330d-PTX. FIG. 16B is a schematic representation of the experimental protocol. Here, anesthetized 6 week-old male NPG mice were inoculated with a 1:1 mix of Matrigel and $1 \times 10^6$ MDA-MB-231-luc cells into the mammary fat pad. Mice bearing MDA-MB-231-luc cell tumors were treated with vehicle (PBS), AGM-330d (8.34 mg/kg), PTX (2 or 10 mg/kg, respectively) or AGM-330d-PTX (10.71 mg/kg) (n = 6/group). FIGS. 16C and 16D show the results of monitoring tumor growth through whole-body bioluminescence imaging. Here, growing MDA-MB-231 cells expressing firefly luciferase were injected into the mammary gland fat pad of mice. The therapeutic effect of AGM-330d-PTX was evaluated by bioluminescence imaging (FIG. 16C), and FIG. 16D shows bioluminescence intensity every 5 days in 5 different mouse groups. FIGS. 16E to 16G show the results of evaluating the therapeutic effect of AGM-330d-PTX in breast cancer xenograft models. On the day of sacrifice, all primary tumors were isolated and their volume was assessed (FIGS. 16E and 16F), and primary tumor weight was measured twice per week until the day of sacrifice (FIG. 16G). The primary tumor volume was calculated using the following formula: Volume ($mm^3$) = (length (mm)) $\times$ (width $(mm))^2$ X 0.5. FIG. 16H is a graph showing body weight changes after 3 weeks of different treatments indicated in xenograft mice. Bar graphs represent the means ± SD, and statistical analyses were performed using one-way ANOVA with Dunnett's multiple comparison. *, **, and *** indicate P < 0.05, P < 0.01, and P < 0.001, respectively.

FIG. 17 shows a process for synthesis of an AGM-330t-mCPP fusion peptide.

FIG. 18 shows the results of HPLC and MS analysis of the AGM-330t-mCPP fusion peptide, AGM-330t, and maleimide-CPP.

FIG. 19 depicts graphs showing the cytotoxic effects of AGM-330t, mCPP, and the AGM-330t-mCPP fusion peptide.

FIG. 20 shows the apoptosis effects of AGM-330t, mCPP and the AGM-330t-mCPP fusion peptide. Here, apoptotic cells were measured by FACS analysis. Bar graphs represent the mean ± SD, and statistical analyses were performed by one-way ANOVA with Dunnett's multiple comparison. *** indicates $P < 0.001$, and NS denotes not significant.

FIG. 21 shows the concentration-dependent apoptosisinducing effect of the AGM-330t-mCPP fusion peptide.

FIG. 22 depicts graphs showing the concentration-dependent cytotoxic effects of AGM-330m-mCPP, AGM-330d-dCPP and the AGM-330t-tCPP fusion peptide.

FIG. 23 depicts graphs showing the concentration-dependent cytotoxic effects of a combination of AGM-330m and AGM-330m-mCPP, a combination of AGM-330d and AGM-330d-dCPP, and a combination of AGM-330t and AGM-330t-tCPP.

**Detailed Description and Preferred Embodiments of the Invention**

Unless otherwise defined, all technical and scientific terms used in the present specification have the same meanings as commonly understood by those skilled in the art to which the present disclosure pertains. In general, the nomenclature used in the present specification is well known and commonly used in the art.

In one aspect, the present invention is directed to a peptide that specifically binds to nucleolin (NCL), the peptide comprising an amino acid sequence selected from the group consisting of:

(a) an amino acid sequence set forth in any one of SEQ ID NO: 1 to SEQ ID NO: 8, and
(b) an amino acid sequence comprising at least one amino acid mutation selected from the following group in the amino acid sequence set forth in SEQ ID NO: 1,

(i) a substitution for a methionine residue at position 5 from the N-terminus,
(ii) a substitution for a tyrosine residue at position 7 from the N-terminus, and
(iii) an insertion of a leucine or lysine residue at the C-terminus.

The discovery of peptide ligands that can functionally and specifically target tumors offers a new opportunity in cancer diagnosis and treatment. However, the use of peptide ligands has largely been limited by their short biological half-life.

In the present invention, about 2,600,000 peptide libraries were synthesized using a one-bead-one-compound (OBOC) combinatorial method combined with a multiple-antigen-peptide (MAP) synthesis method. From the peptide

libraries, AGM-330, AGM-331, AGM-332, AGM-333, AGM-334, AGM-335, AGM-336, and AGM-337, which are the new cancer-specific peptide ligands having the amino acid sequences shown in Table 1 below were identified (see FIG. 3). Using cell binding assay, flow cytometry and fluorescence confocal microscopy, it was confirmed that the peptide ligands specifically bound to cancer cells *in vitro* and *in vivo.*

In addition, through pull-down assay and LC-MS/MS analysis, it was confirmed that membrane nucleolin (NCL) was the target protein of AGM-330, AGM-331, AGM-332, AGM-333, AGM-334, AGM-335, AGM-336, or AGM-337. In addition, it was confirmed that treatment with a conjugate of paclitaxel (PTX) and AGM-330 dramatically inhibited cancer cell growth compared to treatment with PTX alone.

[Table 1]

| **Name** | **Amino acid sequence** | **SEQ ID NO.** |
|---|---|---|
| AGM-330 | RHGAMVYLK | 1 |
| AGM-331 | ADHRHRRSG | 2 |
| AGM-332 | AVARARRRR | 3 |
| AGM-333 | RFLKNKKAR | 4 |
| AGM-334 | RWLKNKKAR | 5 |
| AGM-335 | FGRLKKPLK | 6 |
| AGM-336 | KRRRRERAG | 7 |
| AGM-337 | KRRRKAPTD | 8 |

Three of the eight peptides (AGM-330, AGM-331, and AGM-332) showed strong, specific and preferential binding to the breast cancer cell line (MDA-MB-231) and showed no or only weak binding to the human normal breast cell line (MCF-10A) (see FIGS. 4A and 4B).

In addition, it was confirmed that, even when AGM-330 having the amino acid sequence of SEQ ID NO: 1 has mutations of some amino acid residues, it still showed strong and specific binding properties to nucleolin.

Specifically, in the present invention, the amino acid mutation may be at least one mutation selected from the group consisting of:

(i) a substitution for a methionine residue at position 5 from the N-terminus;
(ii) a substitution for a tyrosine residue at position 7 from the N-terminus; and
(iii) an insertion of a leucine or lysine residue at the C-terminus; in the amino acid sequence of SEQ ID NO: 1, but not limited thereto.

Preferably, the amino acid mutation may be at least one mutation selected from the group consisting of:

(i) a substitution of leucine or norleucine for a methionine residue at position 5 from the N-terminus;
(ii) a substitution of phenylalanine for a tyrosine residue at position 7 from the N-terminus; and
(iii) an insertion of a leucine or lysine residue at the C-terminus; in the amino acid sequence of SEQ ID NO: 1, but not limited thereto.

In the present invention, the amino acid sequence comprising the mutation may be selected from the group consisting of SEQ ID NO: 9 to SEQ ID NO: 15 and SEQ ID NO: 20, but not limited thereto (see Table 2).

[Table 2]

| **Amino acid mutation in AGM-330** | **Amino acid sequence** | **SEQ ID NO.** |
|---|---|---|
| R1 → K1 | **K**HGAMVYLK | 9 |
| H2 → R2 | R**R**GAMVYLK | 10 |
| A4 → L4 | RHG**L**MVYLK | 11 |
| M5 → L5 | RHGA**L**VYLK | 12 |
| M5 → Nle5 (Nle: Norleucine) | RHGA(**Nle**)VYLK | 13 |

(continued)

| Amino acid mutation in AGM-330 | Amino acid sequence | SEQ ID NO. |
|---|---|---|
| V6 → A6 | RHGAM**A**YLK | 14 |
| Y7 → F7 | RHGAMV**F**LK | 15 |
| R1H2 → Deletion | GAMVYLK | 16 |
| R1H2 → Insertion | **RH**RHGAMVYLK | 17 |
| R1H2R3H4 → Insertion | **RHRH**RHGAMVYLK | 18 |
| Reverse sequence | **KLYVMAGHR** | 19 |
| L10K11L12K13 → Insertion | RHGAMVY**LKLK**LK | 20 |

In the amino acid sequence of SEQ ID NO: 13, "Nle" denotes norleucine.

In the present invention, "AGM", "AGM peptide" or "AGM peptide ligand" refers to a peptide having an amino acid sequence selected from the group consisting of SEQ ID NO: 1 to SEQ ID NO: 20, or at least one peptide among the peptides having the amino acid sequences of SEQ ID NO: 1 to SEQ ID NO: 20.

In the present invention, the AGM peptide may specifically bind to nucleolin (NCL).

Although NCL is predominantly localized in the nucleus of normal cells, previous studies showed enhanced overexpression of membrane NCL in many different types of cancer cells (Shen N, et al. Oncotarget. 2014; 5: 5494-509). Consistent with these studies, the present invention demonstrated that NCL is overexpressed on the membrane of actively proliferating cancer cells, but not in normal cells. Accordingly, the use of molecules targeting NCL may be an effective approach for the selective delivery of drugs to tumors while minimizing side effects (Li F, et al. Nat Commun. 2017; 8: 1390). In addition, the present invention demonstrated the role of membrane NCL on cancer cells through neutralization with specific antibodies. As expected, the rate of apoptosis increased, followed by antibody-mediated NCL neutralization (FIG. 13J). In conclusion, NCL is an ideal therapeutic marker for cancer treatment, and NCL-targeted AGM-330 has great potential as a tool for diagnosis and treatment of a wide range of human cancers.

In one example of the present invention, the AGM peptide, particularly AGM-330, specifically bound to cancer cells but bound weakly or not at all to normal breast and colorectal cells. These results suggest that AGM-330 is cancer-specific, making it a prime candidate for the development of cancer-targeting peptides. In particular, in a mouse xenograft study, AGM-330 treatment showed more accumulation in tumors than in the heart, spleen, lung or brain. The specific targeting ability of AGM-330 to cancer cells provides a potential molecular tool for the diagnosis of cancer.

The present invention is also directed to an AGM peptide-PEG conjugate in which the AGM peptide and a polyethylene glycol (PEG) chain comprising at least two ethylene glycol groups, each having a structure of $-[CH_2-CH_2-O]-$, are conjugated together.

In the present invention, the polyethylene glycol chain is used as a concept including not only a general polyethylene glycol chain having a structure of Formula 1, but also derivatives thereof. Examples of derivatives of the polyethylene glycol chain include, but are not limited to, an amino polyethylene glycol carboxylic acid having a structure of Formula 2.

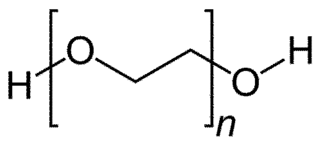

Formula 1 (Polyethylene glycol)

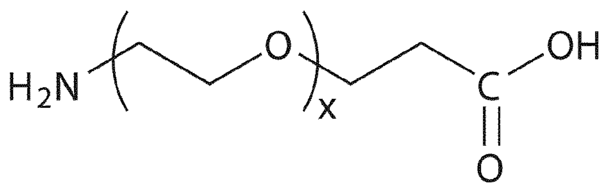

Formula 2 (Amino polyethylene glycol carboxylic acid)

Conjugation with polyethylene glycol (PEG), known as PEGylation, has been widely used to improve the pharmacological properties of therapeutic proteins (Gupta V, et al. J Cell Commun Signal. 2019; 13: 319-30) . In the present invention, PEGylation was used to decrease steric hindrance and increase hydrophilicity. However, PEG has been burdened by immunogenicity, which has a negative clinical effect on therapeutic molecules (Moreno A, et al. Cell Chem. Biol. 2019; 26: 634-44.e3). A previous study suggested that the anti-PEG immune response to the PEGylated molecules depended on the immunogenicity of the extent of PEGylation and the molecular weight of PEG (Wan X, et al. Process Biochem. 2017; 52: 183-91).

In the present invention, the polyethylene glycol chain may have 2 to 24 ethylene glycol units, preferably 4 to 20, more preferably 6 to 18, most preferably 6 to 12 ethylene glycol units, but is not limited thereto.

The polyethylene glycol chain may be linked to the AGM peptide by a linker. Accordingly, the AGM peptide-PEG conjugate may have a structure represented by the following Structural Formula 1, but is not limited thereto.

AGM-$L_1$-PEG (Structural Formula 1)

In Structural Formula 1, PEG represents a polyethylene glycol chain, and $L_1$ represents a linker.

The linker $L_1$ is preferably selected from the group consisting of a single bond (direct bond), $C_1$-$C_{20}$ alkyl, $C_1$-$C_{20}$ alkylene, -S-, -NH- and -O-, but is not limited thereto, and may include a peptide bond that forms -CONH- with the carboxyl group of the C-terminal amino acid of the AGM peptide, but is not limited thereto.

In addition, a functional group for conjugating another substance, preferably a drug, may be introduced at the other end of the PEG conjugated to the AGM in the AGM peptide-PEG conjugate of Structural Formula 1. Examples of the functional group include, but are not limited thereto, a carboxyl group (COOH), an amine group ($NH_2$), and a thiol group.

For example, as shown in Structural Formulas 2 to 4, the AGM peptide-PEG conjugate may be in a form in which lysine (K) having an amine ($NH_2$) and/or cysteine (C) having a thiol group are/is conjugated to the other end of the PEG conjugated to the AGM peptide, but is not limited thereto.

AGM-$L_1$-PEG-K (Structural Formula 2)

AGM-$L_1$-PEG-C (Structural Formula 3)

AGM-$L_1$-PEG-K-C (Structural Formula 4)

Illustratively, in one embodiment of the present invention, an AGM-330 monomer (AGMm), in which lysine and tyrosine are introduced at the other end of the PEG conjugated to the AGM in the AGM-330-PEG conjugate, may have a structure represented by Structural Formula 5.

RHGAMVYLK-$L_1$-PEG-K-C (Structural Formula 5)

In Structural Formula 5 above, PEG may be linked to the C-terminus of AGM-330 by a linker ($L_1$) including a peptide bond as shown in Structural Formula 6 through condensation between the carboxyl group of the C-terminal lysine and the amine, but is not limited thereto.

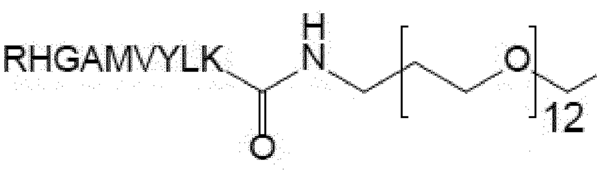

(Structural Formula 6)

In another aspect, the present invention is directed to an AGM peptide-drug conjugate or AGM peptide-PEG-drug conjugate comprising the AGM peptide or AGM peptide-PEG conjugate and a drug.

In the present invention, the drug is an agent exhibiting a pharmacological effect, and specifically, may be a chemotherapeutic agent, a toxin, micro-RNA (miRNA), siRNA, shRNA, or a radioactive isotope. The chemotherapeutic agent may be, for example, a cytotoxic agent or an immunosuppressive agent. Specifically, it may include chemotherapeutic agent capable of functioning as a microtubulin inhibitor, a mitotic inhibitor, a topoisomerase inhibitor, or a DNA intercalator. In addition, examples of the chemotherapeutic agent include an immunomodulatory compound, an anticancer agent and an antiviral agent, or combinations thereof.

Preferably, the drug may be an anticancer agent. The anticancer agent may be at least one selected from the group consisting of taxols and taxanes such as paclitaxel or docetaxel, maytansinoids, auristatin, aminopterin, actinomycin, bleomycin, thalidomide, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, alpha-amanitin, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, pyrrolobenzodiazepine, carmustine, lomustine, busulfan, treosulfan, dacarbazine, etoposide, teniposide, topotecan, 9-aminocamptothecin, crisnatol, mitomycin C, trimetrexate, mycophenolic acid, tiazofurin, ribavirin, 5-ethynyl-1-beta-ribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, berberine, verteporfin, curcumin, Abraxane, FOLFIRINOX, oxaliplatin, Xeloda and indole carboxamide, phthalocyanine, tubulysin, photosensitizer Pe4, demethoxy-hypocrellin A, interferon-a, interferon-$\gamma$, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants, but is not limited thereto.

In one example of the present invention, in a human breast cancer xenograft mouse model, treatment with AGM-330-PTX (a conjugate of the AGM-330-PEG conjugate and paclitaxel (PTX)) resulted in 60% and 46% decreases in the volume and weight, respectively, of tumors compared with those observed in the PTX-treated group (see FIGS. 14C to 14E). The efficient targeting ability of AGM-330 to cancer cells and tissues provides a promising way to deliver anti-cancer drugs for cancer treatment.

Paclitaxel (PTX) is considered one of the most promising cancer chemotherapy drugs and has been tested against many different human malignancies (Ramalingam S, Belani CP. Expert Opin. Pharmacother. 2004; 5: 1771-80). However, treatment with PTX has been impeded by the high hydrophobicity of PTX and related difficulties with its formulation. In addition, many side effects associated with PTX treatment have also been ascribed to the dilution solvents used in the formulation (Hennenfent KL, Govindan R. Ann Oncol. 2006; 17: 735-49). However, unlike PTX, PTX conjugated with the AGM peptide, particularly the AGM peptide-PEG conjugate, is highly hydrophilic. This hydrophilicity may improve its pharmacokinetic profile and enable the use of smaller amounts of toxic dilution buffers, thus further decreasing the general toxicity of chemotherapy. AGM peptide-mediated targeted delivery has advantages in that it may reduce side effects such as toxicity and significantly improve therapeutic effects.

In the present invention, the AGM peptide or the AGM peptide-PEG conjugate may be linked to a drug, preferably an anticancer agent, directly or by a linker ($L_d$).

The linker ($L_d$) may be cleavable. The cleavable linker ($L_d$) allows the drug to be released from the antibody through cleavage of the linker in a form that is cleavable under intracellular conditions, that is, in the intracellular environment.

The linker ($L_d$) may be cleaved by cleaving agents present in the intracellular environments, for example, lysosomes or endosomes. For example, it may be a cleavable peptide linker that may be cleaved by, for example, intracellular peptidases or proteases such as lysosomes or endosomal proteases. In general, the peptide linker is at least two amino acids in length. The cleaving agents may include cathepsin B, cathepsin D, and plasmin, and hydrolyze the peptide to allow the drug to be released into the target cell.

The peptide linker may be cleaved by the thiol-dependent protease cathepsin-B, which is overexpressed in cancer tissues. For example, a Phe-Leu or Gly-Phe-Leu-Gly linker may be used. In addition, the peptide linker may be, for example, a Val-Cit linker or a Phe-Lys linker, which may be cleaved by intracellular proteases.

In one embodiment, the cleavable linker is a pH-sensitive linker which may be sensitive to hydrolysis at certain pH values. In general, the pH-sensitive linker may be hydrolyzed under acidic conditions. Examples thereof include acid-labile linkers that may be hydrolyzed in lysosomes, such as hydrazone, semicarbazone, thiosemicarbazone, cis-aconitic amide, orthoester, acetal, ketal, and the like.

In another embodiment, the linker may be, for example, a disulfide linker which may be cleaved under reducing

conditions. Various disulfide bonds may be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alpha-methyl-alpha-(2-pyridyl-dithio)toluene) .

In addition, the linker may be, for example, a noncleavable linker. In this case, the drug is released through only one step (antibody hydrolysis) to produce, for example, an amino acid-linker-drug conjugate. This type of linker may be a thioether group or a maleimidocaproyl group, which may maintain stability in blood.

In some cases, the AGM peptide or the AGM peptide-PEG conjugate may be linked to a drug, preferably an anticancer agent, directly or through a non-covalent bond or a covalent bond, but is not limited thereto.

The non-covalent bond may be, for example, at least one selected from the group consisting of a hydrogen bond, an electrostatic interaction, a hydrophobic interaction, a van der Waals interaction, a pi-pi interaction, and a cation-pi interaction.

In another aspect, the present invention is directed to a multimer comprising two or more AGM peptides or AGM peptide-PEG conjugates.

In the present invention, the multimer may comprise 2 to 8, preferably 2 to 6, more preferably 2 to 4 AGM peptides or AGM peptide-PEG conjugates, but is not limited thereto.

For example, in the present invention, the multimer may be a dimer in which two AGM peptides or AGM peptide-PEG conjugates are linked together by a linker ($L_2$), or the multimer may be a tetramer in which the dimers are linked together by an additional linker ($L_3$), but are not limited thereto. Furthermore, it is apparent to those skilled in the art that multimers such as a hexamer, an octamer and higher oligomers may be obtained through linkage similar to the above-described linkage.

For example, the dimer and tetramer of the AGM peptide-PEG conjugate according to the present invention may have structures represented by Structural Formulas 7 and 8, respectively, but are not limited thereto.

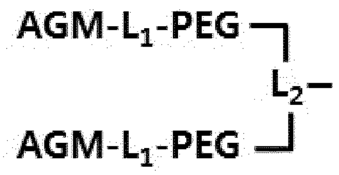

(Structural Formula 7)

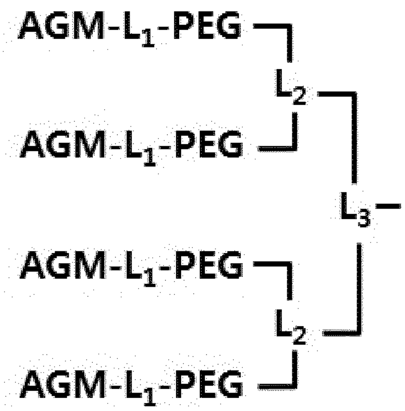

(Structural Formula 8)

The linkers $L_2$ and $L_3$ may be the same as or different from each other, and may be independently cleavable or noncleavable.

The linkers $L_2$ and $L_3$ may be each independently selected from the group consisting of a single bond (direct bond), amino acids or derivatives thereof, $C_1$-$C_{20}$ alkyl, $C_1$-$C_{20}$ alkylene, -S-, -NH- and -O-, but are not limited thereto. Preferably, the linkers may be each independently an amino acid or a derivative thereof, more preferably lysine (Lys, K) or arginine (Arg, R) having at least two amine groups, but are not limited thereto.

Most preferably, the linkers $L_2$ and $L_3$ may be each independently lysine (Lys, K), and in this case, the amine group ($NH_2$) of the lysine may be bound to PEG in a form similar to a peptide bond such as -C-CONH-, but is not limited thereto.

In addition, a functional group for conjugating another substance, preferably a drug, may be introduced at one end of the linker of the AGM peptide or the AGM peptide-PEG conjugate. For example, a functional group for conjugating a drug may be introduced to $L_2$ in Structural Formula 7 or to $L_3$ in Structural Formula 8.

Examples of the functional group include, but are not limited to, an amine group ($NH_2$) and a thiol group. For

example, lysine (K) having an amine ($NH_2$) and/or cysteine (C) having a thiol group may be introduced as the functional group, but are no limited thereto.

For example, in one embodiment of the present invention, the AGM-330 dimer (AGM-330d) and the AGM-330 tetramer (AGM-330t) may have structures in Table 3 below, but are not limited thereto.

[Table 3]

| Name | Structural Formula |
|---|---|
| AGM-330d | 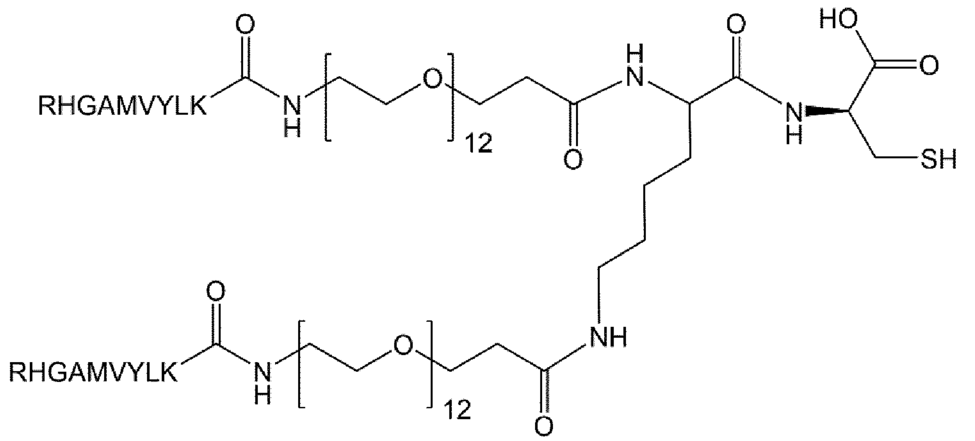 |
| AGM-330t | 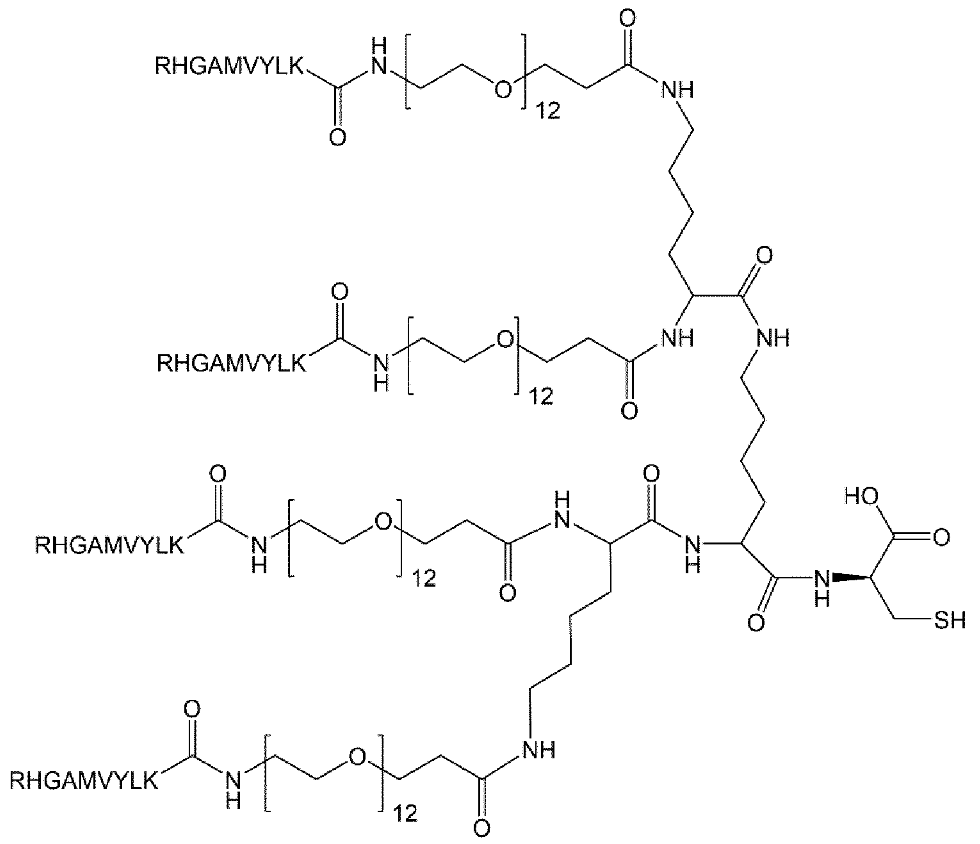 |

In one example of the present invention, anticancer efficacy was tested using AGM-330d-PTX (FIG. 16A). As a result, AGM-330d-PTX-treated mice showed a dramatic reduction in luciferase activity compared with that observed in PTX-treated mice (FIGS. 16C and 16D). Additionally, AGM-330d-PTX treatment resulted in a significant decrease in the volume and weight of tumors compared with that observed in the PTX-treated group (FIG. 16E to 16G). Consistent with the results obtained using AGM-330t-PTX, mouse body weights were not affected by AGM-330d-PTX administration compared to that observed in the vehicle (PBS) -treated mice (FIG. 16H). The cancer specificity of AGM-330d might

have enhanced the efficacy of PTX, increasing the therapeutic drug concentration in tumor tissues.

In another aspect, the present invention is directed to a multimer-drug conjugate comprising: a multimer comprising two or more AGM peptide-PEG conjugates; and a drug, preferably an anti-cancer agent.

In the present invention, for the multimer-drug conjugate, the description of the AGM peptide-drug conjugate or the AGM peptide-PEG-drug conjugate may be equally applied, and thus each linker or drug is as previously defined with respect to the AGM peptide-drug conjugate or AGM peptide-PEG-drug conjugate.

In another aspect, the present invention is directed to an AGM peptide-PEG-CPP fusion peptide in which the AGM peptide-PEG conjugate and a cell-penetrating peptide (CPP) are fused together.

As used herein, the term "cell-penetrating peptide (CPP) " is a kind of signal peptide, which is a combination of specific amino acids which is used for the purpose of intracellularly delivering high-molecular-weight substances such as proteins, DNA, RNA, and the like. Until now, cell-penetrating peptides have been used for intracellular delivery of various low molecular weight compounds, or high molecular weight substances such as proteins, peptides, RNA and DNA. Most cell-penetrating peptides are derived from protein-transduction domains or membrane-translocating sequences. Cell-penetrating peptides move into cells without damaging the cell membrane, unlike the normal intracellular entry route of foreign substances, and are expected to play an innovative role in delivering even DNA or proteins, which are known not to pass through the cell membrane.

The fusion peptide of the present invention uses a cell-penetrating peptide. The cell-penetrating peptide is not particularly limited as long as it has the property of entering the cell by the cell endocytosis mechanism. Preferably, the cell-penetrating peptide that is used in the present invention may be selected from the group consisting of the cell-penetrating peptides listed in Table 4 below or variants thereof.

[Table 4]

| Peptide | References | Sequence | SEQ ID NO. |
|---|---|---|---|
| TAT | Takeshima et al., (2003) J. Biol. Chem. 278(2), 1310-1315 | YGRKKRRQRRR | 31 |
| $TAT_{(48-60)}$ | Dennison et al., (2007) Biochem. and Biophy. Res. Comm. 363, 178 | GRKKRRQRRRPPQ | 32 |
| $TAT_{(49-57)}$ | Baoum et al., (2012) Int. J. Pharm. 427, 134-142 | RKKRRQRRR | 33 |
| Penetratin | Derossi et al., (1994) J. Biol. Chem. 269 (14), 10444-10450 | RQIKIWFQNRRMKWKK | 34 |
| R8 | Chu et al., (2015) Nanotechnol. Biol. Med. 11, 435-446 | RRRRRRRR | 35 |
| R9-TAT | Futaki et al., (2001) J. Biol. Chem. 276, 5836-5840 | GRRRRRRRRRPPQ | 36 |
| Pep-1 | Deshayes et al., (2008) Advanced Drug Delivery Reviews, 60, 537-547 | KETWWETWWTEWSQPKKKRKV | 37 |
| Hph-1 | Choi et al., (2006) Nat. Med. 12, 574-579. | YARVRRRGPRR | 38 |
| MAP | Wada et al., (2013) Bioorg. Med. Chem. 21, 7669-7673 | KLALKLALKALKAALKLA | 39 |
| pVEC | Elmquist et al., (2006) Biochim. Biophys. Acta. 1758, 721-729 | LLIILRRRIRKQAHAHSK | 40 |

(continued)

| Peptide | References | Sequence | SEQ ID NO. |
|---|---|---|---|
| MPG | Simeoni, (2003) Nucleic Acids Res. 31, 2717-2724 | GALFLGFLGAAG STMGAWSQPKKK RKV | 41 |
| Transporta n | Pae et al., (2014) J. Controlled Release, 192, 103-113 | GWTLNSAGYLLG KINLKALAALAK KIL | 42 |
| gH625 | Galdiero et al., (2015) Biochim. Biophys. Acta (BBA) Biomembr. 1848, 16-25. | HGLASTLTRWAH YNALIRAF | 43 |
| VP22 | Elliott and O'Hare (1997) Cell, 88, 223-233 | NAKTRRHERRRK LAIER | 44 |

More preferably, the cell-penetrating peptide may be selected from the group consisting of the cell-penetrating peptides listed in Table 5 below or variants thereof. For the synthesis and properties of the peptides shown in Table 5, reference may be made to Korean Patent No. 10-1169030. Most preferably, the cell-penetrating peptide may comprise the amino acid sequence of SEQ ID NO: 48.

[Table 5]

| Peptide | Sequence | SEQ ID NO. |
|---|---|---|
| DS4 | VQIFRIMRILRILKLARHST | 45 |
| DS4-1 | RIMRILRILKLARHST | 46 |
| DS4-2 | VQIFRIMRILRILKLAR | 47 |
| DS4-3 | RIMRILRILKLAR | 48 |
| KS4 | FRLVRLLRFLRILLIIS | 49 |
| KS4-1 | FRLVRLLRFLRILL | 50 |
| KS4-2 | RLVRLLRFLR | 51 |
| N1 | RIL | 52 |
| N3 | RILRILRIL | 53 |
| N5 | RILRILRILRILRIL | 54 |
| N7 | RILRILRILRILRILRILRIL | 55 |
| K3 | RIFWVIKLARHFI | 56 |
| C3 | KSLRVLRVLRPLKTIK | 57 |
| C4 | RLFRVMRLVKLLSRG | 58 |
| S2 | RSFRLLRVFKLAKSW | 59 |
| S4 | RVIRLARIGRILRLVKGAKGIR | 60 |
| D1 | RAGRILRILKLAR | 61 |

(continued)

| Peptide | Sequence | SEQ ID NO. |
|---|---|---|
| D2 | RIMRGLRILKLAR | 62 |
| D3 | RIMRILRLMKLAR | 63 |
| D4 | RIMRILRILKMFR | 64 |
| D5 | RIMRILRALKLAR | 65 |
| D6 | RIMRGMRILKLAR | 66 |
| D7 | RLFRILRILKLAR | 67 |
| D8 | RIMRMVRILKLAR | 68 |
| D9 | RIMRILRLVKLAR | 69 |
| D10 | RIMRILRILKGVR | 70 |
| D11 | RNLRILRILKLAR | 71 |
| D12 | RIMRDIRILKLAR | 72 |
| D13 | RIMRILRELKLAR | 73 |
| D14 | RIMRILRILKQLR | 74 |
| D15 | RIMRILRHMKLAR | 75 |
| D16 | RIMRSVRILKLAR | 76 |
| D17 | RTMRILRILKLAR | 77 |
| D18 | RIMRYARILKLAR | 78 |
| D19 | RIMRILRQIKLAR | 79 |
| D20 | RIMRILRILKEVR | 80 |

In an example of the present invention, the cell-penetrating peptide of SEQ ID NO: 48 indicated as the DS4-3 peptide in Table 5 was selected and used in an experiment. However, it will be apparent to those skilled in the art that effects similar to those of the present invention appear even when a cell-penetrating peptide other than the actually used cell-penetrating peptide is fused to the peptide of the present invention.

In the present invention, the AGM peptide-PEG conjugate and the cell-penetrating peptide may be linked together through a linker. Preferably, they may be linked together through a maleimide-carboxy bifunctional linker, but is not limited thereto.

In another aspect, the present invention is directed to a multimer-CPP fusion peptide in which a multimer comprising two or more AGM peptide-PEG conjugates, and a cell-penetrating peptide (CPP), are linked together.

In the present invention, for the multimer-CPP fusion peptide, the description of the AGM peptide-PEG-CPP fusion peptide may be equally applied, and thus each linker or the cell-penetrating peptide is as previously defined with respect to the AGM peptide-PEG-CPP fusion peptide.

In another aspect, the present invention is directed to a composition for diagnosing cancer comprising the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide.

In another aspect, the present invention is directed to a method of diagnosing cancer using the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide.

In another aspect, the present invention is directed to the use of the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide for diagnosing cancer.

In another aspect, the present invention is directed to the use of the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide in the manufacture of a medicament for diagnosing cancer.

Cancer whose onset or likelihood of onset may be predicted using the diagnostic composition of the present invention includes leukemia, myeloproliferative disease, lymphoma, breast cancer, liver cancer, stomach cancer, ovarian

cancer, cervical cancer, glioma cancer, colorectal cancer, lung cancer, pancreatic cancer, prostate cancer, liver cancer, gastric adenocarcinoma, uterine cancer, bladder cancer, thyroid cancer, melanoma, squamous cell carcinoma, hematopoietic cancer, kidney cancer, and head and neck cancer, but are not limited to.

As used herein, the term "diagnosis" includes determining susceptibility of a subject to a specific disease or disorder, determining whether a subject has a specific disease or disorder at present, determining the prognosis of a subject having a specific disease or disorder (e.g., identification of pre-metastatic or metastatic cancerous conditions, determination of the stage of cancer or determination of the responsiveness of cancer to treatment), or therametrics (e.g., monitoring the status of a subject state to provide information about the therapeutic efficacy) . For the purpose of the present invention, the term diagnosis refers to confirming the onset or likelihood (risk) of onset of the above-described disease.

In another aspect, the present invention is directed to a composition for preventing or treating cancer comprising the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide.

In another aspect, the present invention is directed to a method for preventing or treating cancer comprising administering to a subject the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide.

In another aspect, the present invention is directed to the use of the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide for preventing or treating cancer.

In another aspect, the present invention is directed to the use of the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide in the manufacture of a medicament for preventing or treating cancer.

Cancer or carcinoma that may be treated with the composition of the present invention is not particularly limited, and may be nucleolin-related cancer such as solid cancer or leukemia. Examples of such cancer include leukemia, myeloproliferative disease, lymphoma, breast cancer, liver cancer, stomach cancer, ovarian cancer, cervical cancer, glioma cancer, colorectal cancer, lung cancer, pancreatic cancer, prostate cancer, liver cancer, gastric adenocarcinoma, uterine cancer, bladder cancer, thyroid cancer, melanoma, squamous cell carcinoma, hematopoietic cancer, kidney cancer, and head and neck cancer, but are not limited thereto.

In the present invention, the terms "cancer" and "tumor" are used interchangeably and refer to or describe a physiological condition in mammals that is typically characterized by uncontrolled cell growth/proliferation.

As used herein, the term "prevention" refers to any action of inhibiting or delaying cancer by administering a composition comprising the peptide or conjugate. In addition, the term "treatment" refers to any action of alleviating or curing symptoms of cancer by administering a composition comprising the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide.

The composition for preventing or treating cancer according to the present invention may comprise a pharmaceutically effective amount of the AGM peptide, AGM peptide-PEG conjugate, AGM peptide-PEG-drug conjugate, multimer, multimer-drug conjugate, AGM peptide-PEG-CPP fusion peptide or multimer-CPP fusion peptide alone or together with at least one pharmaceutically acceptable carrier, excipient or diluent. As used herein, the term "pharmaceutically effective amount" refers to an amount sufficient to prevent, ameliorate and treat symptoms of cancer.

As used herein, "pharmaceutically acceptable" refers to an additive which is physiologically acceptable and, when administered to human beings, does not cause allergic reactions such as gastrointestinal disorders and dizziness, or similar reactions. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. In addition, the composition may further comprise a filler, an antiaggregating agent, a lubricant, a wetting agent, a flavoring agent, an emulsifier and a preservative. Suitable pharmaceutically acceptable carriers and agents are described in detail in Remington’s Pharmaceutical Sciences (19th ed., 1995).

In addition, the composition of the present invention may comprise at least one known active ingredient having a cancer therapeutic effect, together with the AGM peptide, AGM peptide-PEG conjugate, AGM peptide-PEG-drug conjugate, multimer, multimer-drug conjugate, AGM peptide-PEG-CPP fusion peptide or multimer-CPP fusion.

The pharmaceutical composition of the present invention may be formulated using a method known in the art so as to provide quick, sustained or delayed release of the active ingredient after administration to mammals other than human beings. The composition may be in the form of powder, granule, tablet, emulsion, syrup, aerosol, soft or hard gelatin capsule, sterilized injection solution, or sterilized powder.

The composition of the present invention may be administered via several routes including oral, transdermal, subcutaneous, intravenous or intramuscular routes. The dosage of the active ingredient may be appropriately selected depending on various factors, including the route of administration, and the patient’s age, sex, weight, and disease

severity. The composition for preventing or treating cancer according to the present invention may be administered in combination with a known compound having an effect of preventing, ameliorating or treating symptoms of cancer.

Hereinafter, the present invention will be described in more detail with reference to examples. These examples are only for illustrating the present invention, and it will be apparent to those of ordinary skill in the art that the scope of the present invention is not to be construed as being limited by these examples.

## Example 1: Materials and Methods

### Example 1-1: Synthesis of OBOC libraries

OBOC libraries were synthesized on solid-phase TentaGel MB $NH_2$ resin (Rapp Polymere GmbH, Tübingen, Germany). A "split-mix" synthesis method was performed to construct the combinatorial OBOC libraries, each containing random libraries of millions of beads/ligands. 5 g of Tentagel MB $NH_2$ resin (200 μm, 520,000 beads/g) was used for the synthesis of approximately 2,600,000 OBOC libraries. The ligand on the bead surface was synthesized by standard solid-phase peptide synthesis techniques using 9-fluorenylmethoxycarbonyl (Fmoc) chemistry and N-hydroxybenzotriazole (HOBt; *GL Biochem,* Shanghai, China)/N,N'-diisopropylcarbodiimide (DIC; GL Biochem) coupling. The completion of coupling was confirmed with a ninhydrin test. The beads were stored in 70% ethanol at 4°C until use.

### Example 1-2: Ethics and cell culture

All work related to human tissues was preapproved by the Institutional Review Board (IRB) at the Gwangju Institute of Science and Technology (#20191008-BR-48-03-02). All animal experiments were carried out according to the Institutional Animal Care and Use Committee (IACUC) of the Gwangju Institute of Science and Technology (GIST-2019-040). All cultures were grown in a humidified incubator maintained at 37°C with 95% air/5% $CO_2$. MCF-10A normal human breast cell lines were obtained from the American Type Culture Collection and propagated in MEGM complete growth medium (MEGM, Lonza, Walkersville, MD) supplemented with bovine pituitary extract (Cambrex Bioscience, Walkersville, MD). The normal human colorectal cell line CCD-18Co was obtained from the Korea Cell Line Bank (Seoul, Korea). The human breast and colorectal cancer cell lines, including MCF-7, MDA-MB-231, HT-29, and HCT-116 were obtained from the Korea Cell Line Bank. Jurkat T cells were also obtained from the Korea Cell Line Bank. The luciferase-expressing MDA-MB-231 cancer cell line was obtained from PerkinElmer (PerkinElmer, Hopkinton, MA). Each cancer cell line was grown in RPMI1640 (Gibco, Waltham, USA) and DMEM (Gibco) supplemented with 10% heat-inactivated fetal bovine serum (FBS; Gibco), 0.1 mg/ml streptomycin (Gibco), and 100 units/ml penicillin (Gibco).

### Example 1-3: Screening of the OBOC library for cancer-specific ligands

The beads were washed extensively with double-distilled water and phosphate-buffered saline (PBS; Welgene Inc., Korea) before screening. Cancer cells and normal cells were detached from culture dishes with trypsin/EDTA (Gibco), washed with their corresponding culture medium, resuspended at $10^6$ cells/ml, and incubated with OBOC beads in Petri dishes in a humidified $CO_2$ incubator at 37°C with shaking (60 rpm). Beads bound by cells appeared under a microscope as rosettes with a central bead covered by one or more layers of cells. The positive beads were picked by pipette under an inverted microscope, treated with guanidine-HCL (8 M, 20 min) to remove cells and proteins on the bead surface, and subjected to a second round of screening with normal breast epithelial cells to eliminate false positive binding. Only beads with cell binding at both rounds were sent for peptide sequencing.

### Example 1-4: Chemical synthesis of peptides and 2'-maleimide-PTX

All peptides and 2'-maleimide-PTX were synthesized by AnyGen (Gwangju, Korea) using solid-phase peptide synthesis and Steglich esterification, respectively. The purity and molecular masses of the peptides and 2'-maleimide-PTX were determined by using HPLC and matrix-assisted laser desorption ionization time-of-flight mass spectrometry (MALDI-TOF MS), respectively (Shimadzu, Kyoto, Japan).

### Example 1-5: Synthesis of AGM-330-PTX

Peptide branched-thiol intermediates were dissolved in 500 μM dimethylformamide (DMF; Duksan Chemical, Korea) and mixed with a solution of 2'-maleimide-PTX in DMF (5 mM; 10 equivalents) and 0.1% v/v N,N-diisopropylethylamine (DIPEA; Duksan). The mixture was stirred at room temperature for 30 min, and then purified by HPLC (Shimadzu) . After purification by HPLC, the molecular mass of AGM-330-PTX was determined by using MALDI-TOF MS (Shimadzu).

Example 1-6: Serum stability test

Stock solutions of peptides (100 μM) were diluted by a factor of 10 with pre-warmed 100% human serum (Sigma-Aldrich) and incubated at 37°C for 0, 3, 6, 9, and 24 hours. Controls with peptides in PBS were included. The action was stopped by denaturing the serum proteins with urea at a final concentration of 3 M at 4°C for 10 min, followed by precipitation of serum proteins with trichloroacetic acid at a final concentration of 7%(v/v) (4°C, 10 min) and centrifugation (17,000 × g, 10 min). The supernatant of each sample was recovered and run on an analytical column using a linear gradient of 5-65 % solvent B (acetonitrile 90%(v/v) with 0.045%(v/v) TFA in $H_2O$) in solvent A (0.05%(v/v) TFA in $H_2O$) over 25 min at a flow rate of 1 ml/min with monitoring at 215 nm. The elution profile of each peptide was identified by the PBS sample from the 0 time point. The percentage of peptide remaining in serum-treated samples was determined by comparing the height of the peptide peak obtained at each time point with that of the peptide peak obtained at the 0 time point. Each experiment was performed in triplicate.

Example 1-7: Flow cytometry analysis

Fluorescence-activated cell-sorting (FACS) analysis was used to check the binding of the FITC labeled peptides (FITC-peptide). The stock solution for the FITC-peptides (100 μM) was prepared by dissolving the peptide in PBS. MCF-10A, MCF-7, MDA-MB-231, CCD-18Co, HT-29, and HCT-116 cells were seeded in 6-well plates with $10^5$ cells/well containing 3 ml of medium, and the plates were incubated at 37°C overnight. The following day, the medium was replaced with fresh FBS-free medium (1 ml) containing FITC-peptide (1 μM) and further incubated at 37°C for 30 min. Thereafter, the medium was removed, and the cells were washed with cold PBS to remove any remaining peptide. Thereafter, the medium was removed, and the cells were washed with cold PBS to remove any remaining peptide. Adequate trypsin/EDTA was added to each well, followed by 3 to 5 min incubation at 37°C. Trypsin/EDTA was immediately neutralized by adding medium, and the cell suspension was transferred into centrifuge tubes. The cells were separated and washed twice with PBS. The control cells were treated similarly but without peptide. The prepared cells were analyzed on a FACScanto II (BD Biosciences) flow cytometer. The FACS data were analyzed with FlowJo (TreeStar).

Example 1-8: Biotin pull-down assay

The cell lysates (500 μg/ml) were incubated with biotinylated AGM-330 (500 ng/ml) for 12 hours at 4°C and then subjected to pull-down by streptavidin beads (Thermo Fisher Scientific, Rockford, IL) for 1 hour at room temperature. After incubation, the beads were washed three times with washing buffer. Then, the elution buffer was added to the beads, and proteins were extracted from the beads, and finally analyzed by SDS-PAGE. To identify and characterize the proteins derived from the biotin pull-down assay, LC-coupled ESI-MS/MS analysis was performed at ProteomeTech (Seoul, Korea).

Example 1-9: AGM-330 binding domain identification

The NCL constructs, including GFP-NCL (residues 1-710), GFP-ΔN-NCL (residues 322-710), and GFP-ΔC-NCL (residues 1-321) were generated from the human NCL cDNA clone (Addgene) and subcloned into the *Xho*I and *Bam*HI sites of the pEGFP-C2 vector (Addgene). These vectors were transfected into cells by Lipofectamine 2000 (Invitrogen, Carlsbad, CA, USA) according to the manufacturer's recommendations. Biotinylated AGM-330 peptides were added to the streptavidin beads (Thermo Fisher Scientific), and the mixture was incubated under shaking for 1 h at room temperature. The beads were washed 3 times with wash buffer. After washing, the beads were added to the lysate (300 ul) prepared from transfected cell lysate containing GFP-tagged NCL proteins. The reaction mixture was incubated for 12 h at 4°C to allow binding between AGM-330 and GFP-tagged NCL proteins. The beads were subsequently washed with wash buffer. Then, an equal volume of 2X electrophoresis sample buffer was added to the beads, and proteins were extracted from the beads by heating at 95°C for 5 min. Then, the proteins were finally analyzed by SDS-PAGE and immunoblot analysis.

Example 1-10: Purification of NCL from Jurkat cells

NCL was prepared by lysing 1.0 × $10^9$ Jurkat cells in 25 ml of 20 mM Tris/HCl, pH 7.5, 150 mM NaCl, 5 mM $MgCl_2$, 5 mM β-mercaptoethanol, 0.5%(v/v) Triton X-100, 1 mM marimastat (AdooQ Bioscience, Irvine, CA, USA), and protease inhibitor cocktail (Millipore, Billerica, MA, USA) at 4°C for 1 h. Nuclei were pelleted by centrifugation at 1200 xg for 5 min, and then the supernatant was centrifuged at 12,000 xg for 30 min prior to storage at -80°C. A rapid two-step chromatography procedure was used to purify NCL from the nuclear-free extract. All steps were performed at 4°C using ice-cold buffers and columns in the presence of 1 mM marimastat and complete protease inhibitor cocktail. The

cytosol extract of Jurkat cells (25 ml) was diluted 10-fold with 20 mM sodium phosphate, pH 7.0, and passed through a 5 ml Mono Q 5/50 GL column (Sigma-Aldrich). After washing the column with 150 ml of 20 mM sodium phosphate, pH 7.0, the adsorbed proteins were eluted with 10 ml of the same buffer containing 1 M NaCl. The eluate was diluted 10-fold with 50 mM Tris/HCl, pH 7.9, 5 mM $MgCl_2$, 0.1 mM EDTA, 1 mM β-mercaptoethanol (buffer A), and loaded on a 1 ml HiFiQ heparin HP column (Protein Ark, UK) equilibrated with the same buffer. The gel was washed with 20 ml of buffer A containing 0.2 M ammonium sulfate, and the protein was eluted in 50 μl fractions with 2 ml of buffer A containing 0.6 M ammonium sulfate. NCL was collected and dialyzed against PBS containing 1 mM marimastat at 4°C for 2 h before stored at - 80°C. A further control on a 10% SDS acrylamide gel stained with Coomassie blue, confirmed the presence of the purified NCL as a single 105 kDa protein band. Two 70 and 50 kDa protein bands, corresponding to partial degradation products of NCL, were observed in amounts lower than 10% of the total protein.

Example 1-11: Binding affinity

Binding affinity was tested by SPR (surface plasmon resonance) spectroscopy (Biacore T-200) . The recombinant nucleolin protein was immobilized on the CM5 sensorchip, and the treated with AGM-330 at various concentrations (20 nM to 2.5 μM). The sensogram for each sample was analyzed to determine the $K_D$ value.

Example 1-12: Protein isolation and Western blot analysis

Cells were lysed in RIPA buffer (20 mM Tris-HCl, pH 7.5, 200 mM NaCl, and 0.5% Triton X-100) containing protease inhibitor cocktail (Millipore). The protein concentration was measured with a Protein Assay Kit (Bio-Rad) following the manufacturer's protocol. Total protein was subjected to SDS-PAGE and transferred to a polyvinylidene difluoride membrane. The blot was probed with primary antibodies against NCL (Abcam) and GFP (Abcam). As a loading control, anti-β-actin antibody (Santa Cruz Biotechnology), anti-P-cadherin (Abcam), and anti-lamin A/C (Abcam) were used. Subsequently, the blots were washed in TBST (10 mM Tris-HCl, 50 mM NaCl, and 0.25% Tween-20) and incubated with a horseradish peroxidase-conjugated secondary antibody. The presence of target proteins was detected using enhanced chemiluminescence reagents (Thermo Fisher Scientific).

Example 1-13: Immunofluorescence staining

MDA-MB-231-luc xenograft tissues were formalin-fixed and paraffin-embedded for immunofluorescence staining. Cells were seeded on poly-L-lysine and collagen I-coated cover glasses and fixed with 4% formalin. Tissue slides and cells were permeabilized with 0.1% Triton X-100 and blocked with 2% BSA (Sigma-Aldrich). Staining was performed as described previously using primary anti-NCL (1:200), anti-TUNEL (1:200), and anti-Ki-67 (1:500) antibodies. All nuclei were counterstained with DAPI. Immunofluorescence images were matched with H&E-stained images.

Example 1-14: Small interfering RNA (siRNA)-mediated knockdown

siRNAs targeting NCL (NM_005381.3 in NCBI database) and scramble siRNA (scr) were purchased from Bioneer (Daejeon, Korea). For efficient NCL transfection, siRNA transfection was performed using Lipofectamine 2000 (Invitrogen) according to the manufacturer's protocol. Three different siRNA sequences were used, and their efficiencies were evaluated by real-time PCR.
siNCL1: (sense) 5'-GAGCUAACCCUUAUCUGUA (dTdT)-3' (SEQ ID NO: 21)
(antisense) 5'-UACAGAUAAGGGUUAGCUC (dTdT)-3' (SEQ ID NO: 22)
siNCL2: (sense) 5'-CACAAGGAAAGAAGACGAA (dTdT)-3' (SEQ ID NO: 23)
(antisense) 5'-UUCGUCUUCUUUCCUUGUG (dTdT)-3' (SEQ ID NO: 24)
siNCL3: (sense) 5'-GACGAAGUUUGAAUAGCUU (dTdT)-3' (SEQ ID NO: 25)
(antisense) 5'- AAGCUAUUCAAACUUC GUC (dTdT)-3' (SEQ ID NO: 26)

The most effective siRNA was chosen based on mRNA levels determined by RT-qPCR analysis, and the resulting protein levels were validated by Western blot analysis.

Example 1-15: Real-time PCR

Total RNA was extracted using RNAiso (Takara, Shiga, Japan), and the RNA purity was verified by measuring the 260/280 absorbance ratio. First-strand cDNA was synthesized using a PrimeScript™ 1st strand cDNA Synthesis kit

(Takara, Shiga, Japan), and one-tenth of the cDNA was used for each PCR mixture with Power SYBR® Green PCR Master Mix (Applied Biosystems). Real-time PCR was performed using a StepOnePlus Real-Time PCR System (Applied Biosystems). The relative mRNA expression of selected genes was normalized to that of β-actin and quantified using the ddCt method. The sequences of PCR primers are shown in Table 6 below.

[Table 6]

| Primer | Sequence | SEQ ID NO. |
|---|---|---|
| NCL(Forward) | AAAGTGCCCCAGAACCCACA | 27 |
| NCL(Reverse) | TGGCTGACTTCTCGCATTAGG | 28 |
| β-actin(Forward) | GAGCAATGATCTTGATCTTCA | 29 |
| β-actin(Reverse) | AGATGAGTATGCCTGCCGTG | 30 |

Example 1-16: Antibody neutralization assay

Cancer and normal cell lines were seeded in 96-well plates with $1 \times 10^4$ cells/well containing 100 ul of medium, and the plates were incubated at 37°C overnight. After incubating overnight, the cells were incubated for 24 h in the absence or presence of an anti-NCL antibody (Cell Signaling Technology, Beverly, MA, USA) at 37°C overnight. After incubation, cell viability was assessed by a CellVia WST-1 assay (Young In Frontier, Seoul, Korea) according to the manufacturer's instructions.

Example 1-17: Apoptosis assay (Annexin V)

A quantitative assessment of apoptotic cells was performed using an Annexin V-fluorescein isothiocyanate (FITC) Apoptosis Detection Kit I (BD Biosciences). Cells were collected and washed twice with cold PBS, and then resuspended in binding buffer ($1 \times 10^6$ cells/ml). Next, 100 μl of the suspension was transferred to a tube and mixed with 5 μl of FITC Annexin V and propidium iodide (PI). Then, the mixture was incubated at room temperature for 15 min in the dark after gentle vortexing. After incubation, 400 μl of 1× binding buffer was added, and the cells were analyzed by flow cytometry.

Example 1-18: Cell proliferation assay

Cancer and normal cells ($1 \times 10^4$ cells/well) were seeded in 96-well plates. After incubation for 24 h, the cells were treated with increasing concentrations of AGM-330, PTX, and AGM-330-PTX for 48 h. Cell viability was assessed by the CellVia WST-1 assay (Young In Frontier) according to the manufacturer's instructions. The numbers of viable cells were measured at a wavelength of 450 nm using a VersaMax ELISA plate reader (Molecular Devices).

Example 1-19: Tumorigenesis experiment

All animal experiments were performed in accordance with IACUC guidelines (GIST-2019-040). For tumorigenesis experiments, anesthetized 6-week-old female NOD-*scid* $Il2rg^{-/-}$ mice (NPG™, VITALSTAR) were inoculated in the mammary fat pad with $1 \times 10^6$ MDA-MB-231-luc cells in a volume of 100 μl ($n$ = 5~6 for each group). After tumor cell inoculation, when the tumor volume reached approximately 100 $mm^3$, the mice were randomly divided into the following five groups: (i) control, (ii) low-dose paclitaxel (2 mg/kg), (iii) high-dose paclitaxel (10 mg/kg), (iv) AGM-330 (16.68 mg/kg or 8.34 mg/kg), and (v) AGM-330-PTX (19.05 mg/kg or 10.71 mg/kg). The tumor size was measured twice a week, and the tumor volume was calculated with the following formula:

$$\text{Volume } (mm^3) = (\text{length } (mm)) \times (\text{width } (mm))^2 \times 0.5.$$

For the bioluminescent imaging experiment, D-luciferin (150 mg/kg; PerkinElmer) was intraperitoneally injected into each mouse, and an IVIS 100 imaging system (Xenogen, Corporation, Alameda, CA) was used for bioluminescent monitoring. Mice were anesthetized with vaporized isoflurane (BK Pharm, Ilsan, Korea) and placed in an imaging chamber. After 10 min, each animal was imaged with an exposure time of 1 min. All bioluminescent image data were provided by Living Image software (version 4.5.2, PerkinElmer). Photons detected from tumors were converted to average radiance values (photon/sec/$cm^2$/sr), which are quantitative data obtained from a region of intensity (ROI) and include the photons

emitted by bioluminescent cells from an assigned rectangular area over the whole body of each mouse.

Example 1-20: Bioinformatics

The Oncomine Cancer Microarray database (http://www.oncomine.org/) was used to analyze the expression levels of NCL in normal breast and colorectal tissues and in breast carcinoma and colorectal adenocarcinoma tissues. These gene expression data were log2 transformed and median centered. All graphics and statistical values were analyzed by GraphPad Prism 5.0, and *P*-values were calculated by the two-tailed Student's *t*-test ($P < 0.05$). Kaplan-Meier plots were generated using the R2 platform, and patients were grouped according to the NCL expression levels in their breast and colorectal tumors.

Example 1-21: Statistical analysis

All statistical data are expressed as the means ± SD ($n = 3$). Statistical comparisons between two groups were determined by Student's *t*-test, and comparisons among multiple groups were determined by one-way ANOVA with Dunnett's multiple comparison. In the case of *in vivo* experiments, the number of mice is indicated in each legend. The log-rank test was used for Kaplan-Meier analyses. *, **, and *** indicate $P < 0.05$, $P < 0.01$, and $P < 0.001$, respectively.

**Example 2: Identification and characterization of cancer-targeting peptide ligands by OBOC combinatorial screening and MAP synthesis methods**

To identify a novel cancer-specific peptide ligand, the present inventors synthesized about 2,600,000 libraries using 5 g of Tentagel MB $NH_2$ resin (200 μm, 520,000 beads/g; FIG. 3). A peptide library displayed on beads (50,000 to 100,000 beads were used at a time) was mixed with a human breast cancer cell line (MDA-MB-231). A total of about 1,000,000 beads were screened, and 8 positive beads were detected and isolated for microsequencing. Three of these 8 peptides (AGM-330, AGM-331, and AGM-332) showed strong preferential binding to MDA-MB-231 and showed no or only weak binding to the human normal breast cell line MCF-10A (FIGS. 4A and 4B). Then, these 3 peptides were labeled with fluorescein isothiocyanate (FITC), and whether they could bind to MDA-MB-231 cells was determined. Compared with AGM-331 and AGM-332, the strongest fluorescence signals were detected with AGM-330 (FIG. 4C). To confirm the screening results from the cell-growth-on-bead assay and fluorescence imaging, AGM-330 was resynthesized on Tentagel MB $NH_2$ resin. A cell-growth-on-bead assay showed that the AGM-330 beads were fully covered by human breast cancer cell lines (MCF7 and MDA-MB-231) and human colorectal cancer cell lines (HT-29 and HCT-116) within 15 min (FIG. 4D). In contrast, AGM-331 and AGM-332 were relatively nonspecific and bound to the human normal breast cell line MCF-10A and the human normal colorectal cell line CCD-18Co. In addition, AGM-330 bound either very weakly or not at all to MCF-10A or CCD-18Co cells, making them excellent candidates for both imaging and therapeutic targeting agents.

The use of peptides as therapeutic drugs has to date been largely limited by their low stability: peptides are primarily broken down by proteases and peptidases *in vivo* (Bottger R, et al. PLoS One. 2017; 12: e0178943). In the present invention, to improve the stability of the peptide ligand, AGM-330 in a MAP dendrimeric form was synthesized, which can exhibit increased stability due to the acquired resistance to protease and peptidase activity. The synthesis of AGM-330 (FIG. 4E) commenced with Fmoc-Cys(Trt) Wang resin (**1**) and Fmoc-Lys(Fmoc)-OH in the presence of 20% piperidine and DMF to provide lysine core-conjugated Wang resin (**2,3**). Then, Fmoc- and Trt-protected AGM-330 (**4**) was produced by treatment with RHGAMVYLK-PEG12-OH in the presence of 20% piperidine. The Fmoc and Trt protecting group in AGM-330 (**4**) was quickly removed using piperidine in DMF, giving rise to AGM-330 (**5**).

To enhance *in vivo* stability and binding affinity of the selected peptide ligand AGM-330, it was synthesized in dimeric and tetrameric forms by the MAP (Multiple-Antigen-Peptide) synthesis method.

In order to evaluate whether the stability of the peptide was enhanced, the *in vivo* half-life of the peptide was analyzed. 2 mg/kg of AGM-330m, AGM-330d or AGM-330t was injected through the tail vein of C57BL/6 mice, and the remaining peptide portion in the serum was immediately immobilized on an ELISA plate using an anti-AGM-330 antibody. Absorbance was measured at a wavelength of 450 nm using a VersaMax ELISA plate reader (Molecular Devices), and pharmacokinetics was analyzed with phoenix WinNonlin 8.1 (*Pharsight* Corporation, Mountain View, CA, USA) program. The analysis results showed that the half-lives ($T_{1/2}$) of AGM-330m, AGM-330d and AGM-330t were 0.42±0.33 h, 1.82±0.36 h, and 9.43±1.21 h, respectively, and the maximum concentration time ($T_{max}$) in blood was 0.167 hours for all the peptides. The maximum blood concentrations ($C_{max}$) were 1.27±0.12 μg/mL, 1.71±0.11 μg/mL, and 1.875±0.67 μg/mL, respectively, and the areas under the curve (AUC) were 0.64±0.09 μg/h/mL, 1.77±0.12 μg/h/mL, and 21.42±0.81 μg/h/mL, respectively (FIG. 5). As a result, it was confirmed that tetrameric AGM-330 (AGM-330t) had higher stability than the monomeric or dimeric peptides.

Next, to examine whether the MAP ligand can selectively bind to cancer cells, cancer cells treated with FITC-labeled AGM-330 (AGM-330-FITC) were compared with untreated cells. AGM-330-FITC was incubated with the cells ($1 \times 10^5$) for 2 h at 37°C in serum-free medium to keep the conjugates intact. Then, the inherent fluorescences of peptide-treated cells were compared and measured based on changes in the mean fluorescence intensity (MFI) values compared to those of untreated cells (FIG. 4F). The results showed that AGM-330-FITC displayed significant cancer cell binding to MCF-7, MDA-MB-231, HT-29, and HCT-116, as evidenced by the increase in MFI of treated cells relative to the untreated cells. In contrast, the conjugates displayed a significant decrease in binding to normal cells, including MCF-10A and CCD-18Co, versus strong preferential binding to cancer cells after 2 h of incubation.

To examine whether AGM-330 was cytotoxic, the present inventors evaluated $IC_{50}$ values in multiple cancer cells, including MCF-7, MDA-MB-231, HT-29, and HCT-116, and in normal cells, including MCF-10A and CCD-18Co. The $IC_{50}$ values of AGM-330t and AGM-330d were greater than 100 $\mu$M in all cell lines (FIG. 6). This suggests that AGM-330 does not appreciably affect the cell viability of cancer and normal cell lines. Taken together, these data indicate that AGM-330 can be utilized for the targeting of cancer cells without affecting target cell viability.

**Example 3: NCL, a potent regulator of cancer cell growth, is a potential target of AGM-330**

To identify the unknown target protein of AGM-330, affinity column chromatography and mass spectrometry-based proteomic approaches were employed to identify the AGM-330-interacting proteins from the cell lysate of MDA-MB-231 cells (FIG. 7A). After affinity column chromatography, SDS-PAGE analysis revealed several distinct protein bands (from 55 to 100 kDa) that were only present in the elution fraction (FIG. 7B). Six proteins were identified using LC-MS/MS analysis, and peptide identification of these excised bands showed that one of the major protein bands at 100 kDa was characterized as nucleolin (NCL). NCL consists of 710 amino acids, and 22 different peptide fragments that were identified by LC-MS/MS analysis were matched to the amino acid sequence of NCL (total score of 881 and 24% sequence coverage to human NCL in NCBI database (accession number: gi189306; FIGS. 7C and 7D). Moreover, immunoblot analysis using an anti-NCL antibody further confirmed the enrichment of NCL in the elutes from the AGM-330 affinity column (FIG. 7E). Thus, the proteomic study results suggested that NCL is the AGM-330-interacting protein.

To examine the binding affinity of AGM-330 to NCL, an SPR (surface plasmon resonance) test was performed using AGM-330. As shown in FIG. 8, it was confirmed that the $K_d$ value was about 57.7 nM, indicating that AGM-330 has a very high binding affinity for NCL.

To investigate the domain involved in the interaction between AGM-330 and NCL, the present inventors used the biotin pull-down assay. Biotinylated AGM-330 (AGM-330-biotin) was used as bait to pull-down NCL mutants. Several deletion mutants of NCL were generated. Cell extracts were prepared from HEK293T cells transfected with expression vectors encoding the wild-type NCL and the various NCL mutants, including NCL1 (1-710), NCL2 (323-710) and NCL3 (1-322), all fused to GFP. AGM-330-biotin pulled down wild-type NCL1 (1-710) and NCL3 (1-322) but not NCL3 (323-710) (FIG. 7F). These results indicate that the N-terminal region (1-322) of NCL is important for binding thereof to AGM-330. To further confirm the direct interaction between AGM-330 and NCL, purified NCL and AGM-330-Biotin were used for biotin pull-down assay. The immunoblot analysis results obtained using anti-NCL antibody showed that NCL was enriched in the elution fractions (FIG. 9).

Taken together, these results indicate that AGM-330 directly interacts with NCL.

**Example 4: Identification of amino acid residues essential for interaction between AGM-330 and NCL**

Cancer cells ($1 \times 10^4$ cells/well) were cultured in 96-well plates for 24 hours. AGM-330 and the 12 PEGylated peptides shown Table 2 were biotin-conjugated, and the cells were treated with increasing concentrations of the 13 peptides for 2 hours.

Thereafter, avidin-HRP was attached through a high-affinity avidin-biotin interaction, and the TMB substrate was converted to blue color through HRP enzyme. Then, the reaction was terminated by adding an acid, and the affinities of the 13 peptides were assessed by ELISA by reading the wells at 450 mm.

The absorbance of the 13 PEGylated peptides is shown in FIG. 10. The four peptides of SEQ ID NOs: 9, 10, 11 and 14 showed absorbance similar to that of AGM-330. In addition, the four peptides of SEQ ID NOs: 12, 13, 15 and 20 showed higher absorbance than AGM-330. The substitution of leucine or norleucine at a methionine residue at position (M5), the substitution of phenylalanine a tyrosine residue at position 7 (Y7), and the insertion of repetitive leucine (L) and lysine (K) residues at the C-terminus increased the binding affinity. This suggests that the substitution of hydrophobic amino acids for the M5 and Y7 residues or the insertion of L and K at the C-terminus can increase the binding affinity.

However, the peptides of SEQ ID NOs: 16 to 19 showed lower absorbance than AGM-330. The deletion of arginine at position 1 (R1) and histidine at position 2 (H2) residues, the insertion of repetitive arginine (R) and histidine (H) residues at the N-terminus, and the reversed sequence reduced the binding affinity. This suggests that the directionality of the sequence, including R1 and H2, should be preserved.

**Example 5: AGM-330 specifically binds to cancer cells *in vitro* and *in vivo***

The present inventors evaluated the specificity of AGM-330 to NCL with cell binding assays against different cell lines using fluorescence microscopy. The experiment was performed using multiple cancer cell lines, MCF-7, MDA-MB-231, HT-29, and HCT-116, and normal cell lines, MCF-10A, and CCD-18Co. The binding specificity was determined by confocal imaging after 1 h of incubation of the cells with 5 μmol/l AGM-330-FITC. The cells were incubated with DAPI to counterstain the nuclei (blue fluorescence). Double-immunofluorescence staining with an anti-NCL antibody that binds to the N-terminal domain of NCL and AGM-330-FITC revealed an overlap between NCL and AGM-330-FITC, indicating that AGM-330 binds to NCL (FIGS. 11A and 11B).

Next, the present inventors determined the relative amounts and intracellular localization of NCL in MCF-7, MDA-MB-231, and MCF-10A cells to determine whether differences in either the expression level or localization of NCL were related to the sensitivity of cancer cells to AGM-330. The present inventors separated the subcellular fraction of cells and analyzed the expression of NCL in the cytosolic, nuclear, and cell membrane extracts, as well as the total NCL in cells, by Western blotting. The results of this analysis revealed that NCL expression was elevated in membrane and cytoplasmic extracts of MCF-7, MDA-MB-231, HT-29, and HCT-116 cells compared with that observed in MCF-10A and CCD-18Co cells (FIGS. 11C and 12A). No significant differences in the nuclear levels of NCL were observed among all cell lines.

To investigate whether membrane-expressed NCL is critical for AGM-330 binding to cancer cells, the present inventors used specifically targeting siRNAs for NCL knockdown. It was confirmed that three siRNAs targeting NCL (siNCL1, siNCL2, and siNCL3) showed different efficacies in NCL-knockdown cells. Because siNCL1 showed the highest knockdown efficacy in MDA-MB-231 cells, it was selected for further use (FIG. 12B). Next, to confirm whether NCL expression at the cancer cell membrane is decreased after siNCL1 treatment, the present inventors separated the subcellular fraction of siNCL1-treated cells and analyzed the expression of NCL in the cytosolic, nuclear, and cell membrane extracts by Western blotting. The results showed that siNCL1 treatment potently inhibited the basal expression of NCL in cytosolic and cell membrane fractions (FIG. 11D). Following expression level studies of NCL, the present inventors confirmed that decreased expression of membrane NCL affected AGM-330 binding. As a result, scrambled siRNA-treated cells showed fluorescence signals similar to controls. However, little or no fluorescence signal was detected in the siNCL1-treated group due to the decreased AGM-330 binding (FIG. 11E). Fluorescence imaging results revealed that the anti-NCL antibody, which binds to the membrane of cancer cells, and AGM-330-FITC colocalized with anti-NCL antibody fluorescence (FIGS. 11A and 11B). Additionally, mutant analysis showed that the N-terminal domain of NCL was responsible for the interaction between NCL and AGM-330 (FIG. 7F). These results suggest that AGM-330 directly interacts with NCL, which is expressed in the cancer cell membrane.

Next, the present inventors used a xenograft model of human breast cancer to evaluate the cancer-targeting properties of AGM-330 by *in vivo* imaging (FIG. 11F). To examine whether AGM-330 can selectively target cancer cells *in vivo,* tumor-bearing mice treated with Alexa680-labeled AGM-330 (AGM-330-Alexa680) were compared to mice treated with Aleax680 alone. The near-infrared fluorescent (NIRF) intensities of tumors 1 h after AGM-330-Alexa680 fractions were significantly higher than those observed for control tumors: $3.04 \times 10^7 \pm 1.38\ 10^6$ versus $1.19 \times 10^9 \pm 1.90\ 10^7$ (p/sec/cm$^2$/sr)/(μW/cm$^2$) for the control and AGM-330-Alexa680-treated tumors, respectively; $n = 3$, $P < 0.001$ (FIGS. 11G to 11I) . In addition, the percentage of the injected dose of AGM-330-Alexa680 and of Alexa680 alone in the tumor and other major viscera at 2 h was analyzed to quantitatively examine the effect of AGM-330 on the distribution of the conjugated fluorescent. The NIRF total photon counts per gram of each organ from the tumor tissues, except the kidney and liver, were significantly higher than those observed for other organs, providing definitive evidence that the tumor-targeting property of AGM-330 is much greater than that of the controls (FIG. 11J). Taken together, the NIRF images revealed that AGM-330 mainly accumulated throughout the tumor tissue rather than normal organs *in vivo.*

**Example 6: Confirmation of the positive correlation between NCL and cancer progression, and the effect of anti-NCL antibodies on inhibition of cell proliferation via membrane NCL neutralization**

Based on the above Examples, the present inventors confirmed the NCL expression pattern in cancer cell lines. Next, to examine NCL expression in human cancer tissues, the present inventors analyzed the available breast and colorectal cancer datasets using the Oncomine dataset repository (www.oncomine.org). The relative expression of NCL in 144 primary breast tissues versus 67 breast carcinomas was analyzed in the Curtis datasets. NCL mRNA expression was significantly upregulated in breast carcinoma compared to normal breast tissue ($P < 0.001$, FIG. 13A). Additionally, the expression of NCL mRNA was significantly higher in colorectal adenocarcinoma than in the normal counterpart tissue in the Alon datasets ($P < 0.01$, FIG. 13A). Next, the present inventors confirmed that high NCL expression was associated with poor prognosis, such as low disease-free survival, in patients with breast and colorectal cancer (Bertucci and Sveen dataset from "R2: Genomics Analysis and Visualization platform (http://r2.amc.nl)") (FIG. 13B), which is consistent with a previous report showing that high NCL expression serves as a poor prognostic marker in patients with breast and

colorectal cancer (van Long FN, et al. Cancers. 2018; 10: 390) . To determine the distribution of NCL expression in human cancer and normal tissues, the present inventors analyzed NCL expression by immunohistochemistry (IHC) analysis. Consistent with the results of immunofluorescence imaging (FIGS. 11A and 11B) and Western blotting (FIG. 11C), IHC results revealed NCL levels were also increased in the membrane and cytoplasm regions of human breast and colorectal cancer tissues compared with those observed in normal breast and colorectal tissues (FIGS. 13C and 13D). In addition, previous reports have shown that upregulated membrane NCL interacts as a receptor for ligands involved in cancer proliferation and the inhibition of apoptosis (Chen SC, et al. Oncotarget. 2015; 6: 16253-70). Therefore, to examine whether membrane-expressed NCL is essential for regulating functions in cancer cells, the present inventors neutralized membrane-expressed NCL using monoclonal anti-NCL antibodies on MDA-MB-231 and HCT-116 cells and MCF-10A and CCD-18Co cells *in vitro.* First, cell viability was evaluated on normal and cancer cells treated with antibodies at different concentrations for 24 h. Treatment with the NCL antibody (50 μg/ml) led to 35% and 32% reductions in cell viability in MDA-MB-231 and HCT-116 cells, respectively, but no significant decrease was observed in normal cells compared to the control and IgG treated groups (MCF-10a and CCD-18Co) (FIGS. 13E to 13H). Additionally, the present inventors performed apoptosis assays to examine the cause of the cytotoxicity effect of the anti-NCL antibody on cancer cells. Annexin V and PI were used to stain MDA-MB-231 and HCT-116 cells treated with antibodies at different concentrations at 24 h. The flow cytometry results revealed that the percentages of cancer cells in the early and late apoptosis phases increased in an anti-NCL antibody dose-dependent manner (FIG. 13I).

The present inventors confirmed that NCL is overexpressed in the membrane and cytoplasm of various types of cancer and NCL overexpression is correlated with poor prognosis survival in cancer patients. These results demonstrate an important role for the NCL membrane portion in cancer cells, particularly in terms of the control of cancer cell proliferation and apoptosis. Taken together, these results suggest that NCL plays an important role in cancer growth and that targeting membrane NCL may be a promising biomarker for cancer treatment with chemotherapeutic drugs.

**Example 7: Confirmation of the effect of Paclitaxel-conjugated AGM-330 on inhibition of cancer growth in vitro and in mouse xenograft model**

Paclitaxel (PTX) was conjugated with AGM-330 (AGM-330-PTX) to obtain a cancer-targeting ligand linked to a well-established chemotherapeutic agent. PTX was adapted to the orthogonal conjugation with a maleimide-carboxy bifunctional linker. To examine whether AGM-330-PTX inhibits cancer cell proliferation, the present inventors evaluated its $IC_{50}$ values in multiple cancer cell lines, including MCF-7, MDA-MB-231, HT-29, and HCT-116 cells (FIG. 14A). In MDA-MB-231 cells, the $IC_{50}$ values for PTX alone and AGM-330t-PTX were 6.8 and 3.6 μM, respectively. The results were similar in all three cancer cell lines (MCF-7, HCT-116, and HT-29). However, in MCF-10A cells, the $IC_{50}$ values for PTX alone and AGM-330t-PTX were 22.1 and 35.3 μM, respectively.

Following *in vitro* proliferation analysis on cancer lines, the present inventors investigated whether AGM-330t-PTX could be a potential therapeutic agent *in vivo.* The present inventors inoculated MDA-MB-231-luc cells into the mammary fat pad of NPG mice. After the subcutaneous tumors grew to 100 $mm^3$, comparative efficacy studies were performed by dividing the animals into five groups ($n$ = 5/group) treated with vehicle (PBS), AGM-330t (16.68 mg/kg), PTX (2 or 10 mg/kg, respectively) or AGM-330t-PTX (19.05 mg/kg) twice a week by tail vein injection (FIG. 14B). Tumor growth was monitored by measuring volumes twice a week with a caliber rule over the course of 3 weeks. Consistent with *in vitro* experiments, vehicle (PBS)-treated mice and AGM-330-treated mice showed no significant difference in the average tumor volume. However, the average tumor volume of mice treated with AGM-330t-PTX (807 ± 90 $mm^3$ for 19.05 mg/kg, $n$ = 5) had decreased by approximately 52% with respect to animals treated with the same dose PTX (1671 ± 199 $mm^3$ for 2 mg/kg, $n$ = 5, $P$ < 0.001). Furthermore, AGM-330t-PTX-treated mice showed enhanced drug efficacy compared to the 5-fold increased dose of PTX-treated mice at day 21 (988 ± 78 $mm^3$ for 10 mg/kg, $n$ = 5, $P$ < 0.05) (FIG. 14C and 14D) . Consistent with the above results, the final average tumor weight in mice treated with AGM-330t-PTX (1.3 ± 0.1 g for 19.05 mg/kg, n = 5) significantly decreased compared to mice injected with PTX (2.1 ± 0.4 and 1.5 ± 0.1 g for the 2 and 10 mg/kg treatment groups, respectively; $n$ = 5) (FIG. 14E).

The present inventors also performed histological staining of the excised tumors, and an independent pathologist evaluated the slides. Median tumors excised from AGM-330t-PTX-treated mice exhibited a reduction in the number of cancerous cells and an increase in apoptotic cell nuclei, as demonstrated by H&E staining, Ki-67 and TUNEL assays, respectively (FIGS. 14F to 14I). In contrast, the vehicle (PBS)- and AGM-330t-treated tumors contained larger numbers of tumor cells and exhibited few signs of apoptosis. Furthermore, mouse body weights were not affected by AGM-330t-PTX administration compared to vehicle (PBS) administration, and the blood chemistry analysis results revealed no adverse signs of toxicity in the AGM-330t-PTX-treated mice (FIGS. 15A to 15F). Accordingly, these findings suggest that the tumor specificity of AGM-330 may enhance the efficacy of PTX without detectable side effects. As a result, it is suggested that AGM-330 according to the present invention can be a potential therapeutic drug carrier for cancer treatment.

Additionally, to reduce the effects of PEGylation immunogenicity, the present inventors tested the anticancer

efficacy using AGM-330d-PTX, which had a lower extent of PEGylation than AGM-330t (FIG. 16A). As a result, AGM-330d-PTX-treated mice showed a dramatic reduction in luciferase activity compared with that observed in PTX-treated mice (FIGS. 16C and 16D). Additionally, AGM-330d-PTX treatment resulted in a significant decrease in the volume and weight of tumors compared with that observed in the PTX-treated group (FIGS. 16E to 16G). Consistent with the results obtained using AGM-330-PTX, mouse body weights were not affected by AGM-330d-PTX administration compared to that observed in the vehicle (PBS)-treated mice (FIG. 16H). The cancer specificity of AGM-330d might have enhanced the efficacy of PTX, increasing the therapeutic drug concentration in tumor tissues.

**Example 8: Synthesis of fusion peptide in which AGM-330 and CPP are fused together**

In order to increase the cell penetration ability of AGM-330, a fusion protein was produced by fusing a cell-penetrating peptide (CPP).

When AGM-330t is reacted with a maleimide-conjugated CPP (RIMRILRILKLAR; SEQ ID NO: 48) at pH 7.0 to 7.5, the functional group of AGM-330t, thiol group, is bound to AGM-330t by thiol-maleimide reaction, and thus an AGM-330t-mCPP fusion peptide is synthesized (FIG. 17).

As a result of HPLC and MS analysis of the produced AGM-330t-mCPP fusion peptide, it was confirmed that the fusion protein had a retention time of 23.2 minutes and a molecular weight of 7,876 Da. FIG. 18 shows the results of analysis of the fusion peptide together with the results of analysis of AGM-330t and maleimide-mCPP.

**Example 9: Evaluation of cytotoxicity of fusion peptide in which AGM-330 and CPP are fused together**

In order to examine the cytotoxicity of the AGM-330t-mCPP fusion peptide produced in Example 8, the present inventors evaluated its $IC_{50}$ values in various cancer cell lines, including MCF-7, MDA-MB-231, HT-29, HCT-116 and PANC-1, and the normal cell line CCD-18Co (Table 7).

[Table 7]

| Cell type | Cell line | $IC_{50}$ ($\mu$M) |
|---|---|---|
| Human breast cancer cell line | MCF-7 | 7.173 |
| Human breast cancer cell line | MDA-MB-231 | > 20 |
| Human colon cancer cell line | HT-29 | 7.829 |
| Human colon cancer cell line | HCT-116 | 4.714 |
| Human pancreas cancer cell line | PANC-1 | > 20 |
| Human colon normal cell line | CCD-18Co | > 20 |

The $IC_{50}$ values of the AGM-330t-mCPP fusion peptide were 7.829 and 4.714 $\mu$M for the colon cancer cell lines HT-29 and HCT-116, respectively, and 7.173 and more than 20 $\mu$M for the breast cancer cell lines MCF-7 and MDA-MB-231, respectively (FIG. 19). This means that the AGM-330t-mCPP fusion peptide has a cancer cell-specific inhibitory effect.

**Example 10: *In vitro* evaluation of cancer cell apoptosis-inducing effect of fusion peptide in which AGM-330 and CPP are fused together**

To investigate the cancer cell apoptosis-inducing effect of the AGM-330t-mCPP fusion peptide produced in Example 8, HCT-116, HT-29 and CCD-18Co cells, treated with CPP, AGM-330t and AGM-330t-CPP for 48 hours, were stained with Annexin V and PI. As a result, the cancer cell-specific apoptosis-inducing effect was confirmed (FIG. 20). As a result of the experiment conducted at each concentration of AGM-330t-mCPP, it was confirmed that the apoptosis-promoting protein increased in a concentration-dependent manner (FIG. 21) .

**Example 11: Evaluation of effect of combination of AGM-330 and AGM-330-CPP**

In order to investigate the cytotoxicity of each of the AGM-330m-mCPP, AGM-330d-dCPP and AGM-330t-tCPP fusion peptides produced in Example 8 and combinations thereof, the present inventors evaluated their $IC_{50}$ values in HCT-116, HT-29, MCF-7 and MDA-MB-231 cell lines.

The $IC_{50}$ values of the AGM-330m-mCPP, AGM-330d-dCPP and AGM-330t-tCPP fusion peptides were 23.3,

5.47 and 3.79 μM, respectively, for the colon cancer cell line HCT-116, and 20.67, 3.75 and 3.3 μM, respectively, for HT-29. In addition, the $IC_{50}$ values were >20, 4.208 and 3.802 μM, respectively, for the breast cancer cell line MCF-7, and >20, 3.843 and 3.497 μM, respectively, for MDA-MB-231 (FIG. 22). As a result, it can be confirmed that the AGM-330-CPP fusion peptide has a cancer cell-specific inhibitory effect.

The $IC_{50}$ values of a combination of AGM-330m and the AGM-330m-mCPP fusion peptide, a combination of AMG-330d and the AGM-330d-dCPP fusion peptide, and a combination of AGM-330t and the AGM-330t-tCPP fusion peptide were 14.3, 4.25 and 3.15 μM, respectively, for the colon cancer cell line HCT-116, and 14.2, 4.76 and 3.05 μM, respectively, for HT-29. In addition, the $IC_{50}$ values were 18.7, 3.416 and 3.309 μM, respectively, for the breast cancer cell line MCF-7, and 14.7, 3.662 and 3.258 μM, respectively, for MDA-MB-231 (FIG. 23). As a result, it can be confirmed that the combination of AGM-330 and AGM-330-CPP also exhibits a cancer cell-specific inhibitory effect.

**Industrial Applicability**

In the present invention, it has been confirmed that the AGM peptide ligand and variants thereof screened using the MAP synthesis method and the OBOC combinatorial method specifically bind to cancer cells, and that the conjugate thereof with an anticancer agent inhibits cancer growth (*in vitro* and *in vivo*). Therefore, the NCL-targeting AGM may be effectively used for cancer diagnosis and targeted drug delivery in cancer therapy.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

**Claims**

**1.** An AGM peptide that specifically binds to nucleolin (NCL), the AGM peptide comprising an amino acid sequence selected from the group consisting of:

(a) an amino acid sequence set forth in any one of SEQ ID NO: 1 to SEQ ID NO: 8, and
(b) an amino acid sequence comprising at least one amino acid mutation selected from the following group in the amino acid sequence set forth in SEQ ID NO: 1,

(i) a substitution for a methionine residue at position 5 from the N-terminus,
(ii) a substitution for a tyrosine residue at position 7 from the N-terminus, and
(iii) an insertion of a leucine or lysine residue at the C-terminus.

**2.** The AGM peptide according to claim 1, wherein the amino acid sequence comprising the amino acid mutation of (b) is selected from the group consisting of SEQ ID NO: 9 to SEQ ID NO: 15 and SEQ ID NO: 20.

**3.** An AGM peptide-PEG conjugate in which the AGM peptide according to claim 1 and a polyethylene glycol (PEG) chain are conjugated together.

**4.** The AGM peptide-PEG conjugate according to claim 3, wherein the polyethylene glycol chain comprises 2 to 24 ethylene glycol groups.

**5.** The AGM peptide-PEG conjugate according to claim 3, wherein the AGM peptide and the polyethylene glycol chain are linked together by a linker.

**6.** An AGM peptide-PEG-drug conjugate comprising the AGM peptide-PEG conjugate according to claim 3 and a drug.

**7.** The AGM peptide-PEG-drug conjugate according to claim 6, wherein the drug is any one or more selected from the group consisting of taxols and taxanes, including paclitaxel or docetaxel, maytansinoids, auristatin, aminopterin, actinomycin, bleomycin, thalidomide, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, alpha-amanitin, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, pyrrolobenzodiazepine, carmustine, lomustine, busulfan, treosulfan, dacarbazine, etoposide, teniposide, topotecan, 9-aminocamptothecin, crisnatol, mitomycin C, trimetrexate, mycophenolic acid, tiazofurin, ribavirin, 5-ethynyl-1-beta-ribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, berberine, verteporfin, curcumin, Abraxane, FOLFIRINOX, oxaliplatin, Xeloda and indole carboxamide, phthalocyanine, tubulysin, photosensitizer Pe4, demethoxy-hypocrellin A, interferon-a, interferon-γ, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants.

**8.** The AGM peptide-PEG-drug conjugate according to claim 6, wherein the AGM peptide-PEG conjugate and the drug are linked together by a linker.

**9.** A multimer comprising two or more AGM peptide-PEG conjugates according to claim 3.

**10.** The multimer according to claim 9, comprising 2 to 8 AGM peptide-PEG conjugates.

**11.** The multimer according to claim 9, wherein two or more AGM peptide-PEG conjugates are linked together by a linker.

**12.** A multimer-drug conjugate comprising the multimer according to claim 9 and a drug.

**13.** The multimer-drug conjugate according to claim 12, wherein the drug is any one or more selected from the group consisting of taxols and taxanes, including paclitaxel or docetaxel, maytansinoids, auristatin, aminopterin, actinomycin, bleomycin, thalidomide, camptothecin, N8-acetylspermidine, 1-(2-chloroethyl)-1,2-dimethyl sulfonyl hydrazide, esperamicin, etoposide, 6-mercaptopurine, dolastatin, trichothecene, calicheamicin, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, Cytoxan, alpha-amanitin, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, pyrrolobenzodiazepine, carmustine, lomustine, busulfan, treosulfan, dacarbazine, etoposide, teniposide, topotecan, 9-aminocamptothecin, crisnatol, mitomycin C, trimetrexate, mycophenolic acid, tiazofurin, ribavirin, 5-ethynyl-1-beta-ribofuranosylimidazole-4-carboxamide (EICAR), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, berberine, verteporfin, curcumin, Abraxane, FOLFIRINOX, oxaliplatin, Xeloda and indole carboxamide, phthalocyanine, tubulysin, photosensitizer Pe4, demethoxy-hypocrellin A, interferon-a, interferon-γ, tumor necrosis factor, gemcitabine, Velcade, Revlimid, Thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins derived from bacteria or animals and plants.

**14.** The multimer-drug conjugate according to claim 13, wherein the multimer and the drug are linked together by a linker.

**15.** An AGM peptide-PEG-CPP fusion peptide in which the AGM peptide-PEG conjugate according to claim 3 and a cell-penetrating peptide (CPP) are fused together.

**16.** The AGM peptide-PEG-CPP fusion peptide according to claim 15, wherein the cell-penetrating peptide is selected from the group consisting of the amino acid sequences of SEQ ID NO: 31 to SEQ ID NO: 80.

**17.** The AGM peptide-PEG-CPP fusion peptide according to claim 15, wherein the AGM peptide-PEG conjugate and the cell-penetrating peptide are linked together by a linker.

**18.** A multimer-CPP fusion peptide in which the multimer according to claim 9 and a cell-penetrating peptide (CPP) are fused together.

**19.** A composition for diagnosing cancer comprising the AGM peptide according to claims 1 to 3, the AGM peptide-PEG

conjugate according to claim 4 or 5, the AGM peptide-PEG-drug conjugate according to claims 6 to 8, the multimer according to claims 9 to 11, the multimer-drug conjugate according to claims 12 to 14, the AGM peptide-PEG-CPP fusion peptide according to claims 15 to 17, or the multimer-CPP fusion peptide according to claim 18.

**20.** A composition for preventing or treating cancer comprising the AGM peptide according to claims 1 to 3, the AGM peptide-PEG conjugate according to claim 4 or 5, the AGM peptide-PEG-drug conjugate according to claims 6 to 8, the multimer according to claims 9 to 11, the multimer-drug conjugate according to claims 12 to 14, the AGM peptide-PEG-CPP fusion peptide according to claims 15 to 17, or the multimer-CPP fusion peptide according to claim 18.

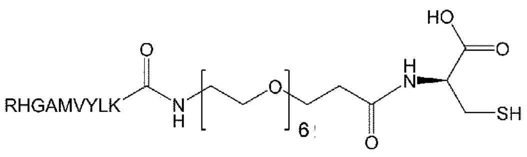

**AGM-330m**
Monomeric AGM-330

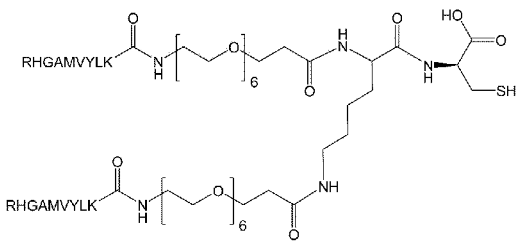

**AGM-330d**
Dimeric AGM-330

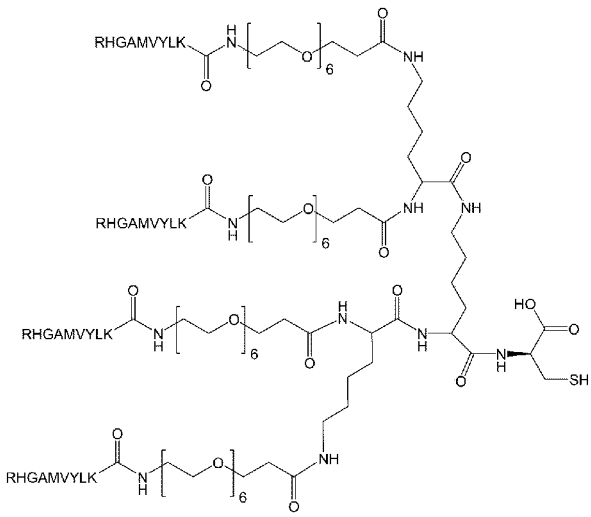

**AGM-330t**
Tetrameric AGM-330

Figure 1

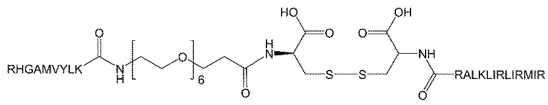

**AGM-330m-mCPP**
Monomeric AGM-330 – Monomeric CPP

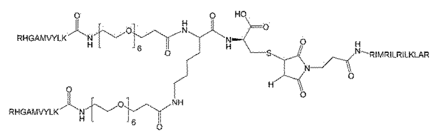

**AGM-330d-mCPP**
Monomeric AGM-330 – Monomeric CPP

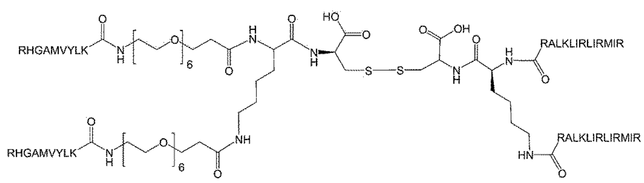

**AGM-330d-dCPP**
Dimeric AGM-330 – Dimeric CPP

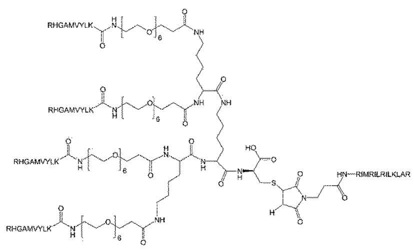

**AGM-330t-mCPP**
Tetrameric AGM-330 – Monomeric CPP

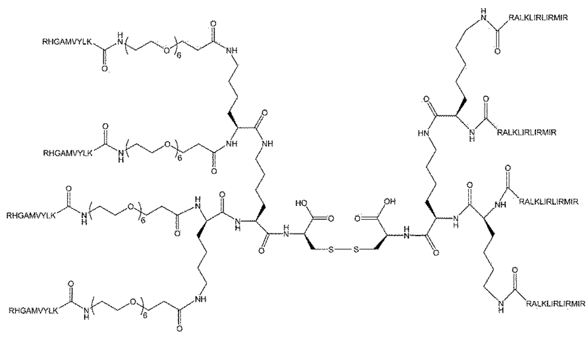

**AGM-330t-tCPP**
Tetrameric AGM-330 – Tetrameric CPP

Figure 2

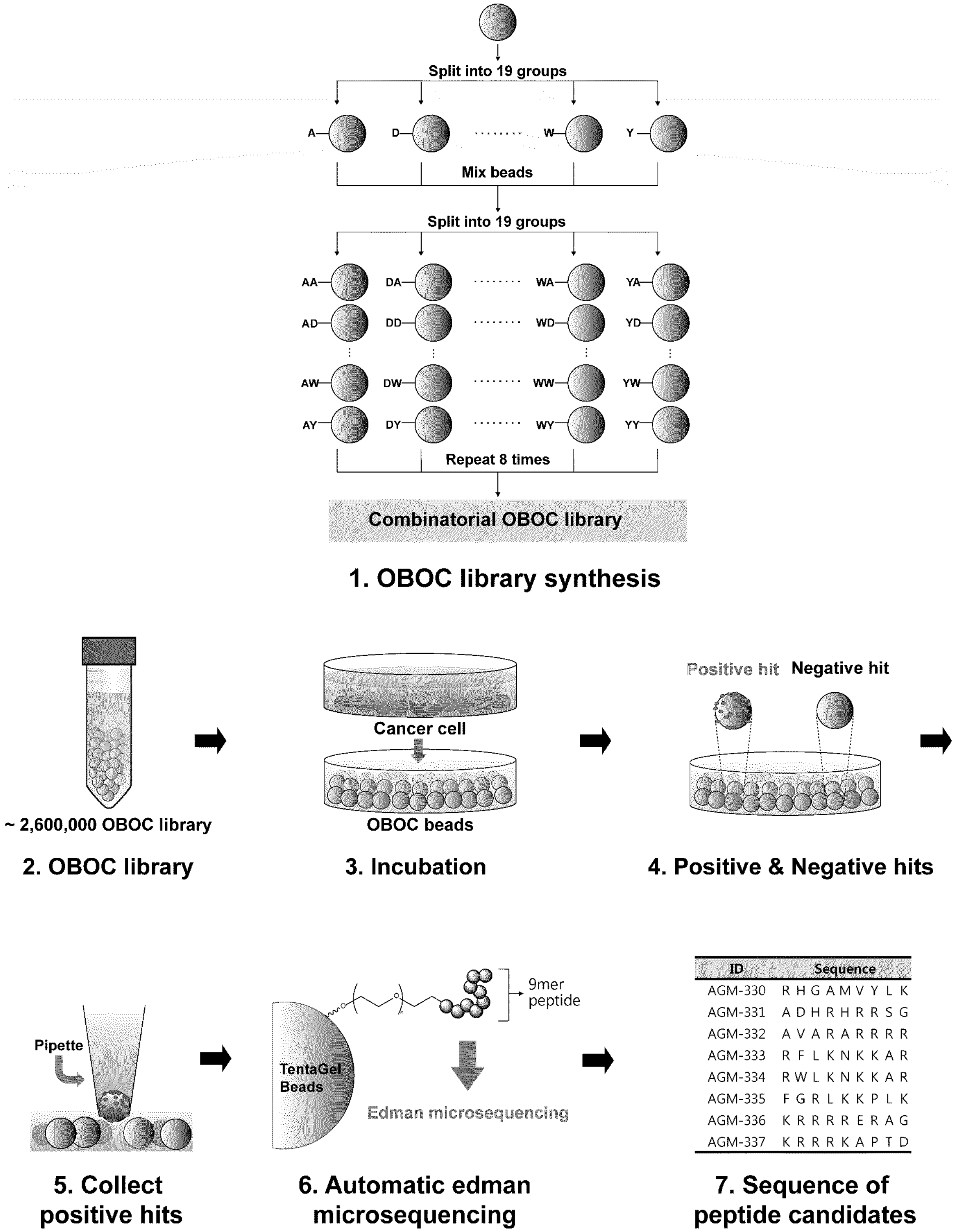
Split into 19 groups
A
D
W
Y
Mix beads
Split into 19 groups
AA DA WA YA
AD DD WD YD
AW DW WW YW
AY DY WY YY
Repeat 8 times
Combinatorial OBOC library
1. OBOC library synthesis
~ 2,600,000 OBOC library
2. OBOC library
Cancer cell
OBOC beads
3. Incubation
Positive hit
Negative hit
4. Positive & Negative hits
Pipette
5. Collect positive hits
TentaGel Beads
9mer peptide
Edman microsequencing
6. Automatic edman microsequencing
ID Sequence
AGM-330 R H G A M V Y L K
AGM-331 A D H R H R R S G
AGM-332 A V A R A R R R R
AGM-333 R F L K N K K A R
AGM-334 R W L K N K K A R
AGM-335 F G R L K K P L K
AGM-336 K R R R R E R A G
AGM-337 K R R R K A P T D
7. Sequence of peptide candidates

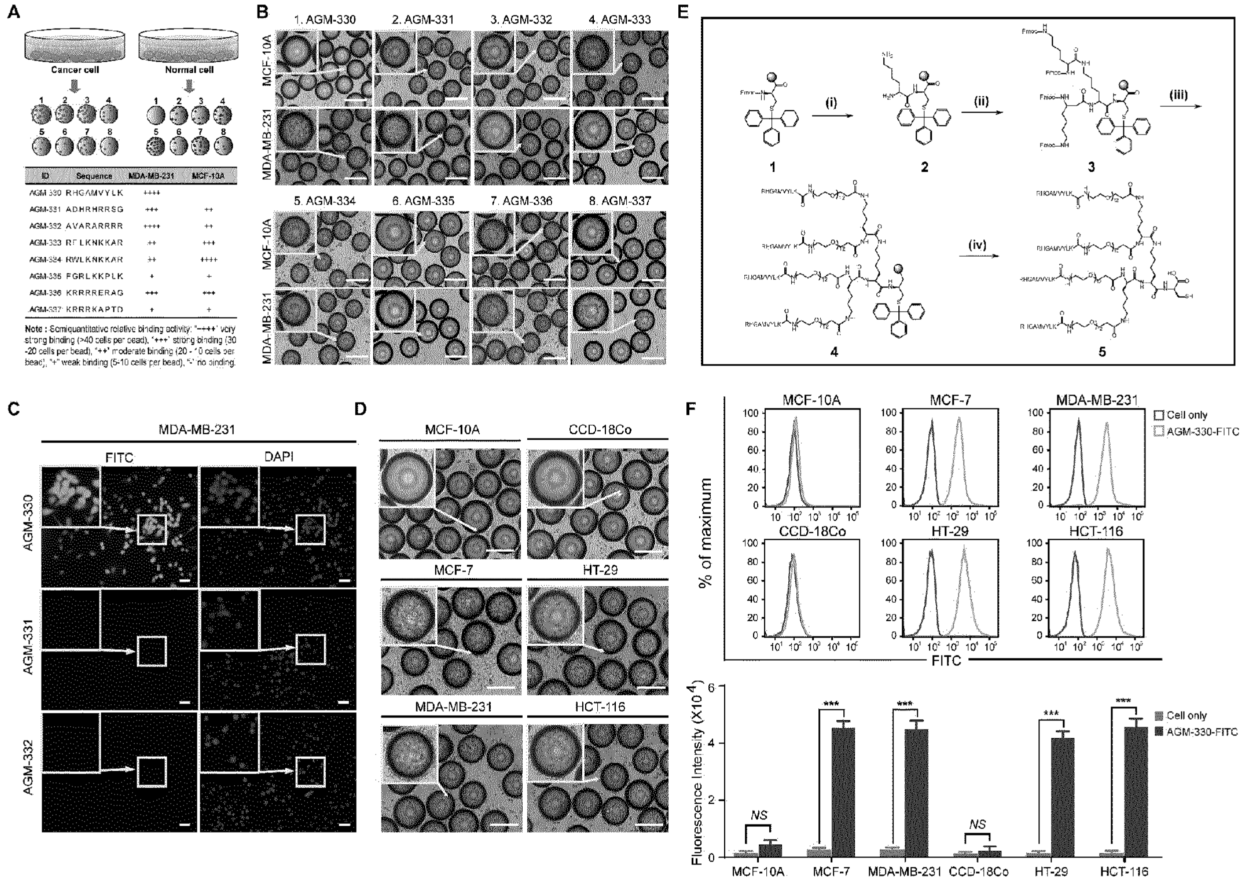
A
Cancer cell
Normal cell
B
1. AGM-330
2. AGM-331
3. AGM-332
4. AGM-333
5. AGM-334
6. AGM-335
7. AGM-336
8. AGM-337
MCF-10A
MDA-MB-231
E
(i)
(ii)
(iii)
(iv)
1
2
3
4
5
C
MDA-MB-231
FITC
DAPI
AGM-330
AGM-331
AGM-332
D
MCF-10A
CCD-18Co
MCF-7
HT-29
MDA-MB-231
HCT-116
F
% of maximum
FITC
Cell only
AGM-330-FITC
Fluorescence Intensity (X10⁴)
NS
***
MCF-10A
MCF-7
MDA-MB-231
CCD-18Co
HT-29
HCT-116

Figure 4

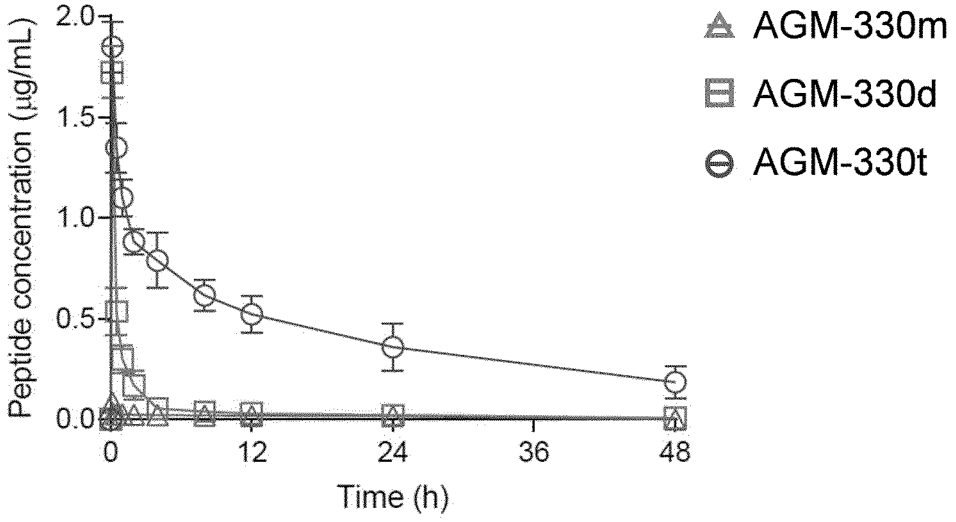


| Parameters | AGM-330m | AGM-330d | AGM-330t |
|---|---|---|---|
| $T_{1/2}$ (h) | 0.42 ± 0.33 | 1.82 ± 0.36 | 9.43 ± 1.21 |
| $T_{max}$ (h) | 0.167 | 0.167 | 0.167 |
| $C_{max}$ (µg/ml) | 1.27 ± 0.12 | 1.71 ± 0.11 | 1.875 ± 0.67 |
| AUC (µg/h/ml) | 0.64 ± 0.09 | 1.77 ± 0.12 | 21.42 ± 0.81 |

$T_{1/2}$: The distribution half-time; $T_{max}$: Time to reach maximum plasma concentration; $C_{max}$: Maximum plasma concentration; AUC: Area under the plasma concentration-time curve

Figure 5

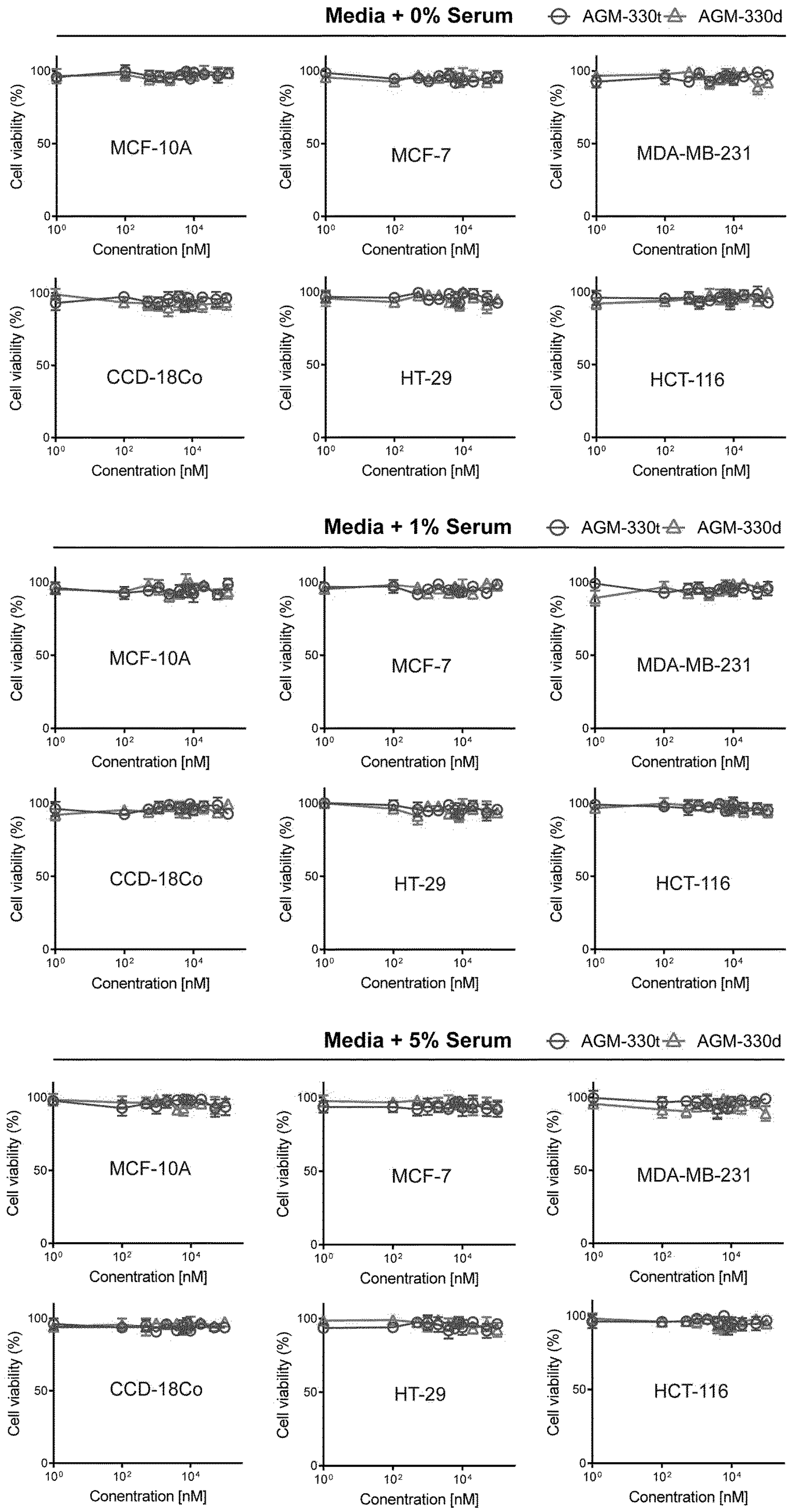
Media + 0% Serum
AGM-330t
AGM-330d
MCF-10A
MCF-7
MDA-MB-231
CCD-18Co
HT-29
HCT-116
Media + 1% Serum
AGM-330t
AGM-330d
MCF-10A
MCF-7
MDA-MB-231
CCD-18Co
HT-29
HCT-116
Media + 5% Serum
AGM-330t
AGM-330d
MCF-10A
MCF-7
MDA-MB-231
CCD-18Co
HT-29
HCT-116
Cell viability (%)
Conentration [nM]

Figure 6

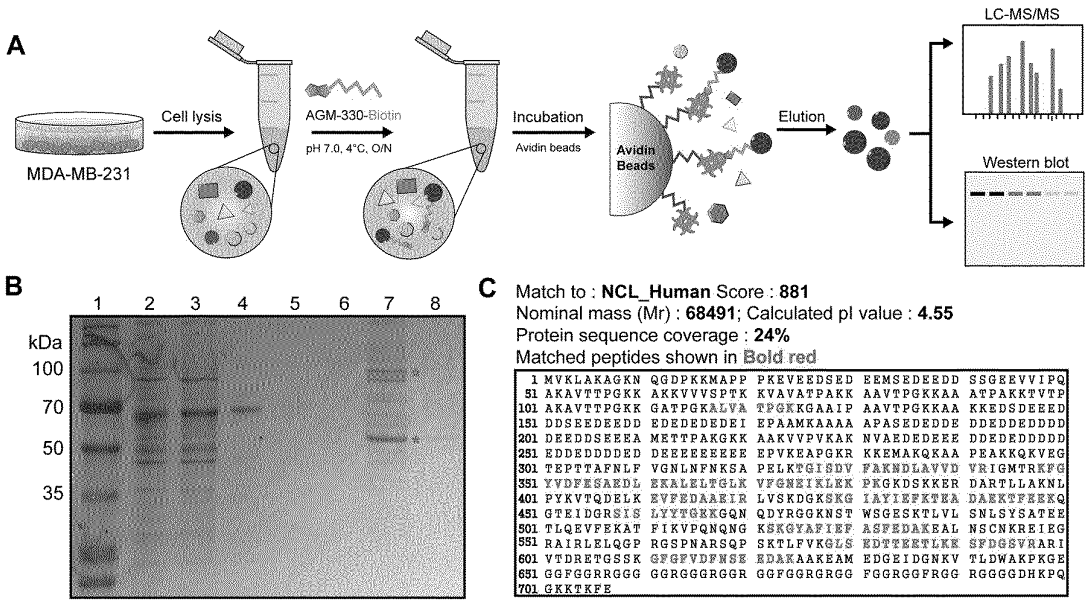


D

| Gene name | NM number | Protein identification | Confidence score[a] | Peptide count | Mass |
|---|---|---|---|---|---|
| NCL | NM_005381 | Nucleolin | 881 | 22 | 68491 |
| ACTN4 | NM_004924 | Actinin alpha 4 | 155 | 12 | 105353 |
| ACTN1 | NM_001102 | Actinin alpha 1 | 127 | 8 | 103666 |
| HSP90B1 | NM_003299 | Heat shock protein 90 beta family member 1 | 110 | 3 | 90362 |
| VCP | NM_007126 | Valosin containing protein | 83 | 5 | 90019 |
| COPG1 | NM_016128 | Coatomer protein complex subunit gamma 1 | 65 | 2 | 79201 |

[a] Condidence score obtained from Mascot.

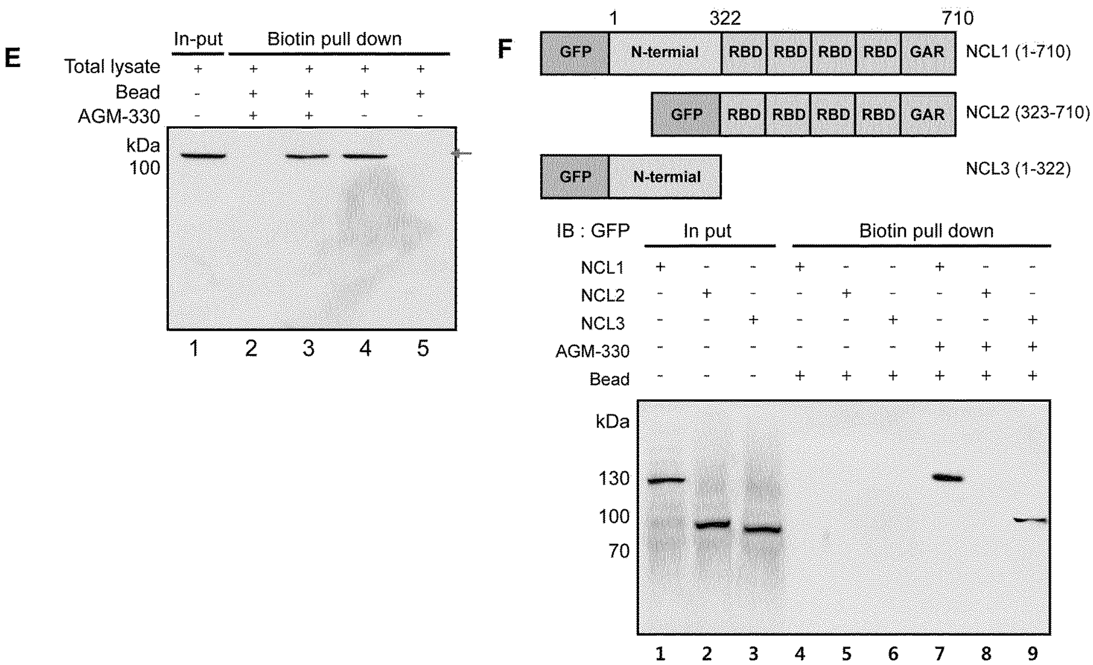


Figure 7

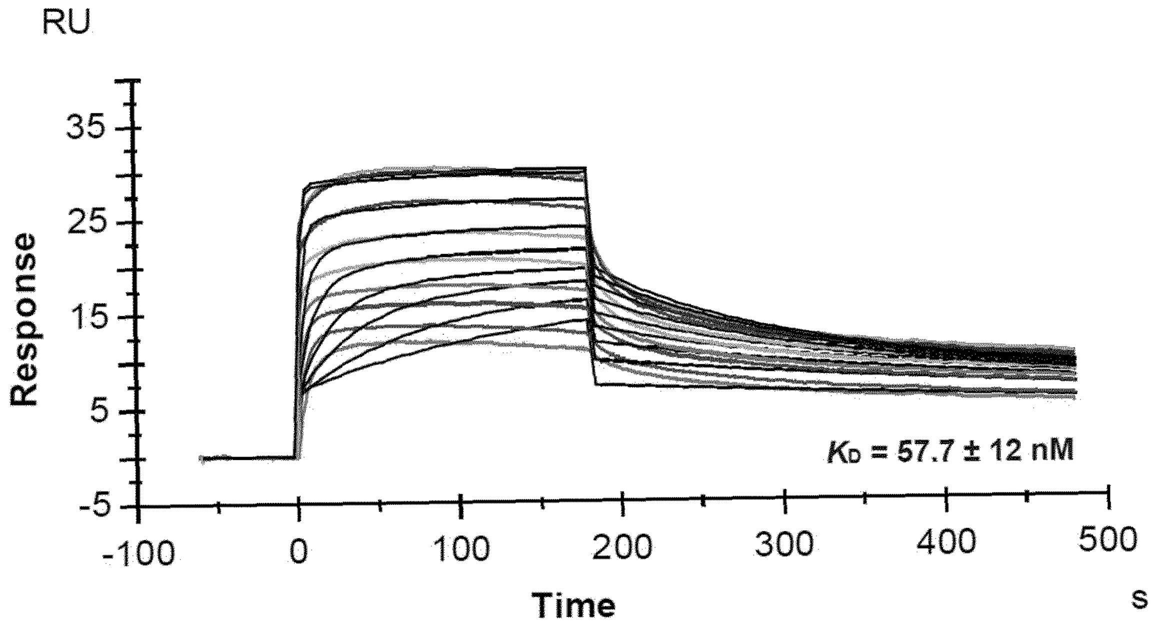
RU
35
25
15
5
-5
Response
-100
0
100
200
300
400
500
Time
s
KD = 57.7 ± 12 nM

Figure 8

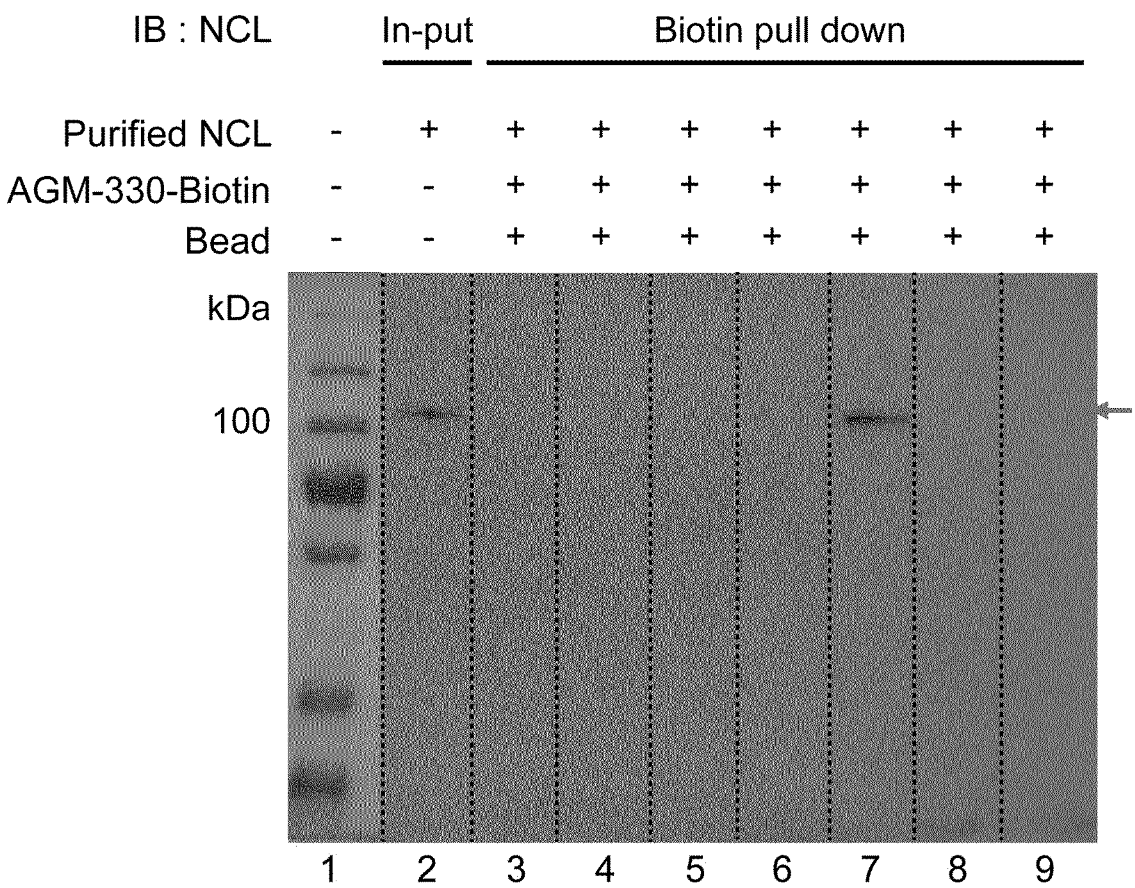


1 : Protein marker

2 : Purified nucleolin

3 : Flow through

4 ~ 6 : Washing

7 ~ 9 : Elution

Figure 9

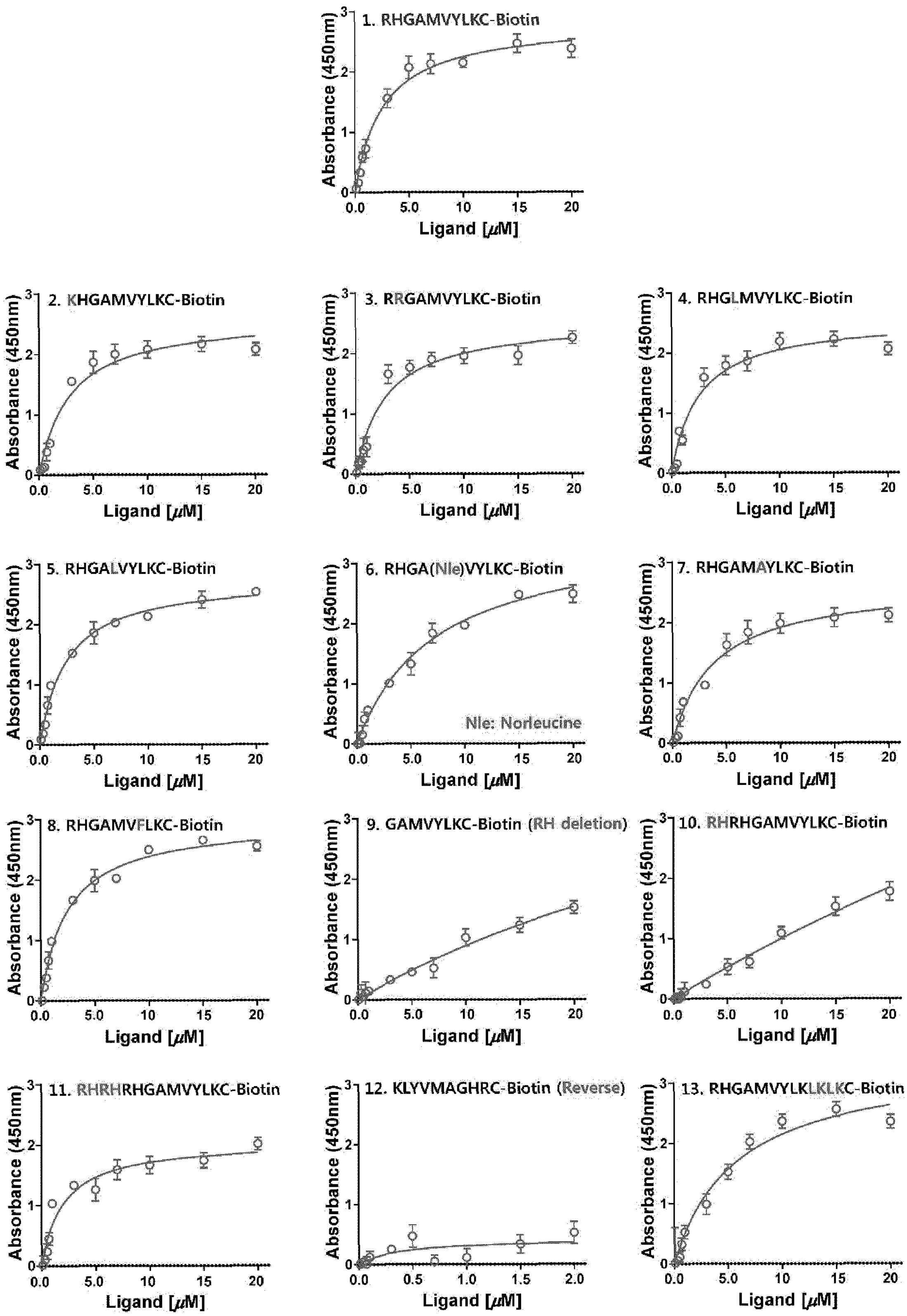
1. RHGAMVYLKC-Biotin
2. KHGAMVYLKC-Biotin
3. RRGAMVYLKC-Biotin
4. RHGLMVYLKC-Biotin
5. RHGALVYLKC-Biotin
6. RHGA(Nle)VYLKC-Biotin
Nle: Norleucine
7. RHGAMAYLKC-Biotin
8. RHGAMVFLKC-Biotin
9. GAMVYLKC-Biotin (RH deletion)
10. RHRHGAMVYLKC-Biotin
11. RHRHRHGAMVYLKC-Biotin
12. KLYVMAGHRC-Biotin (Reverse)
13. RHGAMVYLKLKLKC-Biotin
Absorbance (450nm)
Ligand [μM]

Figure 10

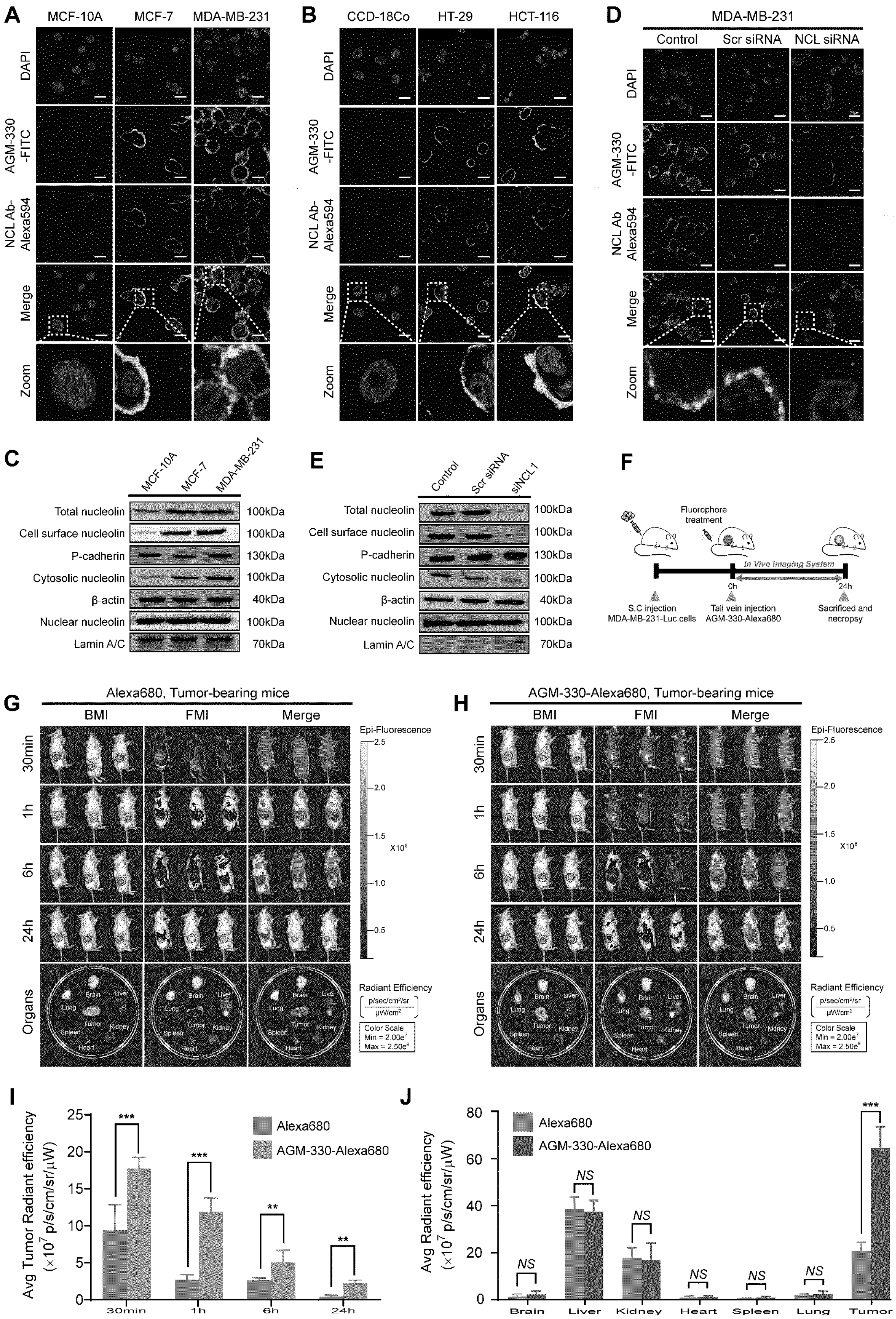

A
MCF-10A
MCF-7
MDA-MB-231
DAPI
AGM-330 -FITC
NCL Ab-Alexa594
Merge
Zoom
B
CCD-18Co
HT-29
HCT-116
D
MDA-MB-231
Control
Scr siRNA
NCL siRNA
C
Total nucleolin
Cell surface nucleolin
P-cadherin
Cytosolic nucleolin
β-actin
Nuclear nucleolin
Lamin A/C
100kDa
130kDa
40kDa
70kDa
E
siNCL1
F
Fluorophore treatment
In Vivo Imaging System
0h
24h
S.C injection MDA-MB-231-Luc cells
Tail vein injection AGM-330-Alexa680
Sacrificed and necropsy
G
Alexa680, Tumor-bearing mice
BMI
FMI
Merge
30min
1h
6h
24h
Organs
Epi-Fluorescence
Radiant Efficiency
H
AGM-330-Alexa680, Tumor-bearing mice
I
Avg Tumor Radiant efficiency ($\times10^7$ p/s/cm/sr/μW)
Alexa680
AGM-330-Alexa680
J
Avg Radiant efficiency ($\times10^7$ p/s/cm/sr/μW)
Brain
Liver
Kidney
Heart
Spleen
Lung
Tumor
NS


Figure 11

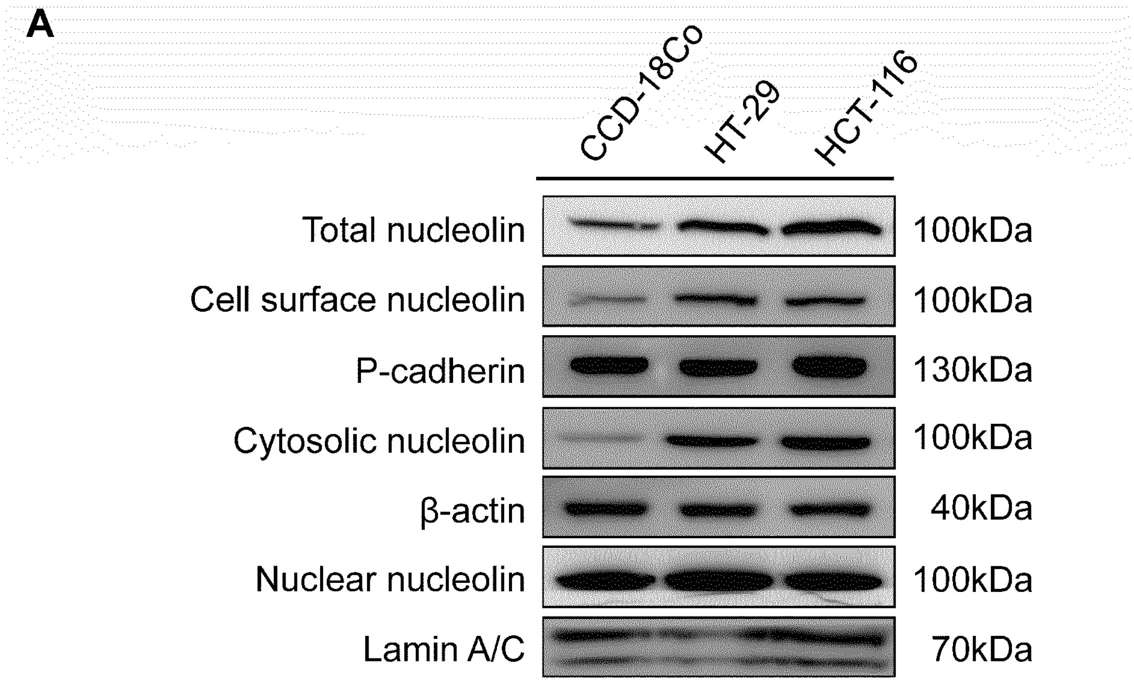
A
CCD-18Co
HT-29
HCT-116
Total nucleolin
100kDa
Cell surface nucleolin
100kDa
P-cadherin
130kDa
Cytosolic nucleolin
100kDa
β-actin
40kDa
Nuclear nucleolin
100kDa
Lamin A/C
70kDa

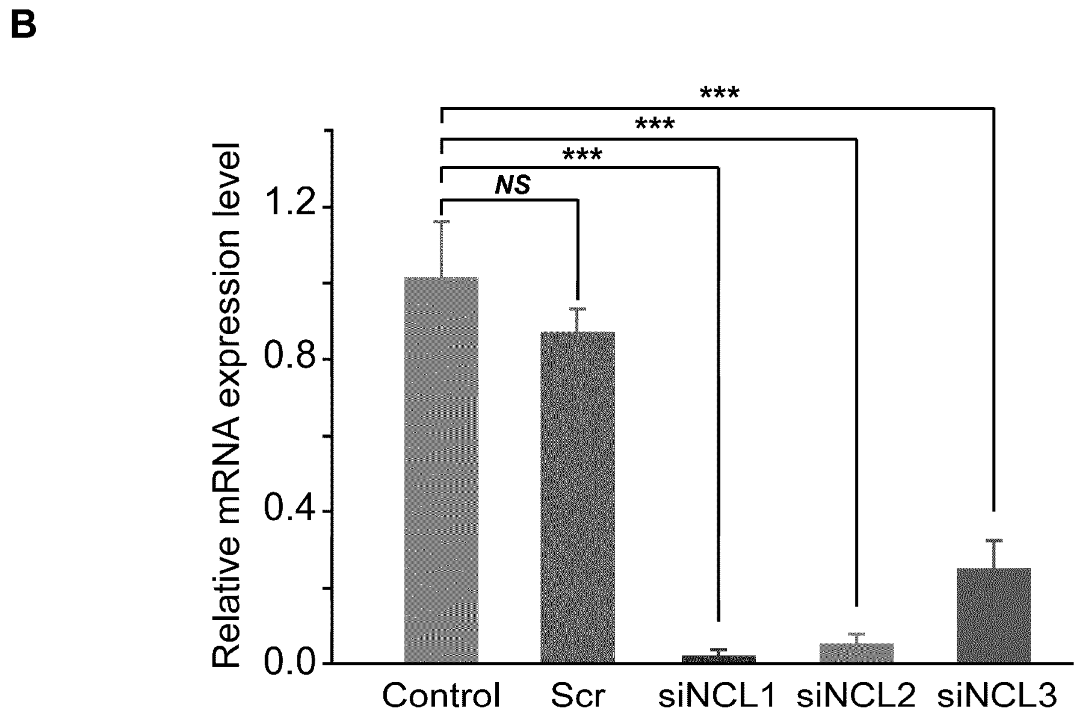
B
***
***
***
NS
Relative mRNA expression level
1.2
0.8
0.4
0.0
Control
Scr
siNCL1
siNCL2
siNCL3

Figure 12

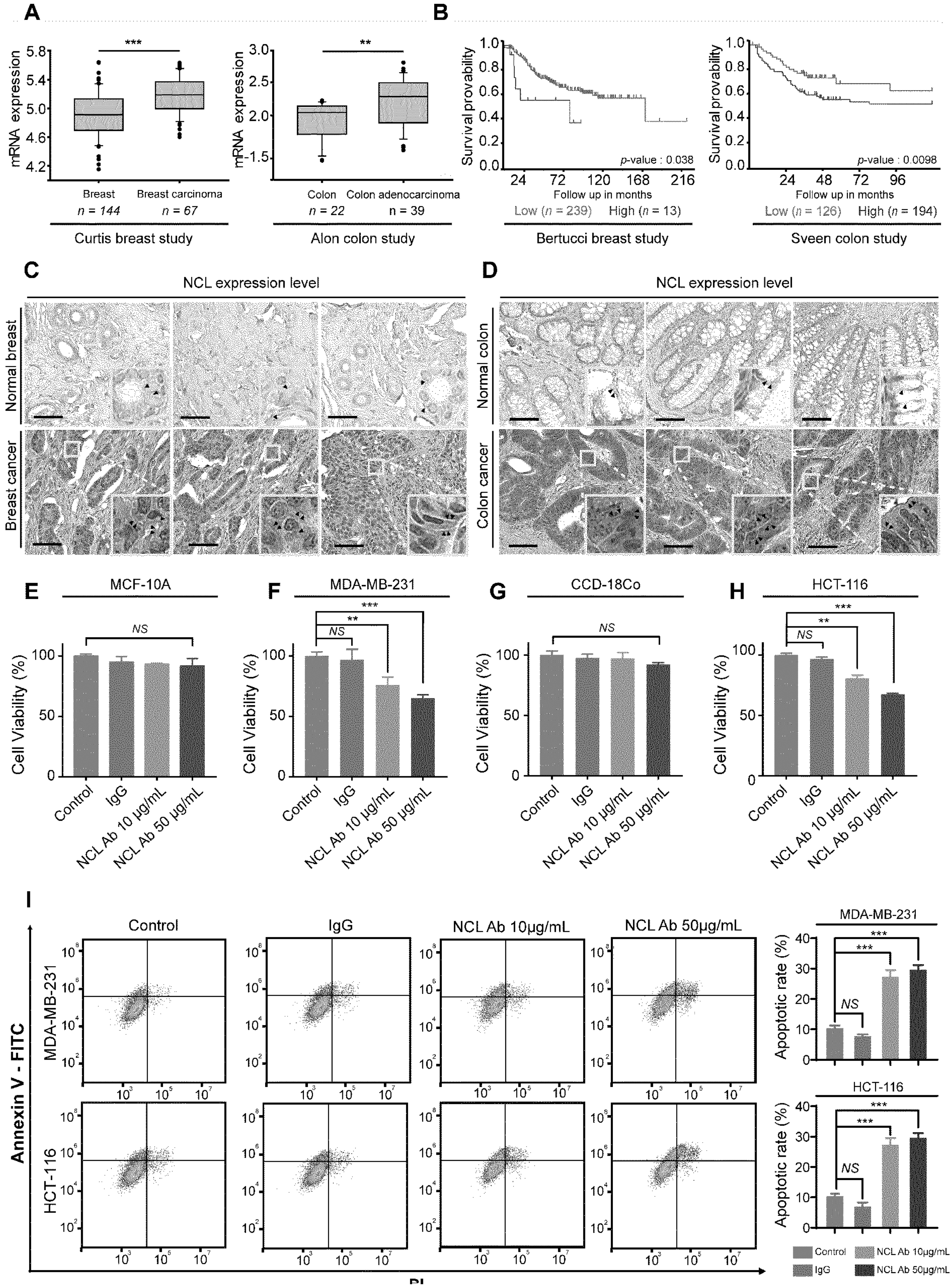
A
B
Curtis breast study
Alon colon study
Bertucci breast study
Sveen colon study
mRNA expression
Breast
Breast carcinoma
n = 144
n = 67
Colon
Colon adenocarcinoma
n = 22
n = 39
Survival provability
Follow up in months
p-value : 0.038
p-value : 0.0098
Low (n = 239) High (n = 13)
Low (n = 126) High (n = 194)
C
D
NCL expression level
Normal breast
Breast cancer
Normal colon
Colon cancer
E
F
G
H
MCF-10A
MDA-MB-231
CCD-18Co
HCT-116
Cell Viability (%)
Control
IgG
NCL Ab 10 µg/mL
NCL Ab 50 µg/mL
NS
I
Control
IgG
NCL Ab 10µg/mL
NCL Ab 50µg/mL
Annexin V - FITC
PI
Apoptotic rate (%)

Figure 13

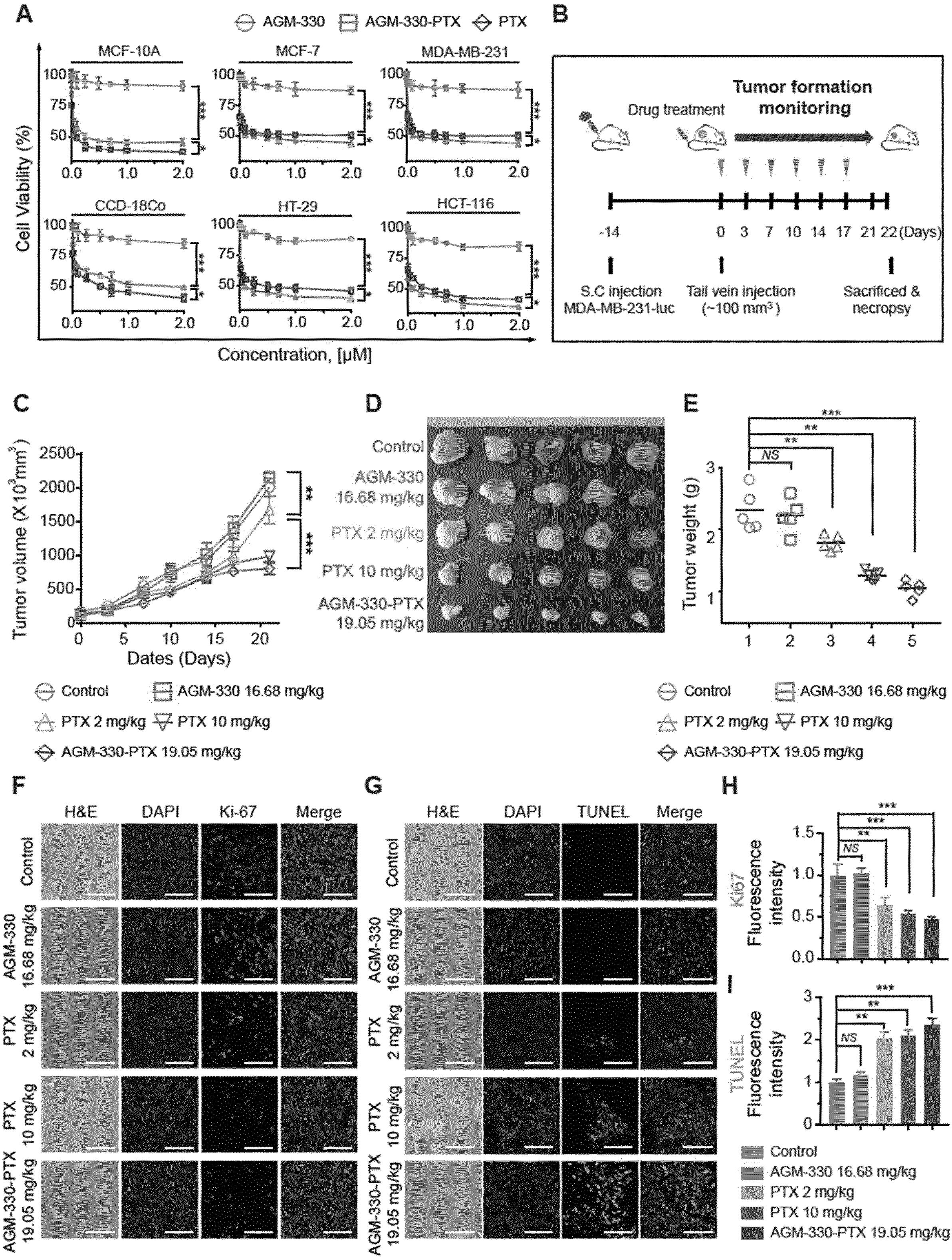
A
AGM-330
AGM-330-PTX
PTX
MCF-10A
MCF-7
MDA-MB-231
CCD-18Co
HT-29
HCT-116
Cell Viability (%)
Concentration, [μM]
B
Drug treatment
Tumor formation monitoring
-14
0 3 7 10 14 17 21 22 (Days)
S.C injection MDA-MB-231-luc
Tail vein injection (~100 mm³)
Sacrificed & necropsy
C
Tumor volume (X10³mm³)
Dates (Days)
D
Control
AGM-330 16.68 mg/kg
PTX 2 mg/kg
PTX 10 mg/kg
AGM-330-PTX 19.05 mg/kg
E
Tumor weight (g)
NS
Control
AGM-330 16.68 mg/kg
PTX 2 mg/kg
PTX 10 mg/kg
AGM-330-PTX 19.05 mg/kg
F
H&E
DAPI
Ki-67
Merge
G
H&E
DAPI
TUNEL
Merge
H
Ki67 Fluorescence intensity
I
TUNEL Fluorescence intensity
Control
AGM-330 16.68 mg/kg
PTX 2 mg/kg
PTX 10 mg/kg
AGM-330-PTX 19.05 mg/kg

Figure 14

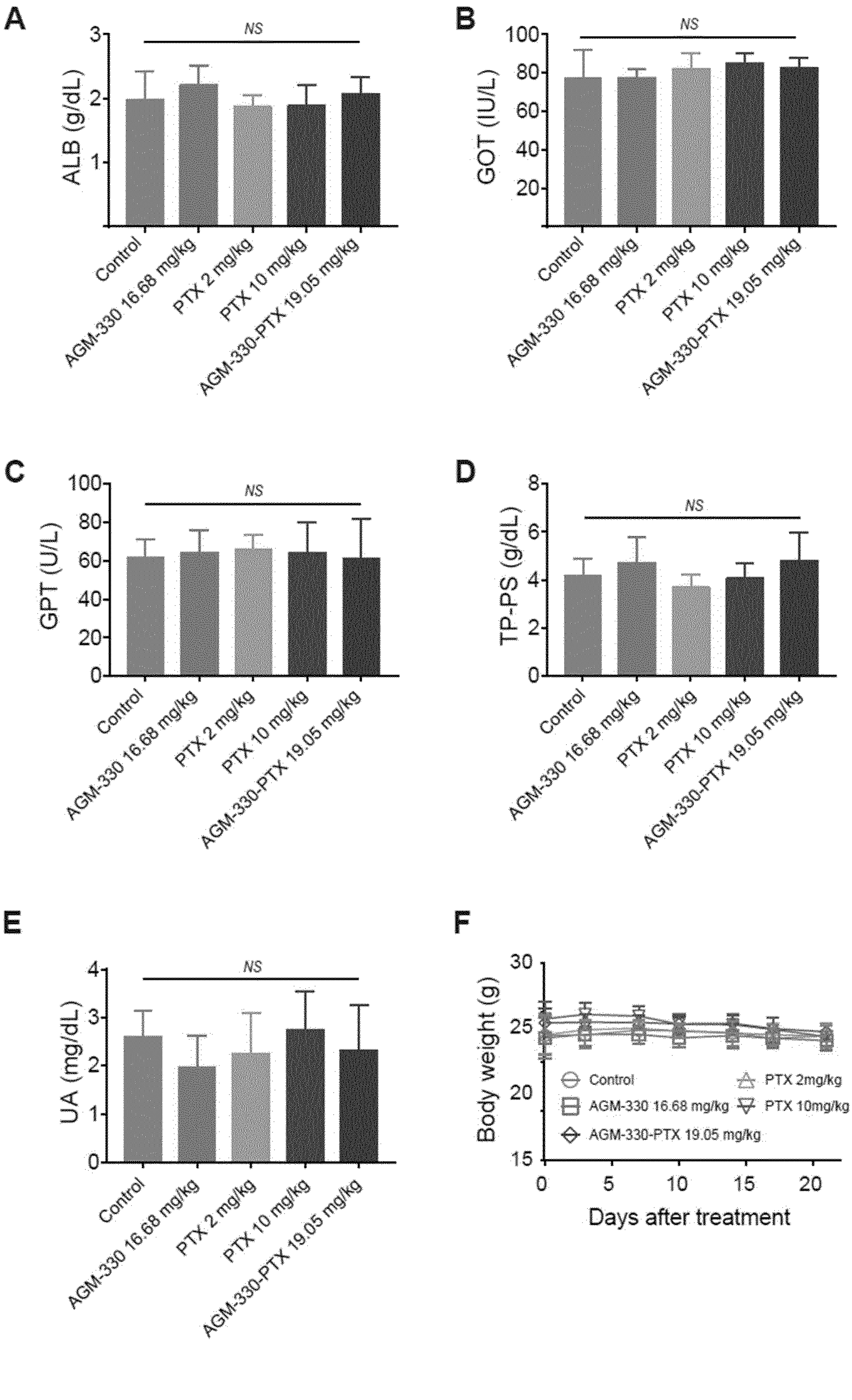
A
ALB (g/dL)
NS
Control
AGM-330 16.68 mg/kg
PTX 2 mg/kg
PTX 10 mg/kg
AGM-330-PTX 19.05 mg/kg
B
GOT (IU/L)
C
GPT (U/L)
D
TP-PS (g/dL)
E
UA (mg/dL)
F
Body weight (g)
Days after treatment
Control
PTX 2mg/kg
AGM-330 16.68 mg/kg
PTX 10mg/kg
AGM-330-PTX 19.05 mg/kg

Figure 15

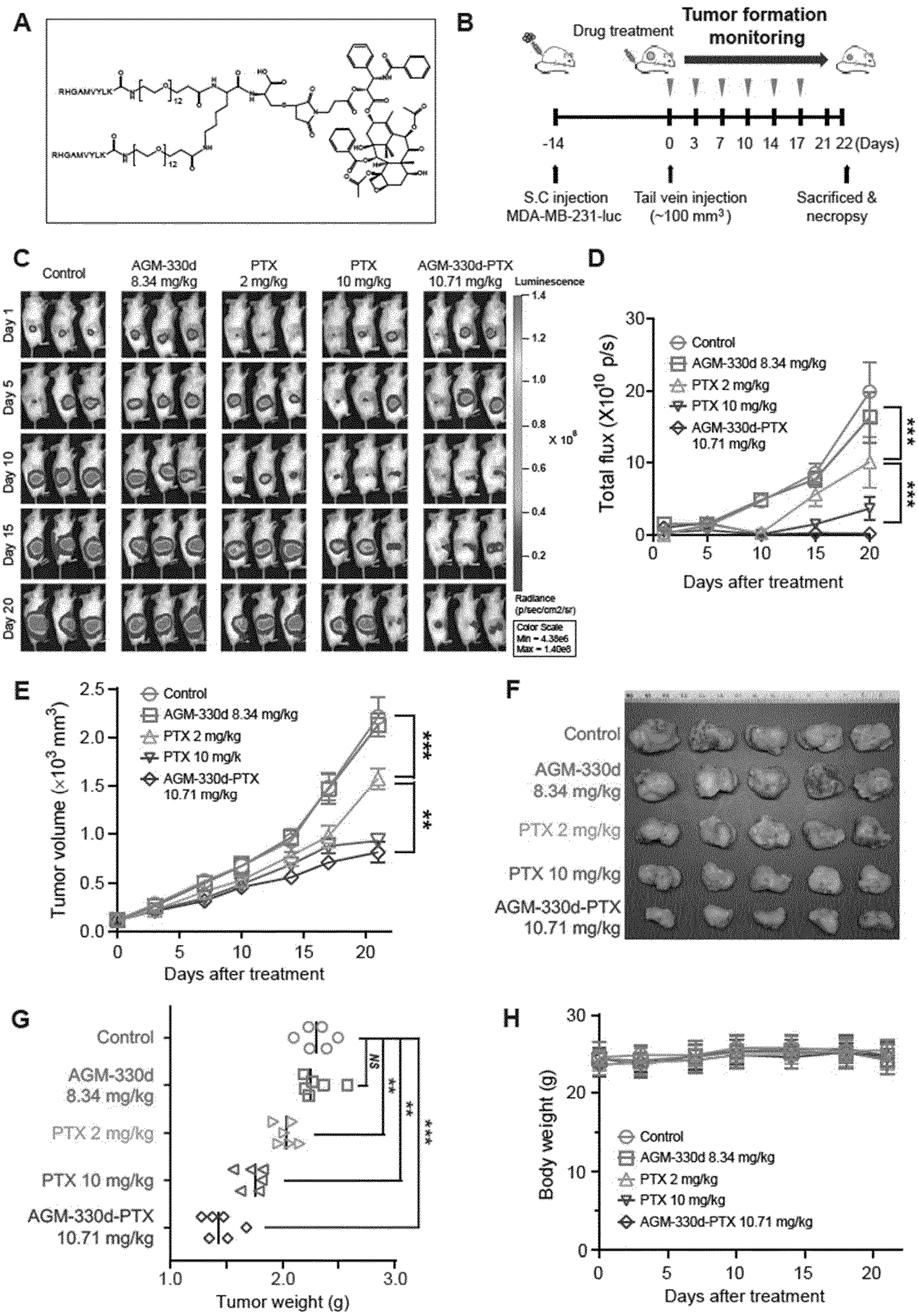
A
RHGAMVYLK
RHGAMVYLK
B
Drug treatment
Tumor formation monitoring
-14
0 3 7 10 14 17 21 22 (Days)
S.C injection MDA-MB-231-luc
Tail vein injection (~100 mm³)
Sacrificed & necropsy
C
Control
AGM-330d 8.34 mg/kg
PTX 2 mg/kg
PTX 10 mg/kg
AGM-330d-PTX 10.71 mg/kg
Luminescence
Day 1
Day 5
Day 10
Day 15
Day 20
Radiance (p/sec/cm2/sr)
Color Scale Min = 4.38e6 Max = 1.40e8
D
Total flux (X10^10 p/s)
Days after treatment
Control
AGM-330d 8.34 mg/kg
PTX 2 mg/kg
PTX 10 mg/kg
AGM-330d-PTX 10.71 mg/kg
E
Tumor volume (×10³ mm³)
Days after treatment
Control
AGM-330d 8.34 mg/kg
PTX 2 mg/kg
PTX 10 mg/k
AGM-330d-PTX 10.71 mg/kg
F
Control
AGM-330d 8.34 mg/kg
PTX 2 mg/kg
PTX 10 mg/kg
AGM-330d-PTX 10.71 mg/kg
G
Control
AGM-330d 8.34 mg/kg
PTX 2 mg/kg
PTX 10 mg/kg
AGM-330d-PTX 10.71 mg/kg
NS
Tumor weight (g)
H
Body weight (g)
Days after treatment
Control
AGM-330d 8.34 mg/kg
PTX 2 mg/kg
PTX 10 mg/kg
AGM-330d-PTX 10.71 mg/kg

Figure 16

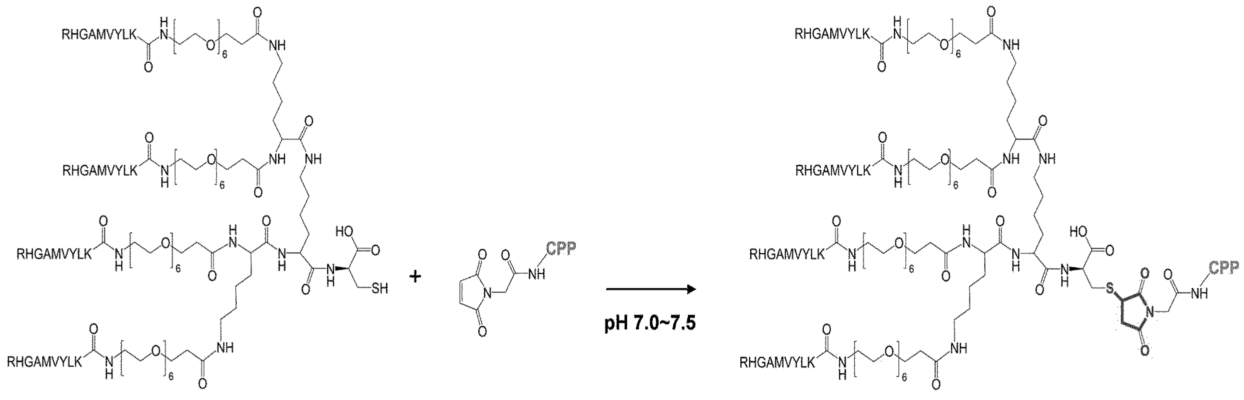


| | | |
|---|---|---|
| **AGM-330t** | **Maleimide-mCPP** | **AGM-330t-mCPP** |
| Cell surface nucleolin targeting ligand | Maleimide conjugated CPP | Intracellular nucleolin targeting ligand |

Figure 17

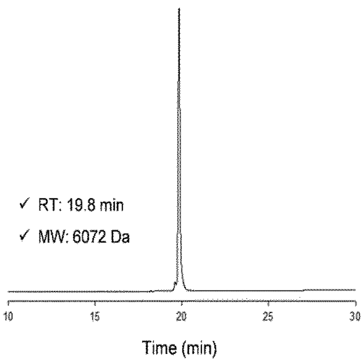


AGM-330t

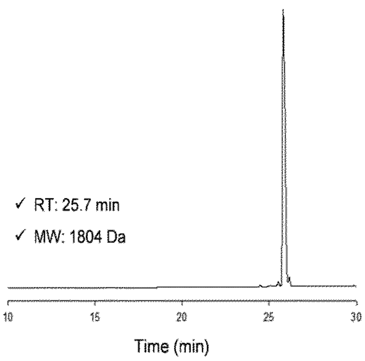


Maleimide-mCPP

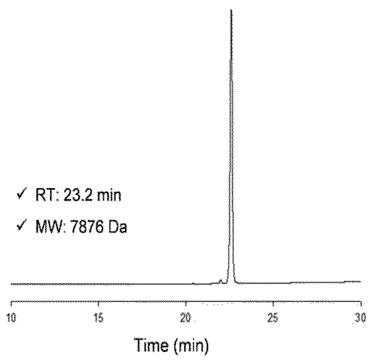


AGM-330t-mCPP

Figure 18

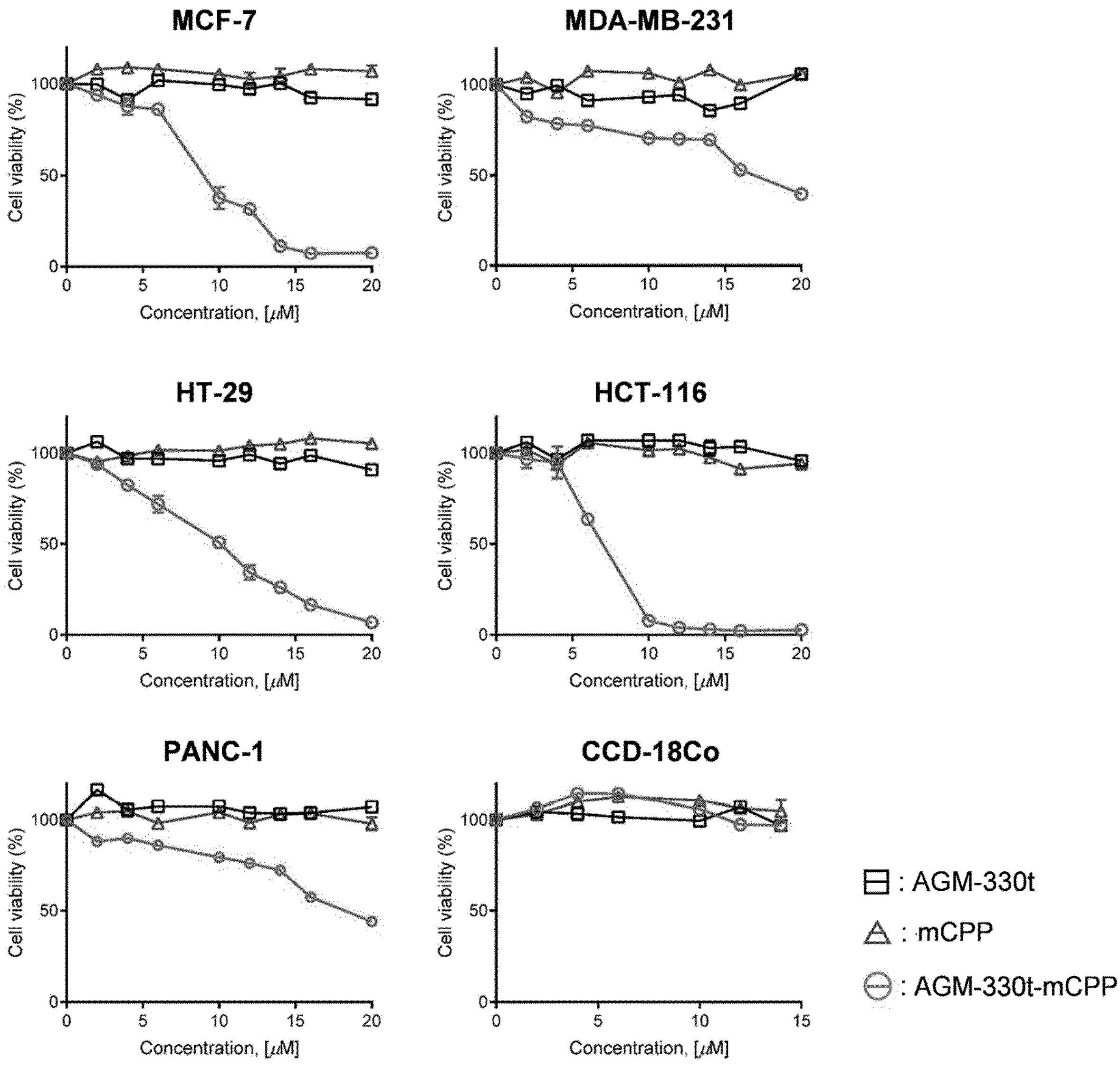
MCF-7
MDA-MB-231
HT-29
HCT-116
PANC-1
CCD-18Co
Cell viability (%)
Concentration, [μM]
0
50
100
5
10
15
20
: AGM-330t
: mCPP
: AGM-330t-mCPP

Figure 19

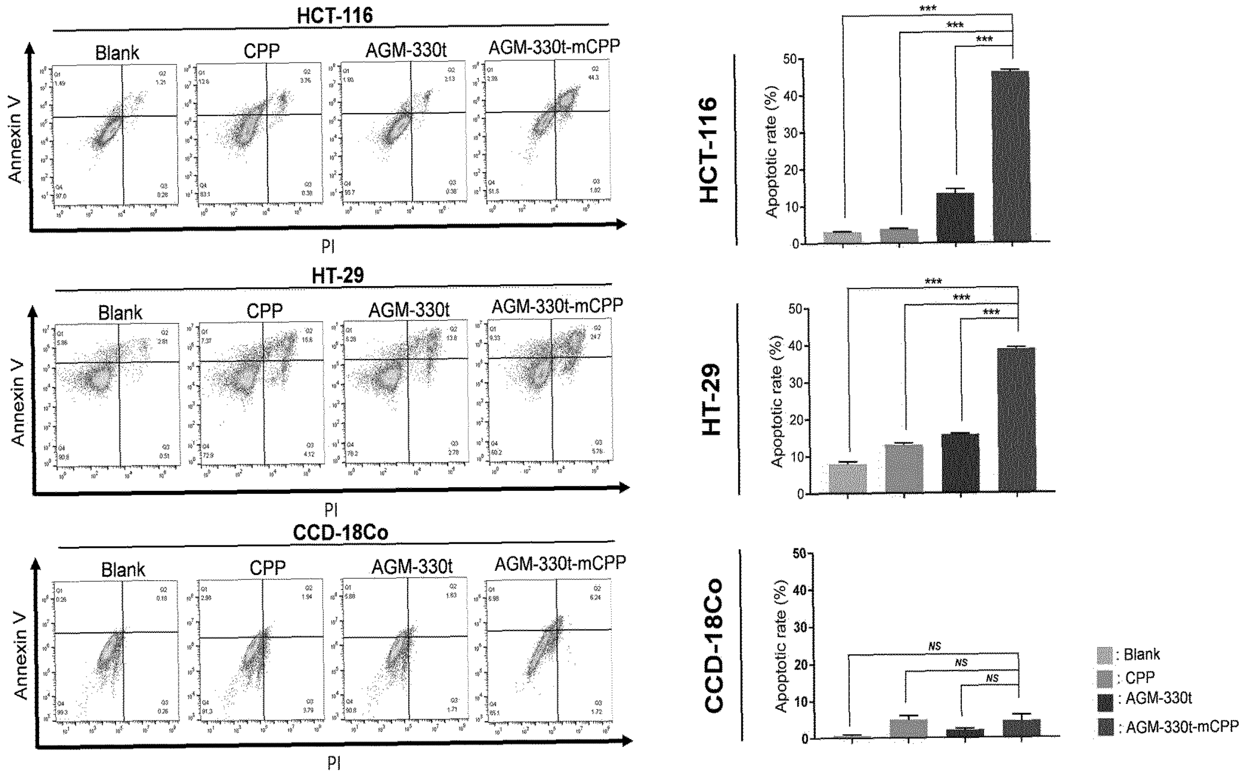
HCT-116
Blank
CPP
AGM-330t
AGM-330t-mCPP
Annexin V
PI
HT-29
CCD-18Co
Apoptotic rate (%)
***
NS
: Blank
: CPP
: AGM-330t
: AGM-330t-mCPP

Figure 20

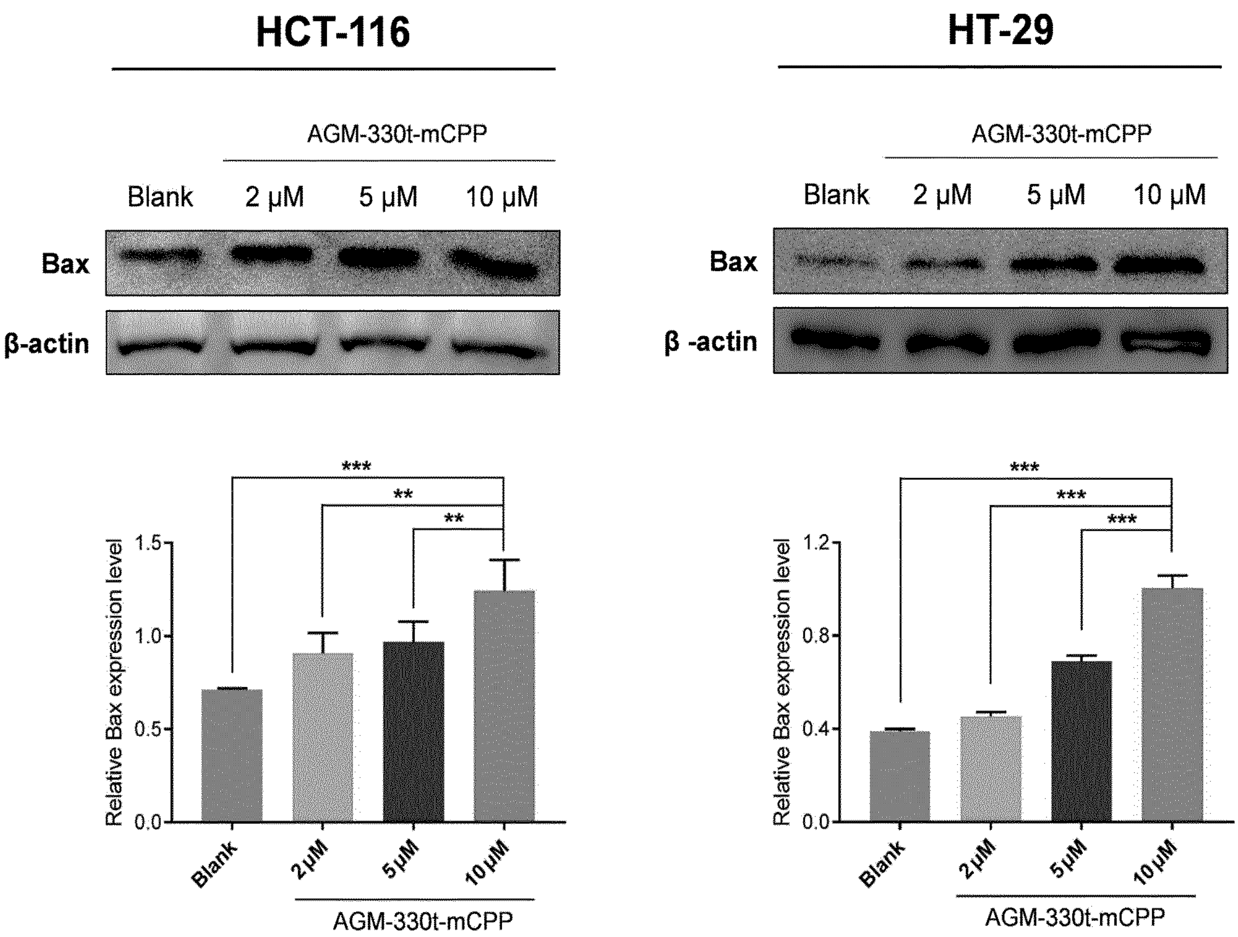
HCT-116
AGM-330t-mCPP
Blank
2 μM
5 μM
10 μM
Bax
β-actin
***
**
**
Relative Bax expression level
1.5
1.0
0.5
0.0
Blank
2μM
5μM
10μM
AGM-330t-mCPP
HT-29
AGM-330t-mCPP
Blank
2 μM
5 μM
10 μM
Bax
β -actin
***
***
***
Relative Bax expression level
1.2
0.8
0.4
0.0
Blank
2μM
5μM
10μM
AGM-330t-mCPP

Figure 21

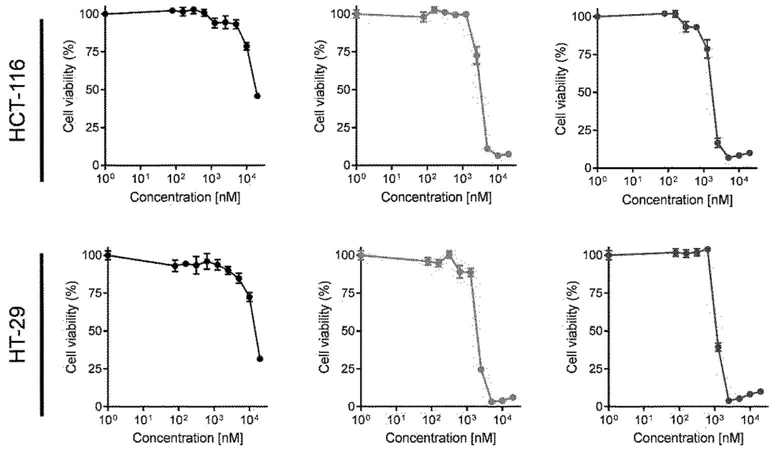


**AGM-330m-mCPP**
(**Monomeric** AGM-330-**Monomeric** CPP)

**AGM-330d-dCPP**
(**Dimeric** AGM-330-**Dimeric** CPP)

**AGM-330t-tCPP**
(Tetrameric AGM-330-tetrameric CPP)

| **Cell line: HCT-116** | |
|---|---|
| **Type** | **$IC_{50}$** |
| AGM-330m-mCPP | 23.3 μM |
| AGM-330d-dCPP | 5.47 μM |
| AGM-330t-tCPP | 3.79 μM |

| **Cell line: HT-29** | |
|---|---|
| **Type** | **$IC_{50}$** |
| AGM-330m-mCPP | 20.67 μM |
| AGM-330d-dCPP | 3.75 μM |
| AGM-330t-tCPP | 3.3 μM |

* Human colon cancer cell : HCT-116, HT-29

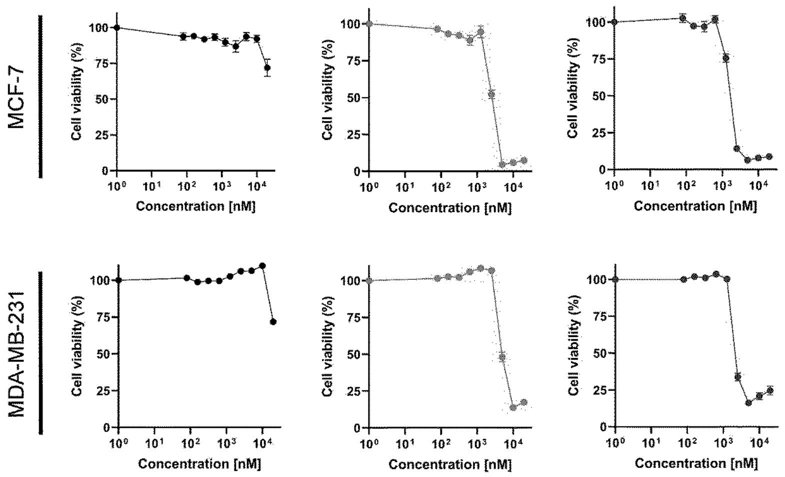


| **Cell line: MCF-7** | |
|---|---|
| **Type** | **$IC_{50}$** |
| AGM-330m-mCPP | > 20 μM |
| AGM-330d-dCPP | 4.208 μM |
| AGM-330t-tCPP | 3.802 μM |

| **Cell line: MDA-MB-231** | |
|---|---|
| **Type** | **$IC_{50}$** |
| AGM-330m-mCPP | > 20 μM |
| AGM-330d-dCPP | 3.843 μM |
| AGM-330t-tCPP | 3.497 μM |

* Human breast cancer cell : MCF-7, MDA-MB-231

**AGM-330m-mCPP**
(**Monomeric** AGM-330-**Monomeric** CPP)

**AGM-330d-dCPP**
(**Dimeric** AGM-330-**Dimeric** CPP)

**AGM-330t-tCPP**
(Tetrameric AGM-330-tetrameric CPP)

Figure 22

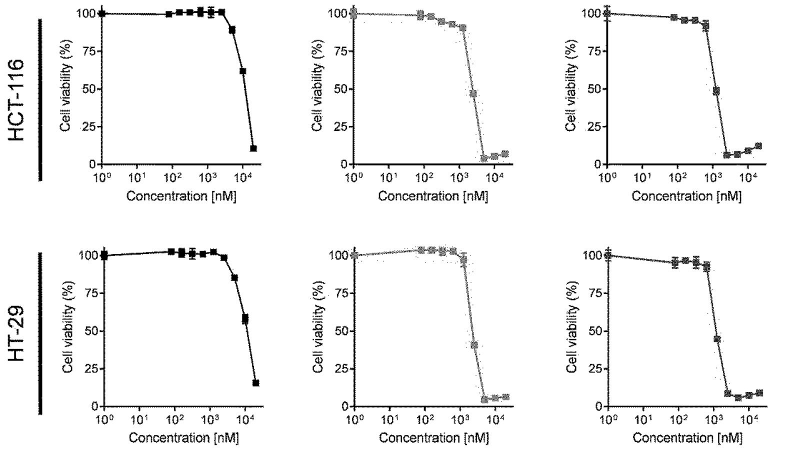


AGM-330m + AGM-330m-mCPP (Monomeric AGM-330-Monomeric CPP)

AGM-330d + AGM-330d-dCPP (Dimeric AGM-330-Dimeric CPP)

AGM-330t + AGM-330t-tCPP (Tetrameric AGM-330-tetrameric CPP)

| Cell line: HCT-116 | |
|---|---|
| Type | $IC_{50}$ |
| AGM-330m + AGM-330m-mCPP | 14.3 μM |
| AGM-330d + AGM-330d-dCPP | 4.25 μM |
| AGM-330t + AGM-330t-tCPP | 3.15 μM |

| Cell line: HT-29 | |
|---|---|
| Type | $IC_{50}$ |
| AGM-330m + AGM-330m-mCPP | 14.2 μM |
| AGM-330d + AGM-330d-dCPP | 4.76 μM |
| AGM-330t + AGM-330t-tCPP | 3.05 μM |

* Human colon cancer cell line : HCT-116, HT-29

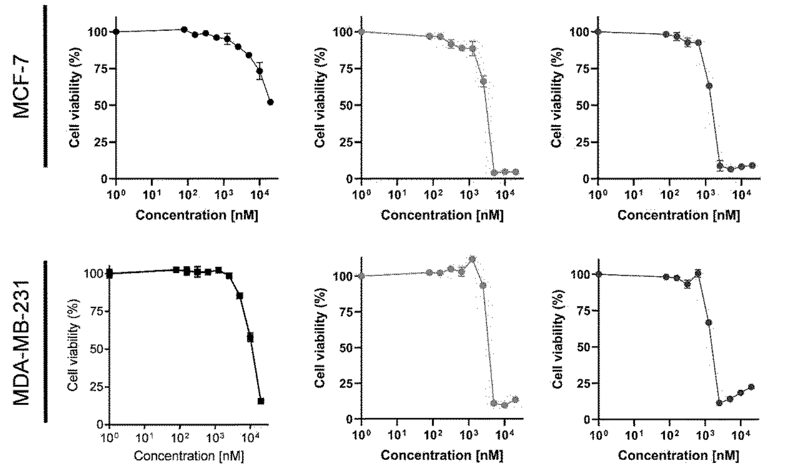


AGM-330m + AGM-330m-mCPP (Monomeric AGM-330-Monomeric CPP)

AGM-330d + AGM-330d-dCPP (Dimeric AGM-330-Dimeric CPP)

AGM-330t + AGM-330t-tCPP (Tetrameric AGM-330-tetrameric CPP)

| Cell line: MCF-7 | |
|---|---|
| Type | $IC_{50}$ |
| AGM-330m + AGM-330m-mCPP | 18.7 μM |
| AGM-330d + AGM-330d-dCPP | 3.416 μM |
| AGM-330t + AGM-330t-tCPP | 3.309 μM |

| Cell line: MDA-MB-231 | |
|---|---|
| Type | $IC_{50}$ |
| AGM-330m + AGM-330m-mCPP | 14.7 μM |
| AGM-330d + AGM-330d-dCPP | 3.662 μM |
| AGM-330t + AGM-330t-tCPP | 3.258 μM |

* Human breast cancer cell : MCF-7, MDA-MB-231

Figure 23

**INTERNATIONAL SEARCH REPORT**

International application No. **PCT/IB2021/054235**

**A. CLASSIFICATION OF SUBJECT MATTER**

**C07K 7/06**(2006.01)i; **A61K 47/60**(2017.01)i; **A61K 47/64**(2017.01)i; **A61P 35/00**(2006.01)i

According to International Patent Classification (IPC) or to both national classification and IPC

**B. FIELDS SEARCHED**

Minimum documentation searched (classification system followed by classification symbols)

C07K 7/06(2006.01); A61K 39/395(2006.01); A61K 47/48(2006.01); C07K 16/28(2006.01); C07K 16/30(2006.01); C07K 7/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 뉴클레오린(nucleolin, NCL), AGM 펩타이드(AGM peptide), 아미노산(amino acid), 변이(variant), 치환(alteration), 삽입(insertion), 폴리에틸렌글리콜(polyethylene glycol, PEG), 약물(drug), 접합체(conjugate), 다량체(multimer), 세포투과성 펩타이드(cell penetrating peptide, CPP), 융합 펩타이드(fusion peptide), 암(cancer)

**C. DOCUMENTS CONSIDERED TO BE RELEVANT**

| Category* | Citation of document, with indication, where appropriate, of the relevant passages | Relevant to claim No. |
|---|---|---|
| A | WO 2011-031477 A2 (ESPERANCE PHARMACEUTICALS, INC.) 17 March 2011 (2011-03-17)<br>See abstract, claims 1-3 and 42, and paragraphs [0011]-[0012]. | 1-20 |
| A | US 2015-0031851 A1 (SANFORD-BUMHAM MEDICAL RESEARCH INSTITUTE) 29 January 2015 (2015-01-29)<br>See entire document. | 1-20 |
| A | WO 2017-156032 A1 (CHARLESTONPHARMA, LLC et al.) 14 September 2017 (2017-09-14)<br>See entire document. | 1-20 |
| A | US 2013-0115674 A1 (SUTKOWSKI, N. et al.) 09 May 2013 (2013-05-09)<br>See entire document. | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 August 2021** | **23 August 2021** |

Name and mailing address of the ISA/KR
**Korean Intellectual Property Office**
**Government Complex-Daejeon Building 4, 189 Cheongsa-ro, Seo-gu, Daejeon 35208**
Facsimile No. **+82-42-481-8578**

Authorized officer

Telephone No.

Form PCT/ISA/210 (second sheet) (July 2019)

**INTERNATIONAL SEARCH REPORT**

International application No.
**PCT/IB2021/054235**

**C. DOCUMENTS CONSIDERED TO BE RELEVANT**

| Category* | Citation of document, with indication, where appropriate, of the relevant passages | Relevant to claim No. |
|---|---|---|
| A | FUJIKI, H. et al. Cell-surface nucleolin acts as a central mediator for carcinogenic, anti-carcinogenic, and disease-related ligands. Journal of Cancer Research and Clinical Oncology. 2014, vol. 140, pp. 689-699. See entire document. | 1-20 |
| PX | KIM, J.-H. et al. A novel nucleolin-binding peptide for cancer theranostics. Theranostics. 14 July 2020, vol . 10, no. 20, pp. 9153-9171. See entire document. | 1-20 |

Form PCT/ISA/210 (second sheet) (July 2019)

**INTERNATIONAL SEARCH REPORT**

International application No.
**PCT/IB2021/054235**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/IB2021/054235**

| Patent document cited in search report | Publication date (day/month/year) | Patent family member(s) | Publication date (day/month/year) |
|---|---|---|---|
| WO 2011-031477 A2 | 17 March 2011 | US 2011-0124564 A1 | 26 May 2011 |
| | | WO 2011-031477 A3 | 21 July 2011 |
| US 2015-0031851 A1 | 29 January 2015 | US 2004-0186056 A1 | 23 September 2004 |
| | | US 2009-0098633 A1 | 16 April 2009 |
| | | US 2012-0121501 A1 | 17 May 2012 |
| | | US 7544767 B2 | 09 June 2009 |
| | | US 8048983 B2 | 01 November 2011 |
| | | US 8598112 B2 | 03 December 2013 |
| | | US 9320810 B2 | 26 April 2016 |
| | | WO 03-087124 A2 | 23 October 2003 |
| | | WO 2003-087124 A3 | 17 August 2006 |
| WO 2017-156032 A1 | 14 September 2017 | CN 109071669 A | 21 December 2018 |
| | | EP 3426691 A1 | 16 January 2019 |
| | | JP 2019-513013 A | 23 May 2019 |
| | | US 2019-0194334 A1 | 27 June 2019 |
| US 2013-0115674 A1 | 09 May 2013 | CN 102770529 A | 07 November 2012 |
| | | CN 102770529 B | 05 June 2018 |
| | | CN 108707584 A | 26 October 2018 |
| | | EP 2501800 A2 | 26 September 2012 |
| | | EP 3037435 A1 | 29 June 2016 |
| | | EP 3037435 B1 | 07 August 2019 |
| | | JP 2013-511260 A | 04 April 2013 |
| | | JP 2016-116536 A | 30 June 2016 |
| | | JP 2018-117628 A | 02 August 2018 |
| | | JP 6174320 B2 | 02 August 2017 |
| | | JP 6554197 B2 | 31 July 2019 |
| | | US 10385128 B2 | 20 August 2019 |
| | | US 2016-0215050 A1 | 28 July 2016 |
| | | US 2019-0382478 A1 | 19 December 2019 |
| | | US 9260517 B2 | 16 February 2016 |
| | | WO 2011-062997 A2 | 26 May 2011 |
| | | WO 2011-062997 A3 | 14 July 2011 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101169030


### Non-patent literature cited in the description


- **ALLEN TM.** *Nat Rev Cancer.,* 2002, vol. 2, 750-63
- **YAO VJ et al.** *J Control Release.,* 2016, vol. 240, 267-86
- **PALMIERI D et al.** *Proc Natl Acad Sci U S A.,* 2015, vol. 112, 9418-23
- **SUKHANOVA A et al.** *Nanoscale Res Lett.,* 2018, vol. 13, 44
- **EPENETOS AA et al.** *Cancer Res.,* 1986, vol. 46, 3183-91
- *Mori T. Curr Pharm Des.,* 2004, vol. 10, 2335-43
- **MCGREGOR DP.** *Curr Opin Pharmacol.,* 2008, vol. 8, 616-9
- **LAM KS et al.** *Chem Rev.,* 1997, vol. 97, 411-48
- **MIKAWA M et al.** *Mol Cancer Ther.,* 2004, vol. 3, 1329-34
- **BORCHARDT TR et al.** *Adv Drug Deliv Rev.,* 1997, vol. 27, 235-56
- **MCGUIRE MJ et al.** *Sci Rep.,* 2014, vol. 4, 4480
- **SHEN N et al.** *Oncotarget.,* 2014, vol. 5, 5494-509
- **LI F et al.** *Nat Commun.,* 2017, vol. 8, 1390
- **GUPTA V et al.** *J Cell Commun Signal.,* 2019, vol. 13, 319-30
- **MORENO A et al.** *Cell Chem. Biol.,* 2019, vol. 26, 634-44
- **WAN X et al.** *Process Biochem.,* 2017, vol. 52, 183-91
- **RAMALINGAM S ; BELANI CP.** *Expert Opin. Pharmacother.,* 2004, vol. 5, 1771-80
- **HENNENFENT KL ; GOVINDAN R.** *Ann Oncol.,* 2006, vol. 17, 735-49
- **TAKESHIMA et al.** *J. Biol. Chem.,* 2003, vol. 278 (2), 1310-1315
- **DENNISON et al.** *Biochem. and Biophy. Res. Comm.,* 2007, vol. 363, 178
- **BAOUM et al.** *Int. J. Pharm.,* 2012, vol. 427, 134-142
- **DEROSSI et al.** *J. Biol. Chem.,* 1994, vol. 269 (14), 10444-10450
- **CHU et al.** *Nanotechnol. Biol. Med.,* 2015, vol. 11, 435-446
- **FUTAKI et al.** *J. Biol. Chem.,* 2001, vol. 276, 5836-5840
- **DESHAYES et al.** *Advanced Drug Delivery Reviews,* 2008, vol. 60, 537-547
- **CHOI et al.** *Nat. Med.,* 2006, vol. 12, 574-579
- **WADA et al.** *Bioorg. Med. Chem.,* 2013, vol. 21, 7669-7673
- **ELMQUIST et al.** *Biochim. Biophys. Acta,* 2006, vol. 1758, 721-729
- **SIMEONI.** *Nucleic Acids Res.,* 2003, vol. 31, 2717-2724
- **PAE et al.** *J. Controlled Release,* 2014, vol. 192, 103-113
- **GALDIERO et al.** *Biochim. Biophys. Acta (BBA) Biomembr.,* 2015, vol. 1848, 16-25
- **ELLIOTT ; O'HARE.** *Cell,* 1997, vol. 88, 223-233
- Remington's Pharmaceutical Sciences. 1995
- **BOTTGER R et al.** *PLoS One.,* 2017, vol. 12, e0178943
- **BERTUCCI ; SVEEN.** *R2: Genomics Analysis and Visualization platform (http://r2.amc.nl)*
- **VAN LONG FN et al.** *Cancers.,* 2018, vol. 10, 390
- **CHEN SC et al.** *Oncotarget.,* 2015, vol. 6, 16253-70